# EUROPEAN PATENT APPLICATION

(11) **EP 3 964 227 A1**
(43) Date of publication of application: **09.03.2022**
(21) Application number: 20195140.7
(22) Date of filing: 08.09.2020
(51) Int. Cl.: A61K 38/18, A61K 38/57, A61P 27/02

(54) **METHOD FOR THE TREATMENT OF A DISEASE USING PIGMENT EPITHELIUM-DERIVED FACTOR (PEDF)**

(71) Applicant: Curebiotec GmbH, 62120 Heidelberg (DE)
(72) Inventor: The designation of the inventor has not yet been filed
(74) Representative: Bohmann, Armin K.

(57) **Abstract**

The present invention is related to a pigment epithelium-derived factor (PEDF) for use in a method for treatment and/or prevention of dry macular degeneration in a subject, preferably dry age-related macular degeneration, wherein the method comprises administering PEDF and VEGF to the subject.

## Description

The present invention is related to a pigment epithelium-derived factor (PEDF) for use in a method for treatment and/or prevention of a disease, and an mRNA coding for a pigment epithelium-derived factor (PEDF) for use in a method for treatment and/or prevention of a disease.

Age-related macular degeneration (AMD) is the most common reason for legal blindness in western countries. Atrophy of the submacular retinal pigment epithelium and the development of choroidal neovascularization (CNV) results secondarily in loss of central visual acuity. Early signs of AMD are deposits (drusen) between retinal pigment epithelium and Bruch's membrane. There are two forms of AMD - wet and dry.

During the disease of wet AMD there is sprouting of choroid vessels into the subretinal space of the macula. These new vessels often develop abnormal, became leaky and cause subretinal edema. These edemas lead to loss of central vision and reading ability.

In patients with dry AMD, the primary effect is loss of the choriocapillaris (Biesemeier, Taubitz et al. 2014). Subsequently, the retinal pigment epithelium (RPE) and the photoreceptors degenerate which leads to geographic atrophy (GA). For dry AMD there is at present no treatment available to prevent loss of the choriocapillaris.

Patients with subfoveal CNV currently were treated with drugs reducing or blocking vascular endothelial growth factor (VEGF). Since 2004, anti-VEGF therapy has become the standard treatment for wet AMD and has revolutionized the management of this disease. Between 2004 and 2006, three anti-VEGF drugs were introduced to ophthalmology after receiving regulatory approval for the treatment of AMD (pegaptanib, ranibizumab) or being used off-label (bevacizumab) (Browning, Kaiser et al. 2012). They exhibit important differences in their sites of activity, formulation methods, binding affinities and biological activities (Julien, Biesemeier et al. 2014). Pegaptanib (Macugen) is an oligonucleotide aptamer that selectively binds to and neutralizes the main pathological isoform of VEGF (VEGF-A165) by attaching to its heparin-binding domain. Ranibizumab (Lucentis, Genentech/Novartis) is an affinity matured, humanized, monoclonal antibody fragment (Fab), whereas bevacizumab (Avastin, Genentech/Roche) is a full-length, humanized monoclonal antibody. Both work by blocking the receptor-binding domain of all isoforms of VEGF-A (Ferrara, Damico et al. 2006). Aflibercept (VEGF Trap-Eye, Eylea, Regeneron/Bayer) is an anti-VEGF agent recently approved by the Food and Drug Administration. It is a fully human, recombinant fusion protein composed of the second immunoglobulin (Ig)-binding domain of VEGFR1 and the third Ig-binding domain of VEGFR2 fused to the fragment crystallizable (Fc) region of human IgG1.

Aflibercept binds to all VEGF-A isoforms, VEGF-B and PlGF (Papadopoulos, Martin et al. 2012). The effects of intravitreally injected bevacizumab in the eyes of monkeys have been extensively described (Peters, Heiduschka et al. 2007, Julien, Biesemeier et al. 2013, Schraermeyer and Julien 2013). The effects included reductions in choriocapillaris fenestrations, photoreceptor damage, formation of immune complexes and thrombotic microangiopathy. A prevailing rationale for thrombosis after bevacizumab treatment was presented by Meyer and colleagues (Meyer, Robles-Carrillo et al. 2009). They found that bevacizumab can induce platelet aggregation, degranulation and thrombosis through complex formation with VEGF, heparin and activation of the platelet Fc gamma RIIa receptor. Moreover, other results have demonstrated effective binding of the Fc domain of bevacizumab to human RPE and human umbilical vascular endothelial cell membranes via Fc receptors or membrane-bound VEGF, activating the complement cascade and leading to cell death (Meyer and Holz 2011). It is unclear whether there is a similar problem with aflibercept as it also contains the Fc domain of human IgG1. Furthermore, the IgG1 isotype is known to be very effective in the activation of the complement system through the classical pathway (Daha, Banda et al. 2011). Indeed, the Fc portion of IgG1 has a high ability to bind C1q causing subsequent activation of the classical pathway (Daha, Banda et al. 2011). In contrast, ranibizumab does not possess the Fc domain avoiding activation of the complement cascade, but nevertheless also induces hemolysis and fibrin formation in non-clinical studies (Julien, Biesemeier et al. 2014).

It was reported that VEGF inhibition can activate thrombocytes in humans treated for cancer (Meyer, Robles-Carrillo et al. 2009) or for neovascular AMD (Schraermeyer and Julien 2013). In addition, VEGF drugs after intravitreal application induced thrombotic microangiopathy in the choriocapillaris of monkeys (Peters, Heiduschka et al. 2007, Schraermeyer and Julien 2012). Anti-VEGF drugs also induce hemolysis, stasis and fibrin formation within the choriocapillaris (Schraermeyer and Julien 2012, Schraermeyer and Julien 2013, Julien, Biesemeier et al. 2014). Avastin forms together with heparin and VEGF protein complexes that induce thrombotic events (Julien, Biesemeier et al. 2013). In blood vessels of surgically excised choroidal membranes from patients suffering from wet AMD, anti-VEGF (bevacizumab) treatment induced thrombosis and protein complex formation (Schraermeyer, Julien et al. 2015).

These side effects are of disadvantage because AMD patients, due to their age, have higher risks to suffer from stroke or other vessel related diseases. Thus, an increased long-term mortality in individuals with wet AMD treated with bevacizumab compared to a same age and gender group without wet AMD was reported (Hanhart, Comaneshter et al. 2017). Particularly after myocardial infarction (Hanhart, Comaneshter et al. 2018) and after a cerebrovascular event (Hanhart, Comaneshter et al. 2018), the mortality caused by anti-VEGF treatment is largely enhanced. Moreover, long term treatment with anti-VEGF drugs causes loss of the original choriocapillaris and geographic atrophy in the peripheral parts of the retina in patients with wet AMD (Schutze, Wedl et al. 2015). Thus, this treatment induces additional visual loss that would not have occurred without this treatment.

Recently, due to the new possibility of using angiography together with ocular coherence tomography (OCTA) (Treister, Nesper et al. 2018) overcoming the short-coming of earlier fluorescein angiography only detecting CNV after leakage had already occurred, there is detected a notable prevalence of subclinical CNV in fellow eyes with unilateral exudative CNV, and significantly greater choriocapillaris nonperfusion adjacent to all CNV lesions.

Treister et al. (Treister et al. 2018) identified a trend for increased choriocapillaris nonperfusion in exudative AMD eyes as compared with their fellow subclinical CNV eyes. This clearly shows that subclinical CNVs exist without reducing the visual acuity of these patients. These new findings support an earlier observation in eyes with late wet AMD in which neovascularization could help photoreceptors to survive (Biesemeier, Julien et al. 2014).

Regarding the long history for treatment of wet AMD, always the same principle as been used, namely removing or blocking the newly formed blood vessels. In order to achieve this, different methods have been used: laser coagulation, surgery, radiation, photodynamic therapy and today intravitreal anti-VEGF drugs. Whereas none of the first methods could improve vision, usage of anti-VEGF (ranibizumab) was successful and can improve visual acuity for a while but is still far away from an optimal rescue.

The problem underlying the present invention is the provision of a means for the treatment of ocular diseases such as age-related macular degeneration (AMD).

A further problem underlying the present invention is the provision of a means for the treatment of ocular disease such as age-related macular degeneration (AMD) providing improved visual acuity for a prolonged period of time.

These and other problems underlying the present invention are solved by the subject matter of the attached independent claims. Preferred embodiments may be taken from the attached dependent claims.

The problem underlying the present invention is also solved in a first aspect, which is also a first embodiment of the first aspect, by a pigment epithelium-derived factor (PEDF) for use in a method for treatment and/or prevention of a disease, wherein the method comprises administering PEDF to a subject and wherein treatment and/or prevention of a disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

In a second embodiment of the first aspect which is also an embodiment of the first embodiment of the first aspect, the disease is an eye disease.

In a third embodiment of the first aspect which is also an embodiment of the second embodiment of the first aspect, the eye disease is macular degeneration, preferably macular degeneration is age-related macular degeneration (AMD), more preferably dry age-related macular degeneration or wet age-related macular degeneration.

In a fourth embodiment of the first aspect which is also an embodiment of the third embodiment of the first aspect, PEDF inhibits growth and/or formation of geographic atrophy in wet AMD and/or dry AMD

In a fifth embodiment of the first aspect which is also an embodiment of the second embodiment of the first aspect, the disease is selected from the group comprising central serous chorioretinopathy, diabetic retinopathy, rubeosis iridis, corneal neovascularization, polypoidal choroidal vasculopathy, retinopathy of the prematurity and retinal and choroidal fibrosis.

In a sixth embodiment of the first aspect which is also an embodiment of the fifth embodiment of the first aspect, PEDF inhibits the progression of retinal and/or choroidal fibrosis.

The problem underlying the present invention is also solved in a second aspect, which is also a first embodiment of the second aspect, by an mRNA coding for a pigment epithelium-derived factor (PEDF) for use in a method for treatment and/or prevention of a disease, wherein the method comprises administering the mRNA coding for PEDF to a subject and wherein treatment and/or prevention of a disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

In a second embodiment of the second aspect which is also an embodiment of the first embodiment of the second aspect, the disease is an eye disease.

In a third embodiment of the second aspect which is also an embodiment of the second embodiment of the second aspect, the eye disease is macular degeneration, preferably macular degeneration is age-related macular degeneration (AMD), more preferably dry age-related macular degeneration or wet age-related macular degeneration.

In a fourth embodiment of the second aspect which is also an embodiment of the third embodiment of the second aspect, PEDF inhibits growth and/or formation of geographic atrophy in wet AMD and/or dry AMD

In a fifth embodiment of the second aspect which is also an embodiment of the second embodiment of the second aspect, the disease is selected from the group comprising central serous chorioretinopathy, diabetic retinopathy, rubeosis iridis, corneal neovascularization, polypoidal choroidal vasculopathy, retinopathy of the prematurity and retinal and choroidal fibrosis.

In a sixth embodiment of the second aspect which is also an embodiment of the fifth embodiment of the second aspect, PEDF inhibits the progression of retinal and/or choroidal fibrosis.

The problem underlying the present invention is also solved in a third aspect, which is also a first embodiment of the third aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is an eye disease.

In a second embodiment of the third aspect which is also an embodiment of the first embodiment of the third aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

In a third embodiment of the third aspect which is also an embodiment of the first and second embodiment of the third aspect, the eye disease is macular degeneration, preferably macular degeneration is age-related macular degeneration (AMD), more preferably dry age-related macular degeneration or wet age-related macular degeneration.

In a fourth embodiment of the third aspect which is also an embodiment of the third embodiment of the third aspect, PEDF inhibits growth and/or formation of geographic atrophy in wet AMD and/or dry AMD

In a fifth embodiment of the third aspect which is also an embodiment of the second embodiment of the third aspect, the disease is selected from the group comprising central serous chorioretinopathy, diabetic retinopathy, rubeosis iridis, corneal neovascularization, polypoidal choroidal vasculopathy, retinopathy of the prematurity and retinal and/or choroidal fibrosis.

In a sixth embodiment of the third aspect which is also an embodiment of the fifth embodiment of the third aspect, PEDF inhibits the progression of retinal and/or choroidal fibrosis.

The problem underlying the present invention is also solved in a fourth aspect, which is also a first embodiment of the fourth aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is macular degeneration.

In a second embodiment of the fourth aspect which is also an embodiment of the first embodiment of the fourth aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

In a third embodiment of the fourth aspect which is also an embodiment of the first and second embodiment of the fourth aspect, the macular degeneration age-related macular degeneration (AMD), more preferably dry age-related macular degeneration or wet age-related macular degeneration.

In a fourth embodiment of the fourth aspect which is also an embodiment of the third embodiment of the fourth aspect, PEDF inhibits growth and/or formation of geographic atrophy in wet AMD and/or dry AMD

The problem underlying the present invention is also solved in a fifth aspect, which is also a first embodiment of the fifth aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is central serous chorioretinopathy.

In a second embodiment of the fifth aspect which is also an embodiment of the first embodiment of the fifth aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The problem underlying the present invention is also solved in a sixth aspect, which is also a first embodiment of the sixth aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is diabetic retinopathy.

In a second embodiment of the sixth aspect which is also an embodiment of the first embodiment of the sixth aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The problem underlying the present invention is also solved in a seventh aspect, which is also a first embodiment of the seventh aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is rubeosis iridis.

In a second embodiment of the seventh aspect which is also an embodiment of the first embodiment of the seventh aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The problem underlying the present invention is also solved in an eighth aspect, which is also a first embodiment of the eighth aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is corneal neovascularization.

In a second embodiment of the eighth aspect which is also an embodiment of the first embodiment of the eighth aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The problem underlying the present invention is also solved in a ninth aspect, which is also a first embodiment of the ninth aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is polypoidal choroidal vasculopathy.

In a second embodiment of the ninth aspect which is also an embodiment of the first embodiment of the ninth aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The problem underlying the present invention is also solved in a tenth aspect, which is also a first embodiment of the tenth aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is retinopathy of the prematurity.

In a second embodiment of the tenth aspect which is also an embodiment of the first embodiment of the tenth aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The problem underlying the present invention is also solved in an eleventh aspect, which is also a first embodiment of the eleventh aspect, by a pigment epithelium-derived factor (PEDF) or an mRNA coding for a pigment epithelium-derived factor (PEDF) for use in a method for treatment and/or prevention of a disease, wherein the method comprises administering the PEDF or the mRNA coding for PEDF to a subject, wherein the disease is retinal and/or choroidal fibrosis.

In a second embodiment of the eleventh aspect which is also an embodiment of the first embodiment of the eleventh aspect, treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

In a third embodiment of the eleventh aspect which is also an embodiment of the first and second embodiment of the eleventh aspect, PEDF and/or mRNA coding for PEDF inhibit progression of retinal and/or choroidal fibrosis.

In an embodiment of the first, second, third, fourth, fifth, sixth, seventh, eighth, ninth, tenth, and eleventh aspect, including each and any embodiment thereof, labyrinth capillary formation is labyrinth capillary formation in an eye, preferably in eye disease.

In an embodiment of the first, second, third, fourth, fifth, sixth, seventh, eighth, ninth, tenth, and eleventh aspect, including each and any embodiment thereof, inducing growth of choriocapillaris comprises or is inducing growth of new choriocapillaris, preferably new choriocapillaris are non-leaking choriocapillaris.

In an embodiment of the first, second, third, fourth, fifth, sixth, seventh, eighth, ninth, tenth, and eleventh aspect, including each and any embodiment thereof, inducing growth of choriocapillaris provides choriocapillaris which are capable of replacing original choriocapillaris, preferably original choriocapillaris are diseased choriocapillaris, more preferably choriocapillaris which are capable of replacing original choriocapillaris are non-leaking choriocapillaris.

In an embodiment of the first, second, third, fourth, fifth, sixth, seventh, eighth, ninth, tenth, and eleventh aspect, including each and any embodiment thereof, tightening choriocapillaris comprises tightening pathological choriocapillaris.

In an embodiment of the first, second, third, fourth, fifth, sixth, seventh, eighth, ninth, tenth, and eleventh aspect, including each and any embodiment thereof, inhibiting extracellular matrix formation comprises inhibition of extracellular matrix formation towards the lumen of a blood vessel and/or around a blood vessel.

In an embodiment of the first, second, third, fourth, fifth, sixth, seventh, eighth, ninth, tenth, and eleventh aspect, including each and any embodiment thereof, protecting choriocapillaris comprises protecting choriocapillaris from the damaging effect of an anti-VEGF drug.

In an embodiment of the first, second, third, fourth, fifth, sixth, seventh, eighth, ninth, tenth, and eleventh aspect, including each and any embodiment thereof, protecting choriocapillaris comprises protecting choriocapillaris from the damaging effect of withdrawal of an anti-VEGF drug.

In an embodiment of the first, second, third, fourth, fifth, sixth, seventh, eighth, ninth, tenth, and eleventh aspect, including each and any embodiment thereof, guiding vessel development comprises development of a functional blood vessel, preferably a functional blood vessel from a pathological blood vessel.

In an embodiment of the first, second, third, fourth, fifth, sixth, seventh, eighth, ninth, tenth, and eleventh aspect, including each and any embodiment thereof, the pathological blood vessel is the result of a pathological condition, preferably of a pathological condition of the subject, more preferably the pathological condition is the disease from which the subject is suffering or at risk of suffering and/or for the treatment of which PEDF or mRNA coding for PEDF is used or intendent for being used.

In an embodiment of the first, second, third, fourth, fifth, sixth, seventh, eighth, ninth, tenth, and eleventh aspect, including each and any embodiment thereof, PEDF or mRNA coding for PEDF is administered intravitreally or sub-retinally.

In an embodiment of the first, second, third, fourth, fifth, sixth, seventh, eighth, ninth, tenth, and eleventh aspect, including each and any embodiment thereof, the method further comprises applying an anti-VEGF therapy, preferably the anti-VEGF therapy comprises administration to the subject of an anti-VEGF drug, wherein the anti-VEGF drug is selected from the group comprising pegaptanib, ranibizumab, bevacizumab and aflibercept. In an embodiment thereof, the combined use of both PEDF and an anti-VEGF therapy allows the decreasing of the amount of the anti-VEGF therapy administered to the subject compared to the sole use of the anti-VEGF therapy. Such decreasing of the amount of the anti-VEGF therapy administered to the subject typically results in a decrease in side effects, in particular side effects of said anti-VEGF therapy such as cardiovascular side effects.

Without wishing to be bound by any theory, the present inventor has surprisingly found that pigment epithelium derived factor (PEDF) is capable of inducing growth of healthy and functional choriocapillaris and effects associated therewith such as inhibiting labyrinth capillary formation, tightening choriocapillaris, inhibiting extracellular matrix formation, inducing growth of choriocapillaris, preferably inducing growth of non-leaking choriocapillaris, protecting choriocapillaris and/or guiding vessel development which is beneficial in the treatment of eye diseases. Insofar, the present invention turns away from the current state of the art in the treatment of eye disease which is based on blocking vessel growth or removing vessels.

Additionally, the present inventor has surprisingly found that by balancing the level of PEDF protein and VEGF protein in the eye, various eye diseases, including dry and wet age-related macular degeneration, as well as certain symptoms and morphological changes, respectively, associated with said eye diseases such as treatment-naive quiescent choroidal neovascularization and geographic atrophy, can be treated. In connection therewith it will be acknowledged by a person skilled in the art that such balancing of the level of PEDF protein and VEGF protein may, apart from the administration of PEDF, require the administration of VEGF or an anti-VEGF agent. Such anti-VEGF agent is an agent which interferes with the activity of VEGF, in particular with the activity of VEGF in an eye, more specifically the angiogenesis activity of VEGF. In an embodiment of each and any aspect of the present invention, such anti-VEGF agent is an anti-VEGF therapy and more specifically an anti-VEGF drug described herein. Methods for determining the level of PEDF protein and VEGF protein in the vitreous body or in the intraocular fluid as a basis for determining whether the level of PEDF and/or VEGF is to be decreased or increased, and if so, to which extent, are known in the art and, for example, described in Biesemeier A et al. (2014).

It will be further acknowledged that in each and any aspect of the present invention, including any embodiment thereof, when referring to the administration of PEDF, the administration of an mRNA coding for PEDF is equally disclosed and encompassed. Similarly, when referring to the administration of VEGF, the administration of an mRNA coding for VEGF is equally disclosed and encompassed. Finally, it will be acknowledged that in each and any aspect of the present invention, including any embodiment thereof, when both PEDF and VEGF are administered to a patient, such administration may encompass (a) the administration of PEDF protein and of VEGF protein, (b) the administration of an mRNA coding for PEDF and of a VEGF protein, (c) the administration of PEDF and of an mRNA coding for VEGF, and (d) the administration of an mRNA coding for PEDF and of an mRNA coding for VEGF.

In light of such inhibition of labyrinth capillary formation by means of PEDF, the therapeutic effectiveness of PEDF in the treatment of eye diseases is plausible, in particular for those eye diseases showing labyrinth capillary formation such as age-related macular degeneration (AMD), both dry AMD and wet AMD, central serous chorioretinopathy, diabetic retinopathy, rubeosis iridis, corneal neovascularization, polypoidal choroidal vasculopathy and retinopathy of the prematurity. For example, if patients with wet AMD are injected with Fluorescein it is observed that high amounts of liquid leak out from the pathological vessels in a short time. The most plausible cause for this finding is that there are large gaps between or within the endothelial walls. However, gaps in the endothelium which connect blood cells and extracellular matrix would immediately be closed by thrombocytes which is not happening. Recently, capillaries with many microvilli-like projections of the endothelium which formed a labyrinth-like structure into the vessel's lumen were found in surgically excised choroidal neovascularizations (CNVs) from AMD patients. The lumen of such capillaries showed open connections towards the interstitium. These capillaries were connected to the network of blood vessels because they were filled with plasma and, therefore, they were a source for leakage. This type of capillary was frequently observed in CNVs and was called "labyrinth capillary". Leaky sites in these labyrinth capillaries cannot be closed by thrombocytes because due to the reduced lumen of the labyrinth capillaries thrombocytes cannot enter. Therefore, this vessel type causes chronic plasma exudation and is the origin of edema (Schraermeyer, Julien et al. 2015).

Furthermore, pigment epithelium derived factor (PEDF) has a very strong neurotrophic and neuroprotective effect (King and Suzuma 2000). This factor is produced by RPE under normoxic conditions. Production is stopped during hypoxia. This greatly promotes neovascularization. In age-related macular degeneration (AMD) the damaged RPE cells produce too little PEDF. This produces uncontrolled neoangiogenesis. It was believed that the central effect of PEDF in the eye is to prevent neogenesis of vessels (King and Suzuma 2000) but in accordance with the present invention, PEDF can stabilize CNV vessels and avoid Labyrinth capillary formation if pathological vessel formation has been initiated by VEGF.

In a first aspect, which is also a first embodiment of the first aspect, the problem underlying the present invention is solved by a pigment epithelium-derived factor (PEDF) for use in a method for treatment and/or prevention of a disease, wherein the method comprises administering PEDF to a subject and wherein treatment and/or prevention of a disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

In a preferred embodiment thereof, PEDF is the human PEDF protein, in a more preferred embodiment, PEDF comprises an amino acid sequence according to SEQ ID NO: 1:

QNPASPPEEG SPDPDSTGAL VEEEDPFFKV PVNKLAAAVS
NFGYDLYRVR SSTSPTTNVL LSPLSVATAL SALSLGAEQR
TESIIHRALY YDLISSPDIH GTYKELLDTV TAPQKNLKSA
SRIVFEKKLR IKSSFVAPLE KSYGTRPRVL TGNPRLDLQE
INNWVQAQMK GKLARSTKEI PDEISILLLG VAHFKGQWVT
KFDSRKTSLE DFYLDEERTV RVPMMSDPKA VLRYGLDSDL
SCKIAQLPLT GSMSIIFFLP LKVTQNLTLI EESLTSEFIH
DIDRELKTVQ AVLTVPKLKL SYEGEVTKSL QEMKLQSLFD
SPDFSKITGK PIKLTQVEHR AGFEWNEDGA GTTPSPGLQP

AHLTFPLDYH LNQPFIFVLR DTDTGALLFI GKILDPRGP (SEQ ID NO: 1)

In a preferred embodiment thereof, PEDF is a derivative of PEDF, preferably of human PEDF, and more preferably of PEDF comprising an amino acid sequence according to SEQ ID NO: 1. It will be appreciated by a person skilled in the art, that any derivative of PEDF may be used as long as the PEDF is capable of causing the above effects and in particular the effect of inhibiting labyrinth capillary formation, inducing growth of choriocapillaris, tightening choriocapillaris, inhibiting extracellular matrix formation, protecting choriocapillaris, and guiding vessel development. In an embodiment, the PEDF is one having a homology or identity to the amino acid sequence of SEQ ID NO: 1of at least, 85%, 86%, 87 %, 88%, 89%, 90%, 91%, 92 %, 93 %, 94 %, 95 %, 96 %, 97 %, 98 % or 99 %. In a further embodiment, a derivative of PEDF is one where at amino acid position 20 of SEQ ID NO: 1 the amino acid residue is pyrrolidone carboxylic acid, at amino acid position 24 of SEQ ID NO: 1 the amino acid residue is phosphoserine, at amino acid position 114 of SEQ ID NO: 1 the amino acid residue is phosphoserine, at amino acid position 227 of SEQ ID NO: 1 the amino acid residue is phosphoserine and/or at amino acid position 285 of SEQ ID NO: 1 the amino acid residue is N-linked (GlcNAc) asparagine.

It will be appreciated that any of the above effects and, in particular, labyrinth capillary formation and any change thereof including inhibition thereof, induction of growth of choriocapillaris, tightening of choriocapillaris, inhibition of extracellular matrix formation, protection of choriocapillaris, and guidance of vessel development can be assessed by optical coherence tomography (OCT-A), preferable when combined with fluorescein angiography (FA) which is suitable for detecting and assessing, respectively, leaking vessels (Spaide et al. 2015). Optical coherence tomography angiography (OCT-A) emerged as a non-invasive technique for imaging the microvasculature of the retina and choroid (Spaide et al.2015). Briefly, OCT-A technology uses laser light reflectance of the surface of moving red blood cells to accurately depict vessels through different segmented areas of the eye, thus eliminating the need for intravascular dyes. The OCT scan of a patient's retina consists in multiple individual A-scans, which compiled into a B-scan provides cross-sectional structural information. With OCT-A technology, the same tissue area is repeatedly imaged and differences analyzed between scans, thus allowing one to detect zones containing high flow rates, i.e. with marked changes between scans, and zones with slower, or no flow at all, which will be similar among scans.

OCT-A and FA may also be used for the detection and assessment, respectively, of edema which are located within the retina and/or subretinal space.

In a second embodiment of the first aspect, which is also an embodiment of the first embodiment of the first aspect, the disease is an eye or ocular disease.

In a third embodiment of the first aspect, which is also an embodiment of the first and second embodiment of the first aspect, the eye disease is macular degeneration, preferably age-related macular degeneration (AMD), more preferably dry age-related macular degeneration of wet age-related macular degeneration.

In a fourth embodiment of the first aspect, which is also an embodiment of the first and second embodiment of the first aspect, the eye disease is selected from the group comprising central serous chorioretinopathy, diabetic retinopathy, rubeosis iridis, corneal neovascularization, polypoidal choroidal vasculopathy and retinopathy of the prematurity.

In a fifth embodiment of the first aspect, which is also an embodiment of the first, second, third and fourth embodiment of the first aspect, labyrinth capillary formation is labyrinth capillary formation in an eye, preferably in eye disease.

In a sixth embodiment of the first aspect, which is also an embodiment of the first, second, third, fourth and fifth embodiment of the first aspect, inducing growth of choriocapillaris comprises or is inducing growth of new choriocapillaris, preferably inducing growth of non-leaking choriocapillaris.

In a seventh embodiment of the first aspect, which is also an embodiment of the first, second, third, fourth, fifth and sixth embodiment of the first aspect, inducing growth of choriocapillaris provides choriocapillaris which are capable of replacing original choriocapillaris, preferably original choriocapillaris are diseased choriocapillaris. In connection therewith, it will be acknowledged by a person skilled in the art that diseased choriocapillaris is located between Bruch's membrane and RPE and can be seen in OCT-A.

In an eight embodiment of the first aspect, which is also an embodiment of the first, second, third, fourth, fifth, sixth and seventh embodiment of the first aspect, wherein tightening choriocapillaris comprises tightening pathological choriocapillaris. In connection therewith, it will be acknowledged that each neovascular choriocapillaris or vessel located between Bruch's membrane and RPE or within the subretinal space is preferably regarded as pathologic. More preferably, a choriocapillaris is regarded as pathologic only when they develop into labyrinthy capillaries or became leaky by other reasons. Diagnosis thereof may be performed by OCT-A and/or fluorescein angiography (FA).

In a ninth embodiment of the first aspect, which is also an embodiment of the first, second, third, fourth, fifth, sixth, seventh and eighth embodiment of the first aspect, inhibiting extracellular matrix formation comprises inhibition of extracellular matrix formation towards the lumen of a blood vessel and/or around a blood vessel. Preferably, such vessel does not inhibit the flow of red blood cells by absence of endothelial projection into the lumen.

In a tenth embodiment of the first aspect, which is also an embodiment of the first, second, third, fourth, fifth, sixth, seventh, eighth and ninth embodiment of the first aspect, protecting choriocapillaris comprises protecting choriocapillaris from the damaging effect of an anti-VEGF drug. Such anti-VEGF drug is preferably one selected from the group comprising pegaptanib, ranibizumab, bevacizumab and aflibercept. In connection therewith, it will be acknowledged that such damaging effect may encompass regression of blood vessels and degeneration of RPE and photo receptors resulting in geographic atrophy. Geographic atrophy may be detected as a dark spot upon scanning laser ophthalmoscopy (SLO) because autofluorescence of the RPE disappears. The SLO picture is the result of autofluorescence of lipofuscin in RPE.

In an eleventh embodiment of the first aspect, which is also an embodiment of the first, second, third, fourth, fifth, sixth, seventh, eighth, ninth and tenth embodiment of the first aspect, protecting choriocapillaris comprises protecting choriocapillaris from the damaging effect of withdrawal of an anti-VEGF drug. In connection therewith, it will be appreciated that, preferably, blood vessels become leaky and change into labyrinth capillaries; endothelial cells proliferate and migrate.

In a twelfth embodiment of the first aspect, which is also an embodiment of the first, second, third, fourth, fifth, sixth, seventh, eighth, ninth, tenth and eleventh embodiment of the first aspect, guiding vessel development comprises development of a functional blood vessel, preferably a functional blood vessel from a pathological blood vessel. In connection therewith, preferably a pathological blood vessel is a blood vessel which does not allow proper blood flow, is leaky of forms too many or atypical extracellular matrix proteins.

In a 13^{th} embodiment of the first aspect, which is also an embodiment of the twelfth embodiment of the first aspect, the pathological blood vessel is the result of a pathological condition. Such pathological condition may be one or a combination of hypoxia, upregulation of HIF 1 alpha, atypical formation of growth factors and VEGF.

In a 14^{th} embodiment of the first aspect, which is also an embodiment of the first, second, third, fourth, fifth, sixth, seventh, eighth, ninth, tenth, eleventh, twelfth and 13^{th} embodiment of the first aspect, PEDF is administered intravitreally or sub-retinally, or as a vector such as adeno-associated virus coding for PEDF.

In a 15^{th} embodiment of the first aspect, which is also an embodiment of the first, second, third, fourth, fifth, sixth, seventh, eighth, ninth, tenth, eleventh, twelfth, 13^{th} and 14^{th} embodiment of the first aspect, the method further comprises applying an anti-VEGF therapy, preferably the anti-VEGF therapy comprises administration to the subject of an anti-VEGF drug, wherein the anti-VEGF drug is selected from the group comprising pegaptanib, ranibizumab, bevacizumab and aflibercept. In connection therewith, it will be acknowledged by a person skilled in the art that PEDF may be used early, for example when the CNV is detected in one eye, the fellow eye may be treated prophylactically; also if a subclinical CNV with normal vision of the eye is diagnosed the treatment may begin in order to keep the CNV stable.

In a 16^{th} embodiment of the first aspect, which is also an embodiment of the first, second, third, fourth, fifth, sixth, seventh, eighth, ninth, tenth, eleventh, twelfth, 13^{th}, 14^{th} and 15^{th} embodiment of the first aspect, the subject is subject who is suffering from side effects of anti-VEGF treatment, preferably visual loss arising from anti-VEGF treatment.

In a second aspect, which is also a first embodiment of the second aspect, the problem underlying the present invention is solved by an mRNA coding for a pigment epithelium-derived factor (PEDF) for use in a method for treatment and/or prevention of a disease, wherein the method comprises administering PEDF to a subject and wherein treatment and/or prevention of a disease comprises inhibiting labyrinth capillary formation, inducing growth of choriocapillaris, tightening choriocapillaris, inhibiting extracellular matrix formation, protecting choriocapillaris, and/or guiding vessel development. In an embodiment, the mRNA is an mRNA coding for the amino acid sequence according to SEQ ID NO: 1. It is appreciated by a person skilled in the art that if an mRNA coding for PEDF is used in accordance with the present invention, such as in a method for treatment and/or prevention of a disease, the mRNA contains a sequence that codes for a signal peptide that directs the mRNA into the endoplasmic reticulum (ER) and that is the cleaved off. In an embodiment, the mRNA is an mRNA coding for the amino acid sequence according to SEQ ID NO: 2:

ATGCAGGCCCTGGTGCTACTCCTCTGCATTGGAGCCCTCCTCGGGCACAGCAGCT
GCCAGAACCCTGCCAGCCCCCGGAGGAGGGCTCCCAGACCCCGACAGCACAG
GGGCGCTGGTGGAGGAGGAGGATCCTTTCTTCAAAGTCCCCGTGAACAAGCTGG
CAGCGGCTGTCTCCAACTTCGGCTATGACCTGTACCGGGTGCGATCCAGCACGAG
CCCCACGACCAACGTGCTCCTGTCTCCTCTCAGTGTGGCCACGGCCCTCTCGGCCC

TCTCGCTGGGAGCGGAGCAGCGAACAGAATCCATCATTCACCGGGCTCTCTACTA
TGACTTGATCAGCAGCCAGACATCCATGGTACCTATAAGGAGCTCCTTGACACG
GTCACCGCCCCCAGAAGAACCTCAAGAGTGCCTCCCGGATCGTCTTTGAGAAGA
AGCTGCGCATAAAATCCAGCTTTGTGGCACCTCTGGAAAGTCATATGGGACCAG
GCCCAGAGTCCTGACGGGCAACCCTCGCTTGGACCTGCAAGAGATCAACAACTG
GGTGCAGGCGCAGATGAAAGGGAAGCTCGCCAGGTCCACAAAGGAAATTCCCGA
TGAGATCAGCATTCTCCTTCTCGGTGTGGCGCACTTCAAGGGGCAGTGGGTAACA
AAGTTTGACTCCAGAAAGACTTCCCTCGAGGATTTCTACTTGGATGAAGAGAGGA
CCGTGAGGGTCCCCATGATGTCGGACCCTAAGGCTGTTTACGCTATGGCTTGGA
TTCAGATCTCAGCTGCAAGATTGCCCAGCTGCCCTTGACCGGAAGCATGAGTATC
ATCTTCTTCCTGCCCCTGAAAGTGACCCAGAATTTGACCTTGATAGAGGAGAGCC
TCACCTCCGAGTTCATTCATGACATAGACCGAGAACTGAAGACCGTGCAGGCGGT
CCTCACTGTCCCCAAGCTGAAGCTGAGTTACGAAGGCGAAGTCACCAAGTCCCTG
CAGGAGATGAAGCTGCAATCCTTGTTTGATTCACCAGACTTTAGCAAGATCACAG
GCAAACCCATCAAGCTGACTCAGGTGGAACACCGGGCTGGCTTTGAGTGGAACG
AGGATGGGGCGGGAACCACCCCCAGCCCAGGGCTGCAGCCTGCCCACCTCACCT
TCCCGCTGGACTATCACCTTAACCAGCCTTTCATCTTCGTACTGAGGGACACAGA
CACAGGGGCCCTTCTCTTCATTGGCAAGATTCTGGACCCCAGGGGCCCCTAA.

The first 57 nucleotides of the nucleotide sequence of SEQ ID NO: 2 code for the signal peptide of the human PEDF. It is, however, within the present invention that said signal peptide and the nucleotide sequence coding therefor, is replaced by a different signal peptide and the nucleotide sequence coding for such different signal peptide, respectively. Sch different signal peptides are known in the art.

In an alternative embodiment, the mRNA is a nucleotide sequence of SEQ ID NO: 3:

GGACGCTGGATTAGAAGGCAGCAAAAAAAGATCTGTGCTGGCTGGAGCCCCCTC
AGTGTGCAGGCTTAGAGGACTAGGCTGGGTGTGGAGCTGCAGCGTATCCACAG
GCCCCAGGATGCAGGCCCTGGTGCTACTCCTCTGCATTGGAGCCCTCCTCGGGCA
CAGCAGCTGCCAGAACCCTGCCAGCCCCCGGAGGAGGGCTCCCCAGACCCCGA

CAGCACAGGGGCGCTGGTGGAGGAGGAGGATCCTTTCTTCAAAGTCCCCGTGAA
CAAGCTGGCAGCGGCTGTCTCCAACTTCGGCTATGACCTGTACCGGGTGCGATCC
AGCATGAGCCCCACGACCAACGTGCTCCTGTCTCCTCTCAGTGTGGCCACGGCCC
TCTCGGCCCTCTCGCTGGGAGCGGACGAGCGAACAGAATCCATCATTCACCGGGC
TCTCTACTATGACTTGATCAGCAGCCCAGACATCCATGGTACCTATAAGGAGCTC
CTTGACACGGTCACTGCCCCCCAGAAGAACCTCAAGAGTGCCTCCCGGATCGTCT
TTGAGAAGAAGCTRCGCATAAAATCCAGCTTTGTGGCACCTCTGGAAAAGTCATA
TGGGACCAGGCCCAGAGTCCTGACGGGCAACCCTCGCTTGGACCTGCAAGAGAT
CAACAACTGGGTGCAGGCGCAGATGAAAGGGAAGCTCGCCAGGTCCACAAAGGA
AATTCCCGATGAGATCAGCATTCTCCTTCTCGGTGTGGCGCACTTCAAGGGGCAG
TGGGTAACAAAGTTTGACTCCAGAAAGACTTCCCTCGAGGATTTCTACTTGGATG
AAGAGAGGACCGTGAGGGTCCCCATGATGTCGGACCCTAAGGCTGTTTTACGCTA
TGGCTTGGATTCAGATCTCAGCTGCAAGATTGCCCAGCTGCCCTTGACCGGAAGC
ATGAGTATCATCTTCTTCCTGCCCCTGAAAGTGACCCAGAATTTGACCTTGATAG
AGGAGAGCCTCACCTC (SEQ ID NO: 3)

In a further embodiment of the second aspect, including any embodiment thereof, the mRNA is a recombinant or heterologous mRNA which preferably comprises a structural element such as a 5' UTR and/or 3' UTR, which is different from the 5' UTR and/or 3' UTR of the mRNA form which the coding sequence of PEDF is taken.

The disclosure of the first aspect, including any embodiment thereof, equally applies to the second aspect. In other words, each and any embodiment of the first aspect is also an embodiment of the second aspect, including any embodiment thereof.

In a twelfth aspect, which is also a first embodiment of the twelfth aspect, the problem underlying the present invention is also solved by a pigment epithelium-derived factor (PEDF) for use in a method for the treatment and/or prevention of dry macular degeneration in a subject, preferably dry age-related macular degeneration, wherein the method comprises administering PEDF and VEGF to the subject.

In a second embodiment of the twelfth aspect which is also an embodiment of the first embodiment of the twelfth aspect, the subject is suffering from treatment-naive quiescent choroidal neovascularization. In connection with this embodiment it is to be acknowledged that treatment-naive quiescent choroidal neovascularization is observed in individuals between the choroid and the retinal pigment epithelium with the absence of clinical symptoms. These CNV's are fully perfused but lack any leakage and exudation of liquid. Also the visual acuity in these individuals can be normal. (See, Querques et al., and Mentes J and Yildirim S).

In a third embodiment of the twelfth aspect which is also an embodiment of the first and second embodiment of the twelfth aspect, the visual acuity of the subject is normal.

In a fourth embodiment of the twelfth aspect which is also an embodiment of the first, second and third embodiment of the twelfth aspect, wherein the subject is suffering from geographic atrophy. In connection with this embodiment, it is to be acknowledged that geographic atrophy (GA) represents the nonexudative late stage of age-related macular degeneration (AMD). It is typically characterized by areas of loss of outer retinal layers including photoreceptors, degeneration of the retinal pigment epithelium, and rarefication of the choriocapillaris. (See, Muller, P. L., et al. (2020)).

In a fifth embodiment of the twelfth aspect which is also an embodiment of the fourth embodiment of the twelfth aspect, geographic atrophy is the result of anti-VEGF therapy.

In a sixth embodiment of the twelfth aspect which is also an embodiment of the first, second, third, fourth and fifth embodiment of the twelfth aspect, PEDF induces growth of choriocapillaris.

In a seventh embodiment of the twelfth aspect which is also an embodiment of the sixth embodiment of the twelfth aspect, choriocapillaris are non-leaking choriocapillaris.

In an eighth embodiment of the twelfth aspect which is also an embodiment of the first, second, third, fourth, fifth, sixth and seventh embodiment of the twelfth aspect, PEDF inhibits growth and/or formation of geographic atrophy.

It is to be acknowledged that the disclosure of the first aspect, including any embodiment thereof, equally applies to the twelfth aspect. In other words, each and any embodiment of the first aspect is also an embodiment of the twelfth aspect, including any embodiment thereof.

In a 13^{th} aspect, which is also a first embodiment of the 13^{th} aspect, the problem underlying the present invention is also solved by an mRNA coding for pigment epithelium-derived factor (PEDF) for use in a method for the treatment and/or prevention of dry macular degeneration in a subject, preferably dry age-related macular degeneration, wherein the method comprises administering the mRNA coding for PEDF and VEGF or an mRNA coding for VEGF to the subject.

It is to be acknowledged that the disclosure of the second and twelfth aspect, including any embodiment thereof, equally applies to the 13^{th} aspect. In other words, each and any embodiment of the second aspect and each and any embodiment of the twelfth aspect is also an embodiment of the 13^{th} aspect, including any embodiment thereof.

In a 14^{th} aspect, which is also a first embodiment of the 14^{th} aspect, the problem underlying the present invention is also solved by a pigment epithelium-derived factor (PEDF) for use in a method for the treatment and/or prevention of wet macular degeneration in a subject, preferably wet age-related macular degeneration, wherein the method comprises administering PEDF and an anti-VEGF therapy to the subject.

In a second embodiment of the 14^{th} aspect which is also an embodiment of the first embodiment of the 14^{th} aspect, PEDF induces growth of non-leaking choriocapillaris.

In a third embodiment of the 14^{th} aspect which is also an embodiment of the first and second embodiment of the 14^{th} aspect, the anti-VEGF therapy comprises administration to the subject of an anti-VEGF drug.

In a fourth embodiment of the 14^{th} aspect, which is also an embodiment of the first, second and third embodiment of the 14^{th} aspect, the anti-VEGF drug is selected from the group comprising pegaptanib, ranibizumab, bevacizumab and aflibercept.

It is to be acknowledged that the disclosure of the first aspect, including each and any embodiment thereof, equally applies to the 14^{th} aspect. In other words, each and any embodiment of the first aspect is also an embodiment of the 14^{th} aspect, including any embodiment thereof.

In a 15^{th} aspect, which is also a first embodiment of the 15^{th} aspect, the problem underlying the present invention is also solved by an mRNA coding for pigment epithelium-derived factor (PEDF) for use in a method for the treatment and/or prevention of wet macular degeneration in a subject, preferably wet age-related macular degeneration, wherein the method comprises administering the mRNA coding for PEDF and an anti-VEGF therapy to the subject.

It is to be acknowledged that the disclosure of the second and 14^{th} aspect, including any embodiment thereof, equally applies to the 15^{th} aspect. In other words, each and any embodiment of the second aspect and each and any embodiment of the 14^{th} aspect is also an embodiment of the 15^{th} aspect, including any embodiment thereof.

In a 16^{th} aspect, which is also a first embodiment of the 16^{th} aspect, the problem underlying the present invention is also solved by a pigment epithelium-derived factor (PEDF) for use in a method for the treatment and/or prevention of treatment-naive quiescent choroidal neovascularization in a subject, wherein the method comprises administering PEDF and an VEGF to the subject.

In a second embodiment of the 16^{th} aspect which is also an embodiment of the first embodiment of the 16^{th} aspect, the visual acuity of the subject is normal.

In a third embodiment of the 16^{th} aspect which is also an embodiment of the first and second embodiment of the 16^{th} aspect, the subject is suffering from dry macular degeneration, preferably dry age-related macular degeneration.

In a fourth embodiment of the 16^{th} aspect which is also an embodiment of the first, second and third embodiment of the 16^{th} aspect, the subject is suffering from geographic atrophy.

In a fifth embodiment of the 16^{th} aspect which is also an embodiment of the fourth embodiment of the 16^{th} aspect, geographic atrophy is the result of anti-VEGF therapy.

In a sixth embodiment of the 16^{th} aspect which is also an embodiment of the first, second, third, fourth and fifth embodiment of the 16^{th} aspect, PEDF induces growth of choriocapillaris.

In a seventh embodiment of the 16^{th} aspect which is also an embodiment of the sixth embodiment of the 16^{th} aspect, choriocapillaris are non-leaking choriocapillaris.

It is to be acknowledged that the disclosure of the first aspect, including any embodiment thereof, equally applies to the 16^{th} aspect. In other words, each and any embodiment of the first aspect is also an embodiment of the 16^{th} aspect, including any embodiment thereof.

In a 17^{th} aspect, which is also a first embodiment of the 17^{th} aspect, the problem underlying the present invention is also solved by an mRNA coding for pigment epithelium-derived factor (PEDF) for use in a method for the treatment and/or prevention of treatment-naive quiescent choroidal neovascularization in a subject, wherein the method comprises administering the mRNA coding for PEDF and VEGF or an mRNA coding for VEGF to the subject.

It is to be acknowledged that the disclosure of the second and 16^{th} aspect, including any embodiment thereof, equally applies to the 17^{th} aspect. In other words, each and any embodiment of the second aspect and each and any embodiment of the 16^{th} aspect is also an embodiment of the 17^{th} aspect, including any embodiment thereof.

In an 18^{th} aspect which is also a first embodiment of the 18^{th} aspect, the problem underlying the present invention is solved by a pigment epithelium-derived factor (PEDF) for use in a method for the treatment and/or prevention of subclinical neovascularization in a subject, wherein the method comprises administering PEDF or an mRNA coding the PEDF to the subject.

In a second embodiment of the 18^{th} aspect which is also an embodiment of the first embodiment of the 18^{th} aspect, the subject is suffering from dry macular degeneration, preferably dry age-related macular degeneration.

In a third embodiment of the 18^{th} aspect which is also an embodiment of the first and second embodiment of the 18^{th} aspect, wherein PEDF or the mRNA coding for PEDF induces growth of choriocapillaris, preferably induces growth of non-leaking choriocapillaris.

In a fourth embodiment of the 18^{th} aspect which is also an embodiment of the first, second and third embodiment of the 18^{th} aspect, the method further comprises administering to the subject VEGF, an mRNA coding for VEGF, or an anti-VEGF drug.

In a fifth embodiment of the 18^{th} aspect which is also an embodiment of the first embodiment of the 18^{th} aspect, the subject is suffering from wet macular degeneration, preferably wet age-related macular degeneration.

In a 19^{th} aspect which is also a first embodiment of the 19^{th} aspect, the problem underlying the present invention is solved by a pigment epithelium-derived factor (PEDF) or an mRNA coding for PEDF for use in a method for the treatment and/or prevention of macular degeneration in a subject, wherein the subject is suffering from subclinical neovascularization.

In a second embodiment of the 19^{th} aspect which is also an embodiment of the first embodiment of the 19^{th} aspect, macular degeneration is age-related macular degeneration.

In a third embodiment of the 19^{th} aspect which is also an embodiment of the first and the second embodiment of the 19^{th} aspect, macular degeneration is dry macular degeneration.

In a fourth embodiment of the 19^{th} aspect which is also an embodiment of third embodiment of the 19^{th} aspect, the method further comprises administering to the subject a VEGF or an mRNA coding for VEGF.

In a fifth embodiment of the 19^{th} aspect which is also an embodiment of the first, second, third and fourth embodiment of the 19^{th} aspect, PEDF or the mRNA coding for PEDF induces growth of choriocapillaris, preferably growth of non-leaking choriocapillaris.

In a sixth embodiment of the 19^{th} aspect which is also an embodiment of the first and the second embodiment of the 19th aspect, macular degeneration is wet macular degeneration.

It is to be acknowledged that the disclosure of the first and second aspect, including any embodiment thereof, equally applies to the 18^{th} aspect and 19^{th} aspect. In other words, each and any embodiment of the first aspect is also an embodiment of the 18^{th} and 19^{th} aspect, including any embodiment thereof.

In an 20^{th} aspect, which is also a first embodiment of the twelfth aspect, the problem underlying the present invention is also solved by a pharmaceutical composition either comprising a pigment epithelium-derived factor (PEDF) or an mRNA coding for a pigment epithelium-derived factor (PEDF), wherein the pharmaceutical composition is for use in a method for treatment and/or prevention of a disease, wherein the method comprises administering PEDF to a subject and wherein treatment and/or prevention of a disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development. Preferably, the pharmaceutical composition comprises a pharmaceutically acceptable excipient or diluent.

The disclosure of the first aspect, the second aspect, the twelfth aspect, 13^{th} aspect, 14^{th} aspect, 15^{th} aspect, 16^{th} aspect, 17^{th} , 18^{th} and 19^{th} aspect, including any embodiment thereof, equally applies to the twelfth aspect, including any embodiment thereof. In other words, each and any embodiment of the first aspect, the second aspect, the twelfth aspect, 13^{th} aspect, 14^{th} aspect, 15^{th} aspect, 16^{th} aspect, 17^{th} aspect 18^{th} aspect and 19^{th} aspect, including any embodiment thereof, is also an embodiment of the 20^{th} aspect including any embodiment thereof.

In a 21^{st} aspect which is also a first embodiment of the 21^{st} aspect, the problem underlying the present invention is also solved by a pharmaceutical composition either comprising a pigment epithelium-derived factor (PEDF) or an mRNA coding for a pigment epithelium-derived factor (PEDF), wherein the pharmaceutical composition is for use in a method for treatment and/or prevention of a disease, wherein the disease is an eye disease.

The disclosure of the third, fourth, fifth, sixth, seventh, eighth, ninth, tenth, eleventh, twelfth, 13^{th}, 14^{th}, 15^{th}, 16^{th}, 17^{th}, 18^{th} , 19^{th} and 20^{th} aspect, including any embodiment thereof, equally applies to the 13^{th} aspect, including any embodiment thereof. In other words, each and any embodiment of the third, fourth, fifth, sixth, seventh, eighth, ninth, tenth, eleventh, twelfth, 13^{th}, 14^{th}, 15^{th}, 16^{th}, 17^{th}, 18^{th}, 19^{th} and 20^{th} aspect, including any embodiment thereof, is also an embodiment of the 21^{st} aspect including any embodiment thereof.

In a 22^{nd} aspect, which is also a first embodiment of the 22^{nd} aspect, the problem underlying the present invention is solved by the use of a pigment epithelium-derived factor (PEDF) or an mRNA coding for a pigment epithelium-derived factor (PEDF) for the manufacture of a medicament for the treatment and/or prevention of a diseases, wherein treatment and/or prevention of a disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The disclosure of the first aspect, the second aspect, the twelfth, 13^{th} aspect, 14^{th} aspect, 15^{th} aspect, 16^{th} aspect, 17^{th} aspect, 18^{th} aspect and 19^{th} aspect, including any embodiment thereof, equally applies to the 22^{nd} aspect. In other words, any embodiment of the first aspect, the second aspect, the twelfth, 13^{th} aspect, 14^{th} aspect, 15^{th} aspect, 16^{th} aspect, 17^{th} aspect 18^{th} aspect and 19^{th} aspect, is also an embodiment of the 22^{nd} aspect including any embodiment thereof.

In a 23^{rd} aspect which is also a first embodiment of the 23^{rd} aspect, the problem underlying the present invention is also solved by the use of a pigment epithelium-derived factor (PEDF) or an mRNA coding for a pigment epithelium-derived factor (PEDF) for the manufacture of a medicament for the treatment and/or prevention of a disease, wherein the disease is an eye disease.

The disclosure of the third, fourth, fifth, sixth, seventh, eighth, ninth, tenth, eleventh, twelfth, 13^{th}, 14^{th}, 15^{th}, 16^{th}, 17^{th} , 18^{th} and 19^{th} aspect, including any embodiment thereof, equally applies to the 22rd aspect, including any embodiment thereof. In other words, each and any embodiment of the third, fourth, fifth, sixth, seventh, eighth, ninth, tenth, eleventh, twelfth, 13^{th}, 14^{th}, 15^{th}, 16^{th}, 17^{th}, 18^{th} and 19^{th} aspect, including any embodiment thereof, is also an embodiment of the 21^{st} aspect including any embodiment thereof.

In a 24^{th} aspect, which is also a first embodiment of the 24^{th} aspect, the problem underlying the present invention is solved by a method for the treatment and/or prevention of a disease in a subject, wherein treatment and/or prevention of a disease comprises administering to the subject a therapeutically effective amount of a pigment epithelium-derived factor (PEDF) or an mRNA coding for a pigment epithelium-derived factor (PEDF) and inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

The disclosure of the first aspect, second aspect, the twelfth, 13^{th} aspect, 14^{th} aspect, 15^{th} aspect, 16^{th} aspect, 17^{th} aspect, 18^{th} and 19^{th} aspect, including any embodiment thereof, equally applies to the 24^{th} aspect, including any embodiment thereof. In other words, any embodiment of the first aspect, the second aspect, the twelfth, 13^{th} aspect, 14^{th} aspect, 15^{th} aspect, 16^{th} aspect, 17^{th}, 18^{th} and 19^{th} aspect, is also an embodiment of the 24^{th} aspect including any embodiment thereof.

In a 25^{th} aspect which is also a first embodiment of the 25^{th} aspect, the problem underlying the present invention is also solved by a method for the treatment and/or prevention of a disease in a subject, wherein treatment and/or prevention of a disease comprises administering to the subject a therapeutically effective amount of a pigment epithelium-derived factor (PEDF) or an mRNA coding for a pigment epithelium-derived factor (PEDF), wherein the disease is an eye disease.

The disclosure of the third, fourth, fifth, sixth, seventh, eighth, ninth, tenth, eleventh, twelfth, 13^{th}, 14^{th}, 15^{th}, 16^{th}, 17^{th}, 18^{th} and 19^{th} aspect, including any embodiment thereof, equally applies to the 23^{rd} aspect, including any embodiment thereof. In other words, each and any embodiment of the third, fourth, fifth, sixth, seventh, eighth, ninth, tenth, eleventh, twelfth, 13^{th}, 14^{th}, 15^{th}, 16^{th}, 17^{th}, 18^{th} and 19^{th} aspect, including any embodiment thereof, is also an embodiment of the 25^{th} aspect including any embodiment thereof. In a preferred embodiment, the treatment and/or prevention of the disease comprises inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development.

In an 26^{th} aspect, which is also a first embodiment of the 26^{th} aspect, the problem underlying the present invention is solved by a pigment epithelium-derived factor (PEDF) for use, in a subject, in a method for inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development, and wherein the method comprises administering PEDF to the subject.

The disclosure of the first aspect, the second aspect, the twelfth, 13^{th} aspect, 14^{th} aspect, 15^{th} aspect, 16^{th} aspect, 17^{th} aspect, 18^{th} aspect and 19^{th} aspect, including any embodiment thereof, equally applies to the 26^{th} aspect including any embodiment thereof. In other words, any embodiment of the first aspect, the second aspect, the twelfth, 13^{th} aspect, 14^{th} aspect, 15^{th} aspect, 16^{th} aspect, 17^{th} aspect, 18^{th} aspect and 19^{th} is also an embodiment of the 26^{th} aspect, including any embodiment thereof.

In a 27^{th} aspect, which is also a first embodiment of the 27^{th} aspect, the problem underlying the present invention is solved by an mRNA coding for a pigment epithelium-derived factor (PEDF) pigment epithelium-derived factor (PEDF) for use, in a subject, in a method for inhibiting labyrinth capillary formation, inducing growth of choriocapillaris, preferably inducing growth of non-leaking choriocapillaris, tightening choriocapillaris, inhibiting extracellular matrix formation, protecting choriocapillaris, and/or guiding vessel development, and wherein the method comprises administering PEDF to the subject.

The disclosure of the first aspect, second aspect, the twelfth, 13^{th} aspect, 14^{th} aspect, 15^{th} aspect, 16^{th} aspect, 17^{th} aspect, 18^{th} aspect and 19^{th} aspect, including any embodiment thereof, equally applies to the 27^{th} aspect. In other words, any embodiment of the first and second aspect is also an embodiment of the 27^{th} aspect including any embodiment thereof.

In an embodiment of each and any aspect, including any embodiment thereof, the subject is a human subject.

In connection with each and any aspect, including any embodiment thereof, VEGF is human VEGF.

In connection with each and any aspect, including any embodiment thereof, preferably the amount of PEDF or of an mRNA coding for PEDF is a therapeutically effective amount thereof.

In connection with each and any aspect, including any embodiment thereof, preferably the amount of VEGF or of an mRNA coding for VEGF is a therapeutically effective amount thereof.

In connection with each and any aspect, including any embodiment thereof, the mRNA coding for PEDF may actually be expressed from an insert, commonly as a cDNA insert, in a suitable non-viral vector or in a viral vector such as an Adeno-associated virus (AAV). If expressed from a non-viral or a viral vector, the insert coding for PEDF is under transcriptional control by a constitutive or a regulatable promoter.

In connection with each and any aspect, including any embodiment thereof, the mRNA coding for VEGF may actually be expressed from an insert, commonly as a cDNA insert, in a suitable non-viral vector or in a viral vector such as an Adeno-associated virus (AAV). If expressed from a non-viral or a viral vector, the insert coding for VEGF is under transcriptional control by a constitutive or a regulatable promoter.

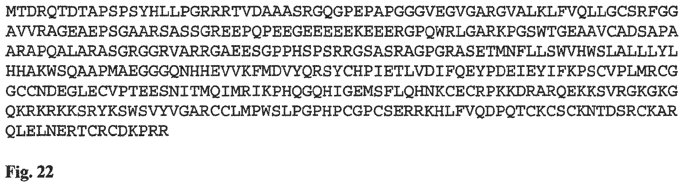

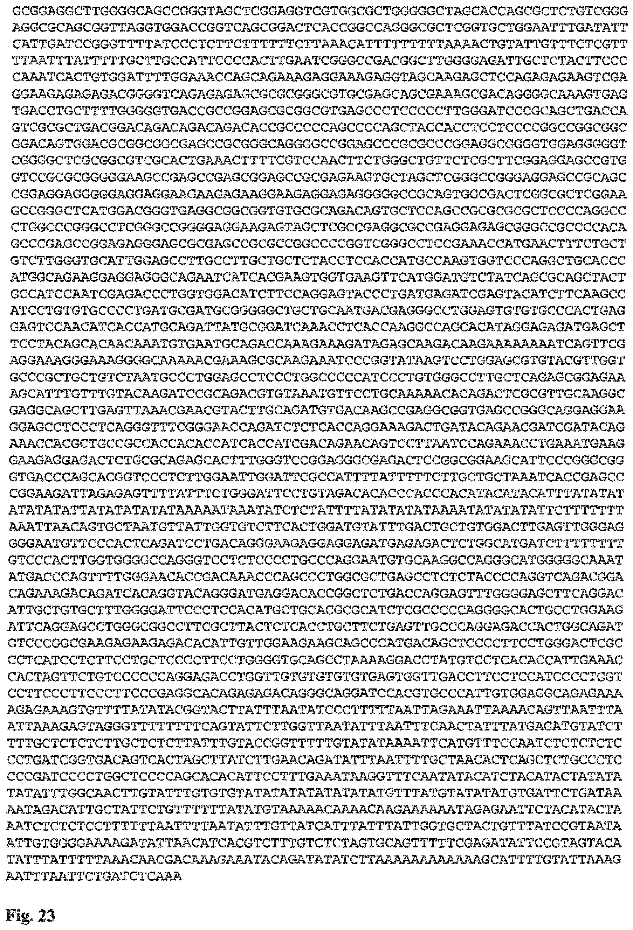

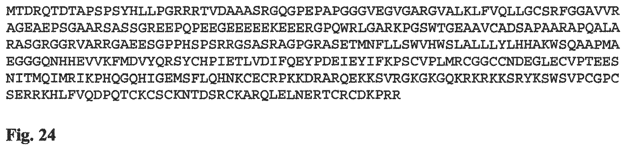

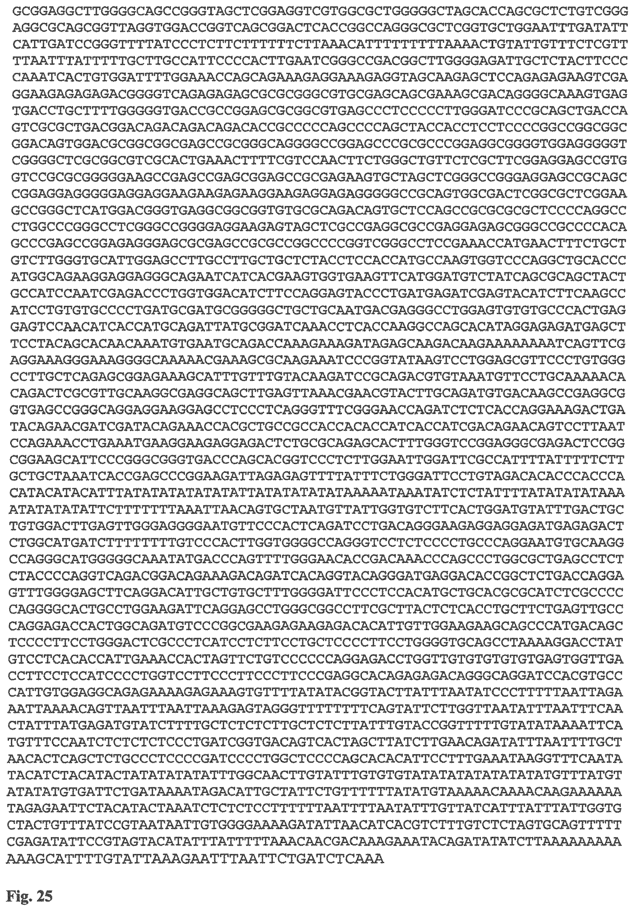

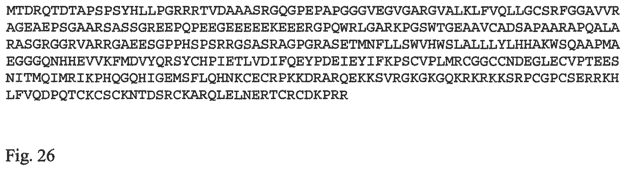

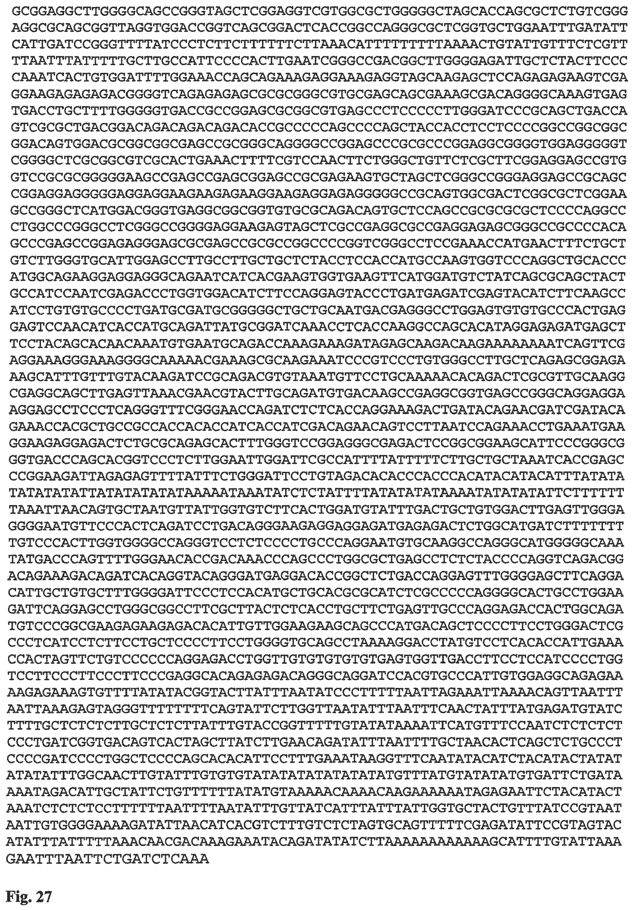

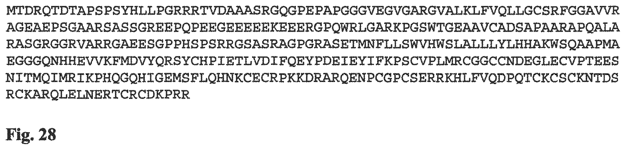

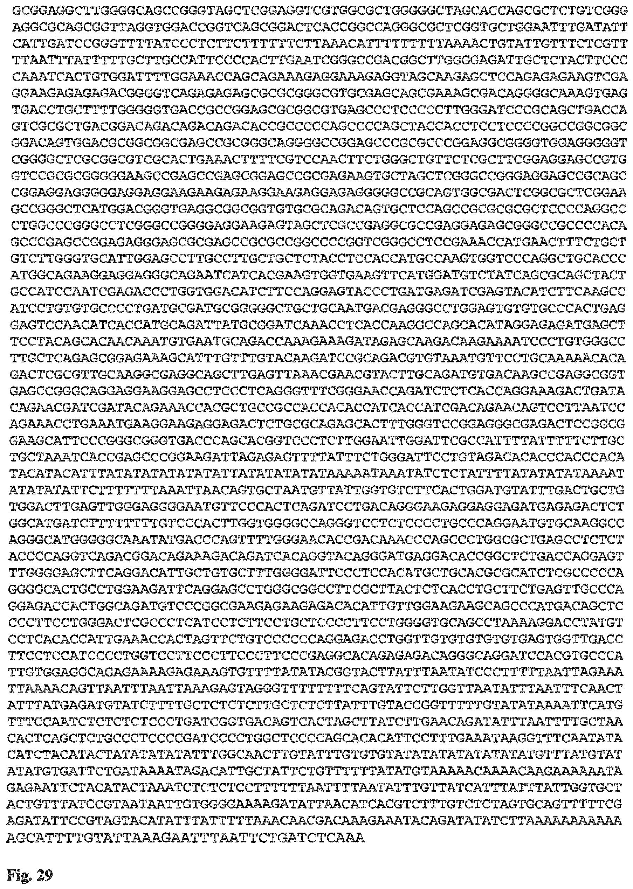

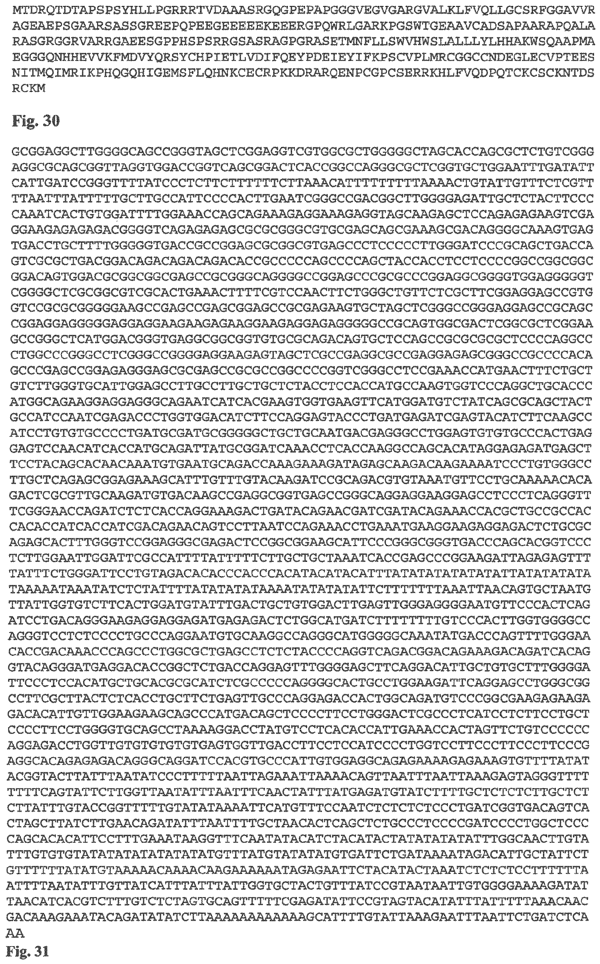

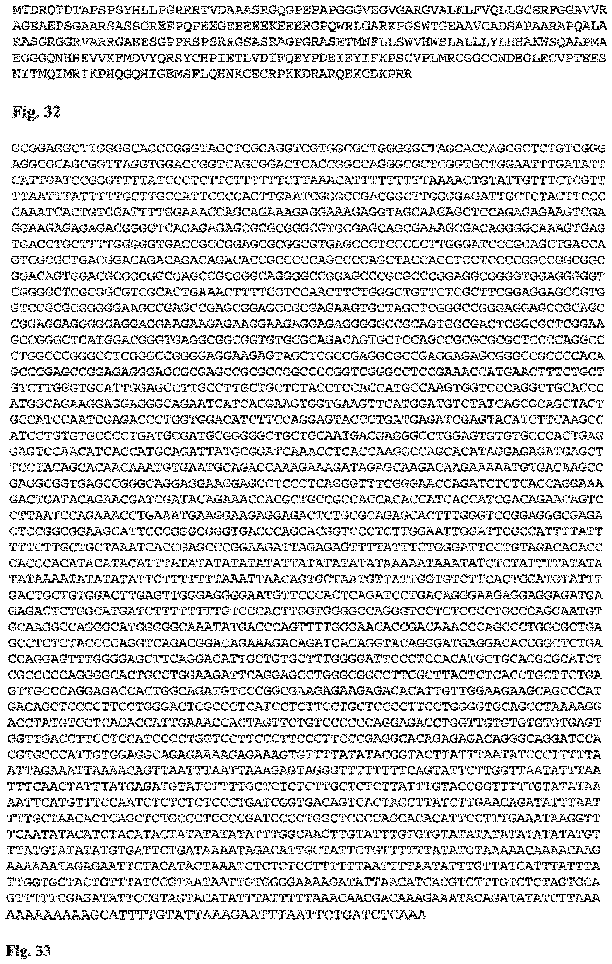

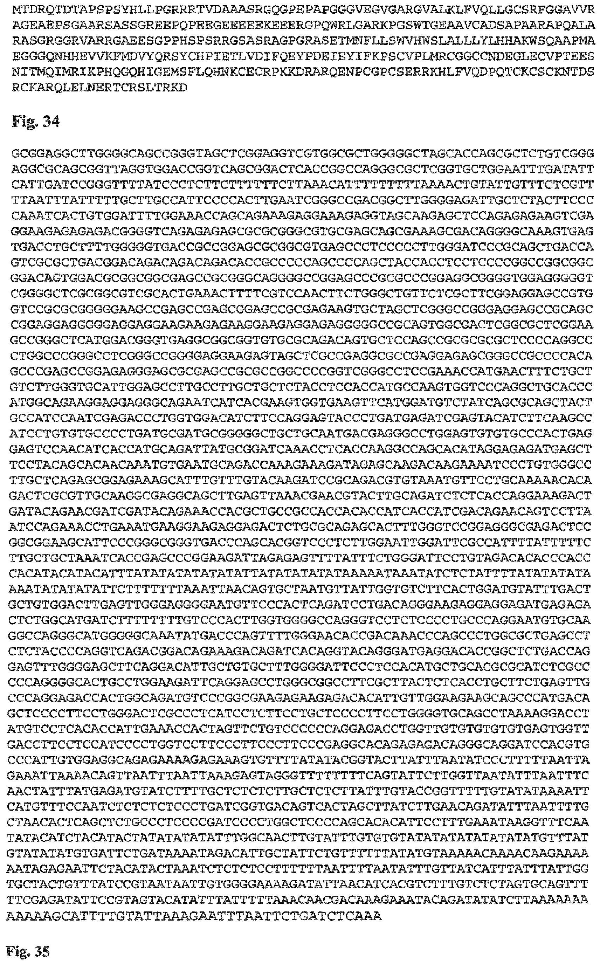

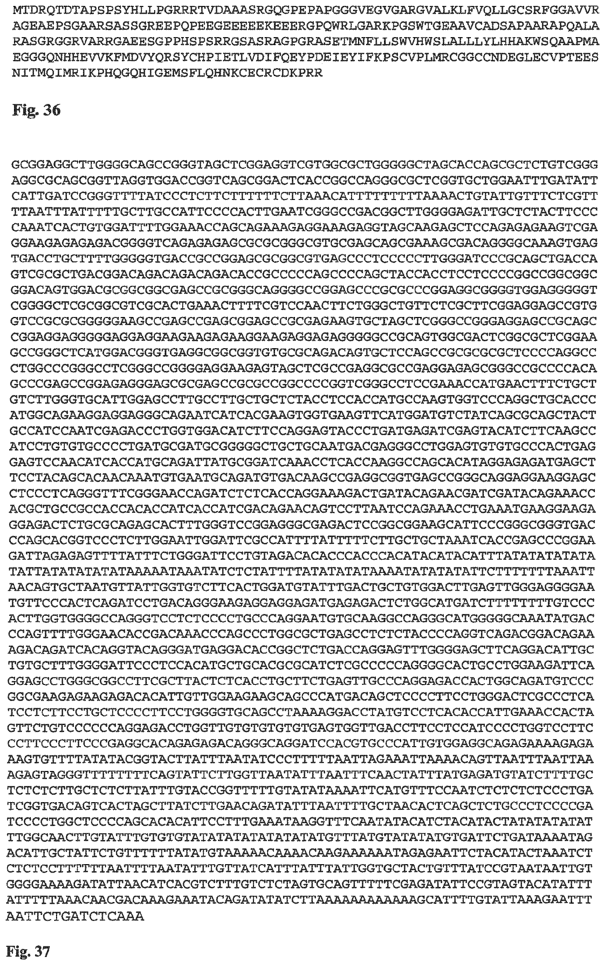

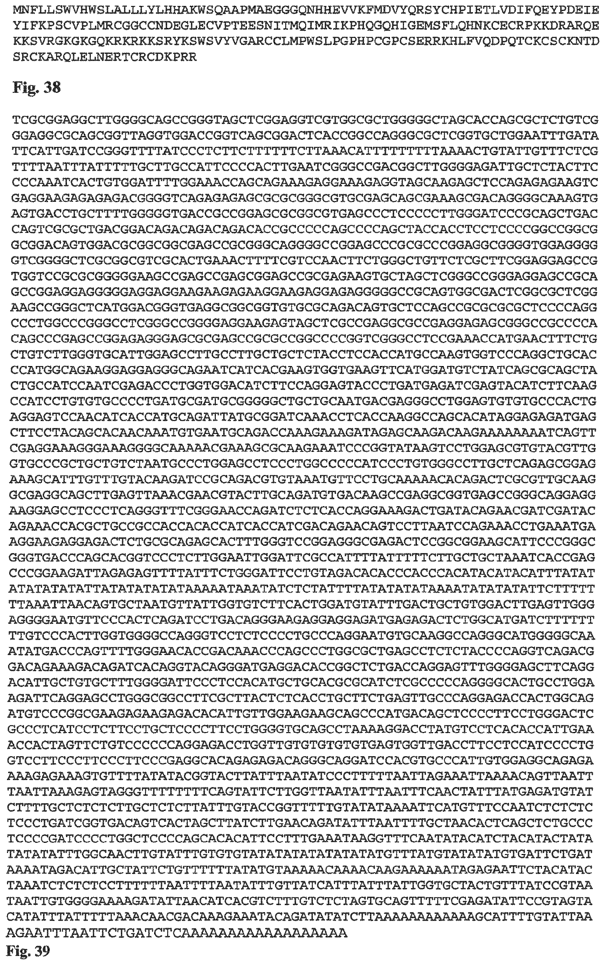

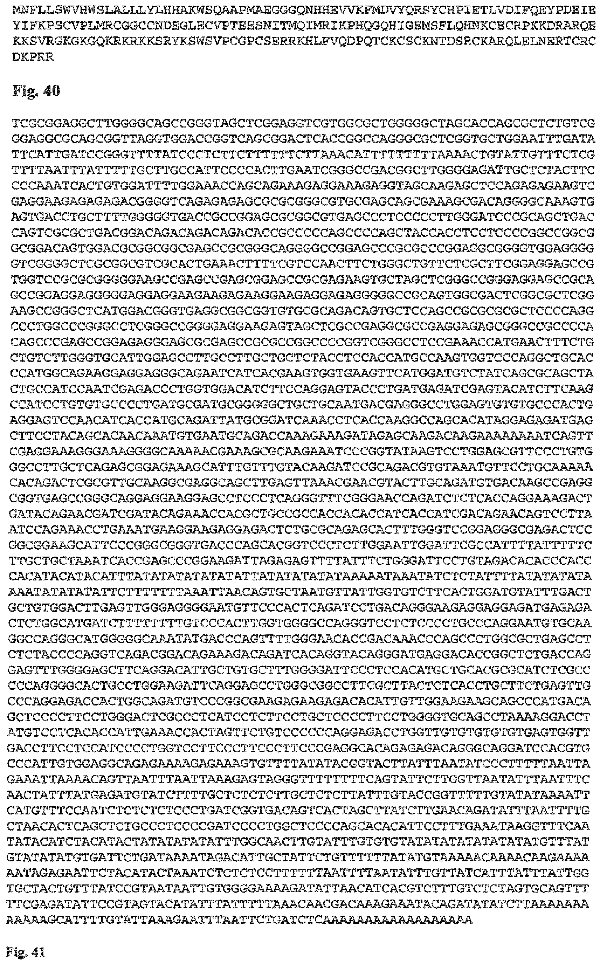

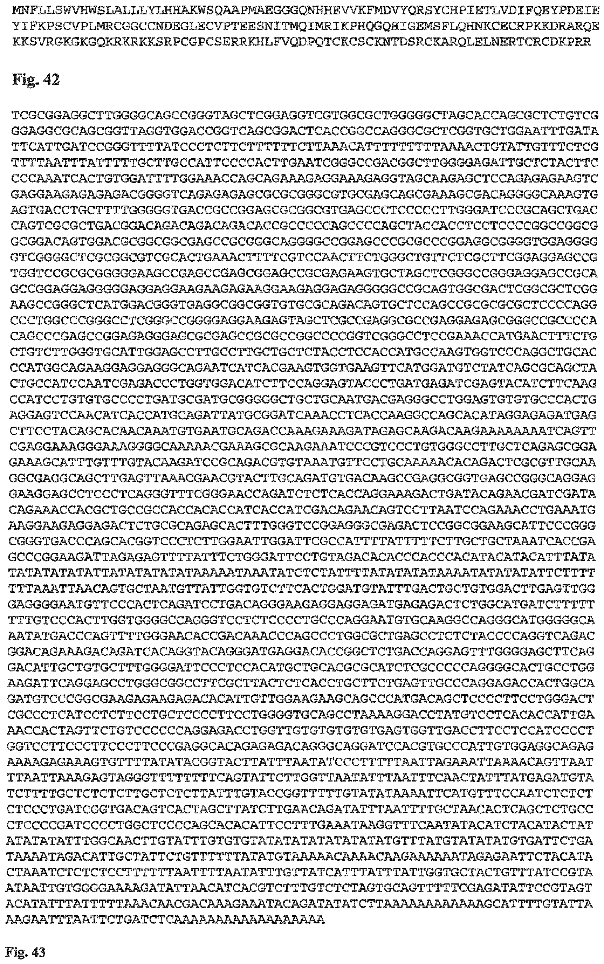

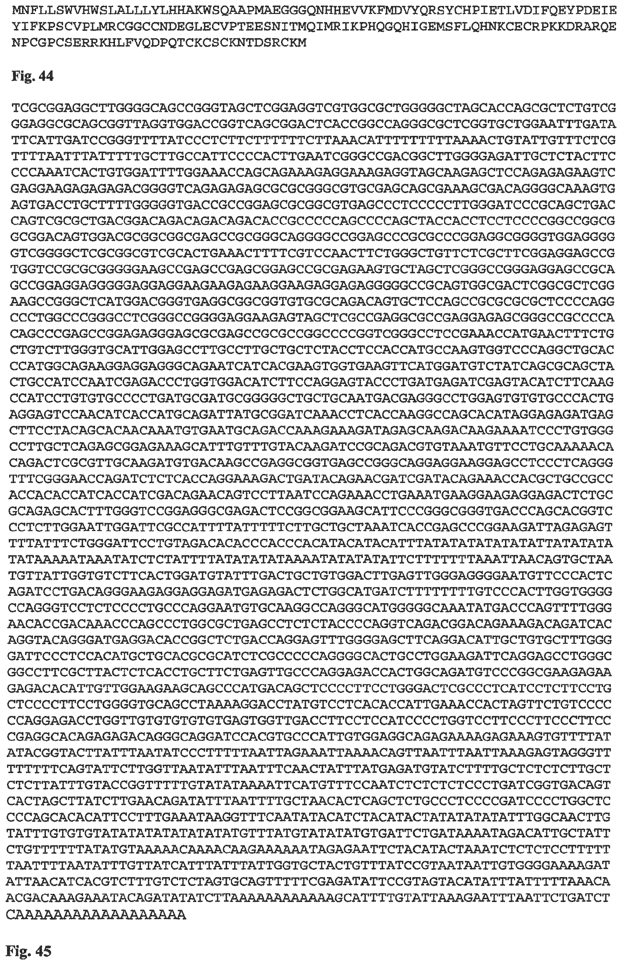

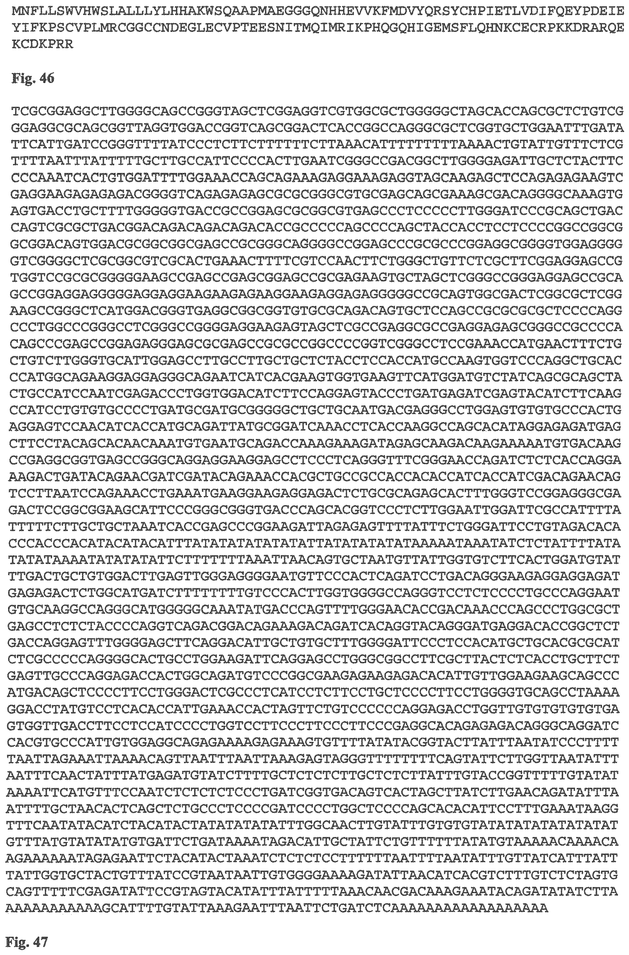

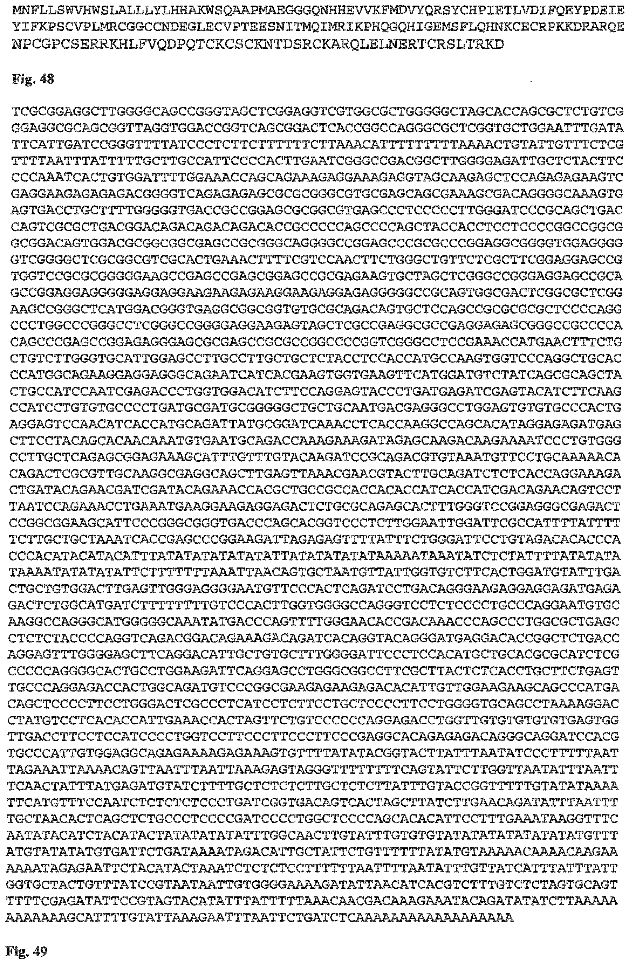

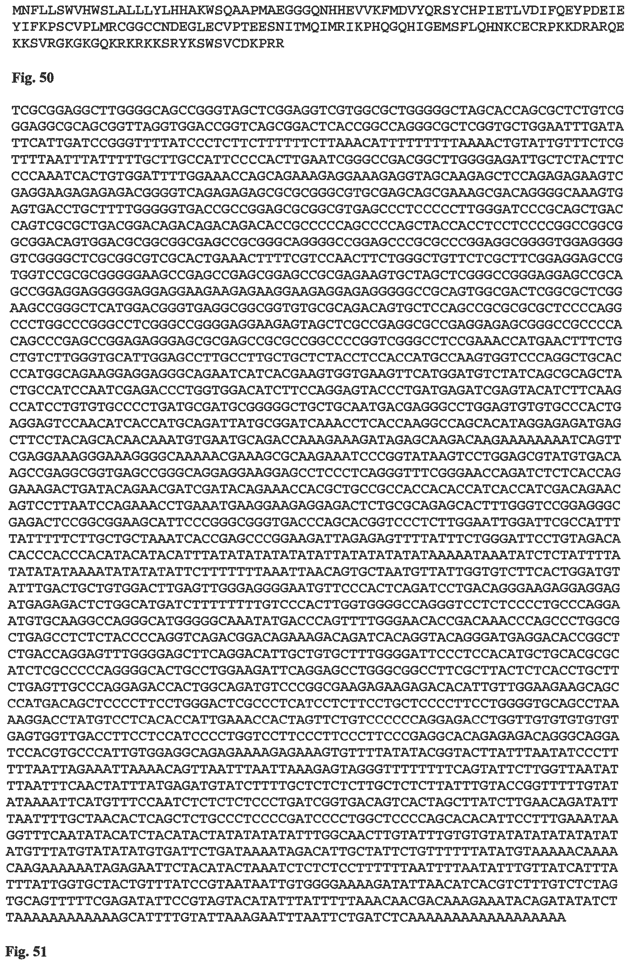

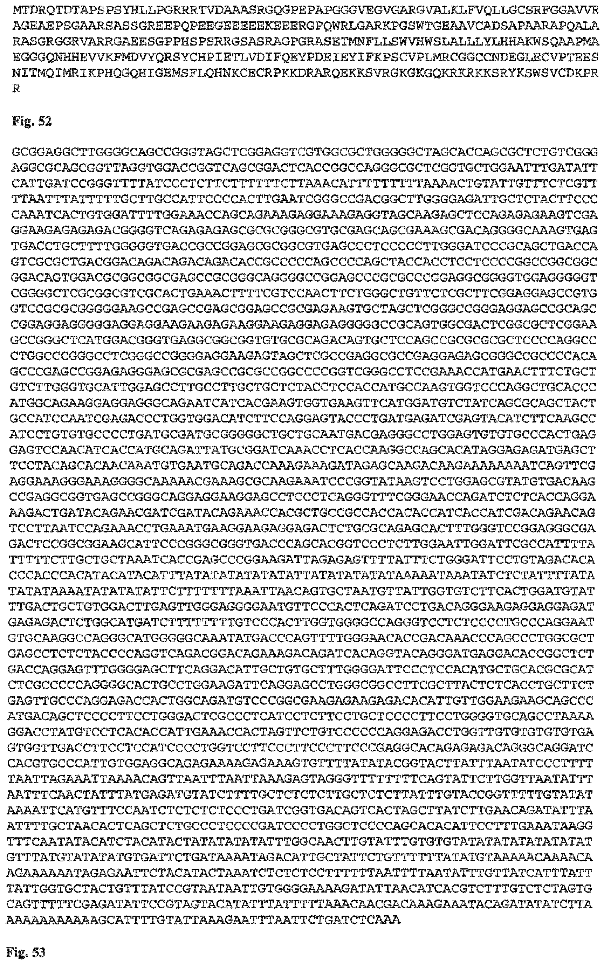

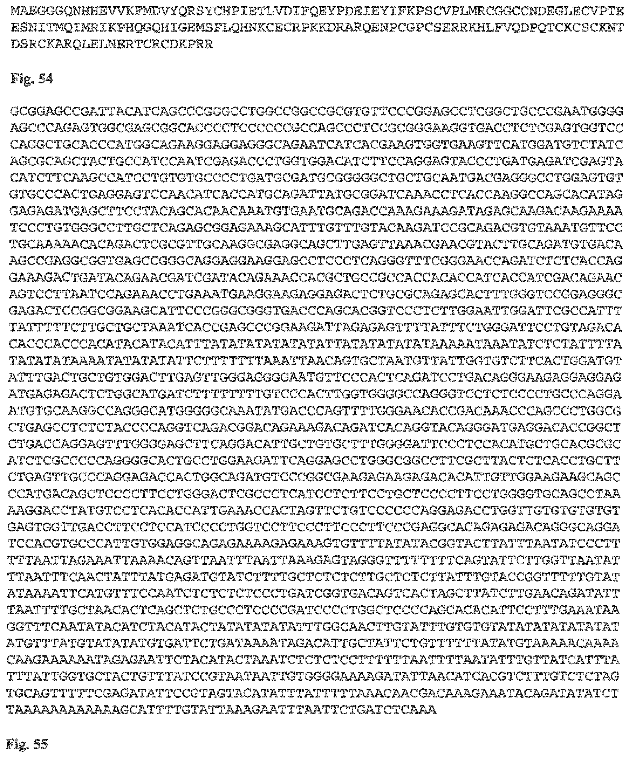

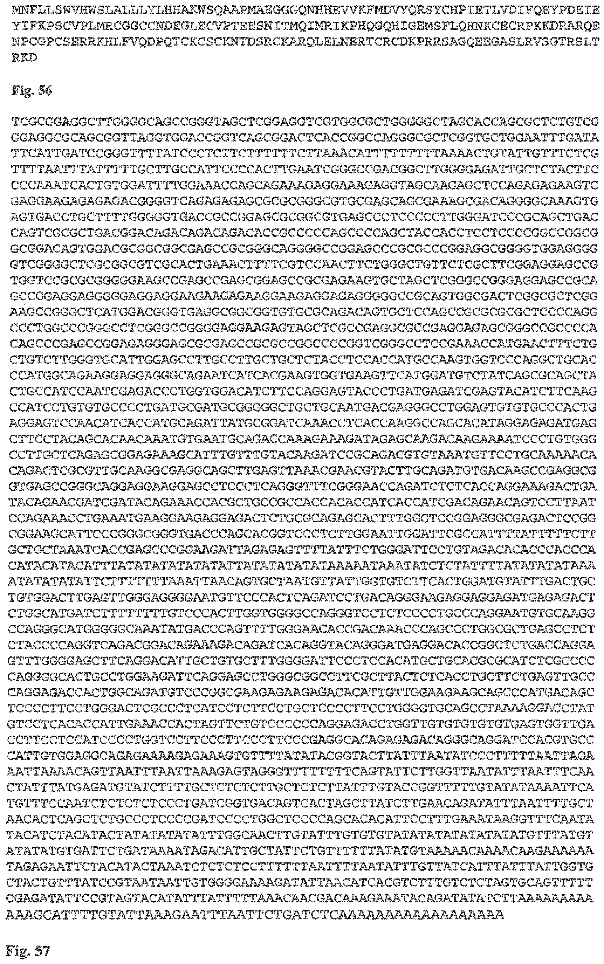

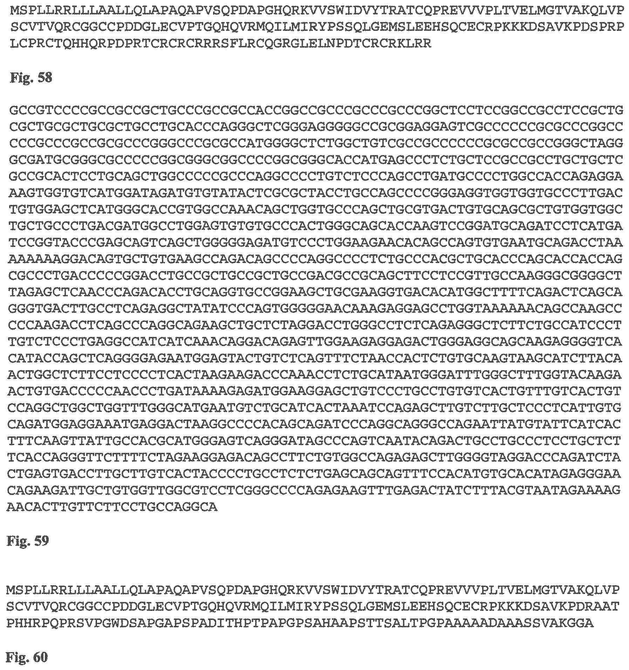

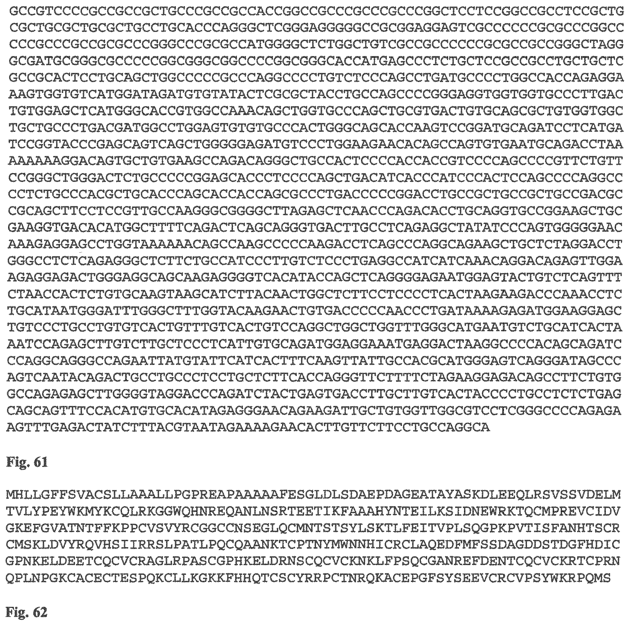

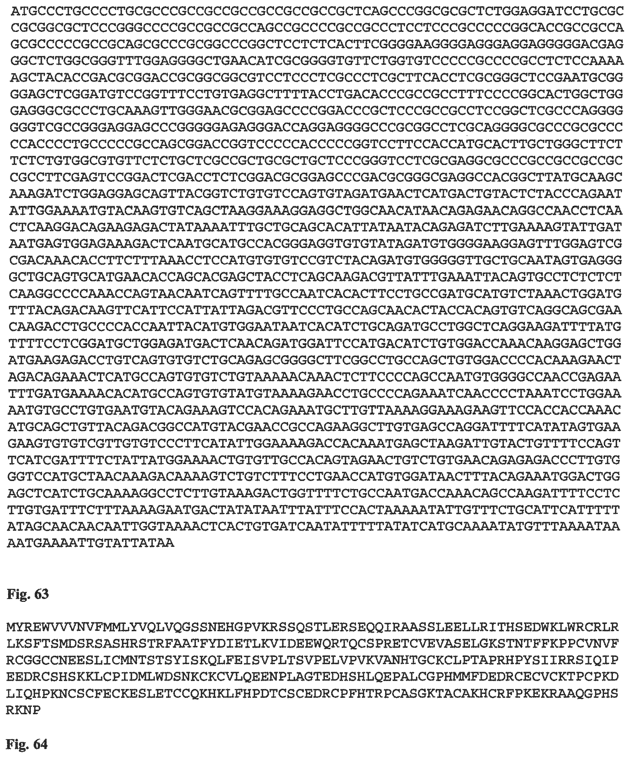

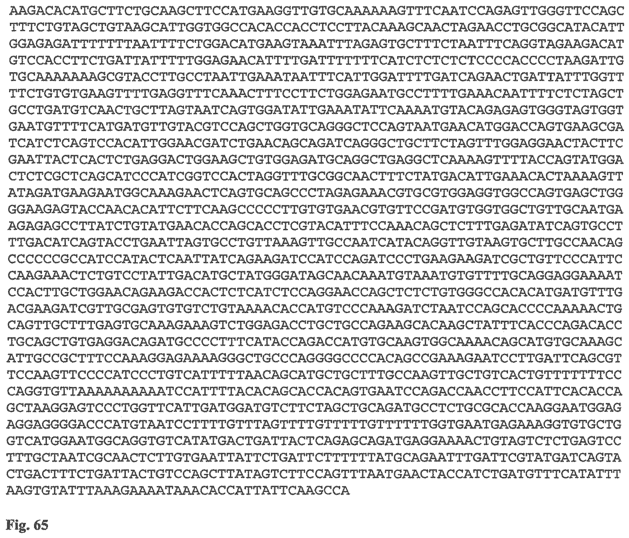

In connection with each and any aspect, including any embodiment thereof, human VEGF is selected from the group comprising human VEGF-A, VEGF-B, VEGF-C, VEGF-D and any isoform thereof. Preferred embodiments of the amino acid sequence and of the mRNA coding for VEGF-A, VEGF-B, VEGF-C and VEGF-D, including the various isoforms thereof, are disclosed in Figs. 22 to 65. It will be acknowledged by a person skilled in the art that the mRNA sequences are actually depicted as cDNA sequences from which the corresponding mRNA sequences can be derived by replacing T by U.

As preferably used herein, labyrinth capillary formation is labyrinth capillary formation in an eye, preferably in eye disease.

As preferably used herein, inducing growth of choriocapillaris comprises or is inducing growth of new choriocapillaris.

As preferably used herein, inducing growth of choriocapillaris provides choriocapillaris which are capable of replacing original choriocapillaris, preferably original choriocapillaris are diseased choriocapillaris.

As preferably used herein, tightening choriocapillaris comprises tightening pathological choriocapillaris.

As preferably used herein, inhibiting extracellular matrix formation comprises inhibition of extracellular matrix formation towards the lumen of a blood vessel and/or around a blood vessel.

As preferably used herein, protecting choriocapillaris comprises protecting choriocapillaris from the damaging effect of an anti-VEGF drug.

As preferably used herein, protecting choriocapillaris comprises protecting choriocapillaris from the damaging effect of withdrawal of an anti-VEGF drug.

As preferably used herein, guiding vessel development comprises development of a functional blood vessel, preferably a functional blood vessel from a pathological blood vessel.

As preferably used herein, PEDF is human PEDF.

As preferably used herein, non-leaking choriocapillaris is choriocapillaris the leaking characteristics of which correspond to the leaking characteristics of a healthy subject, preferably of a healthy human subject.

Preferably, subclinical choroidal neovascularization (subclinical CNV) is detected and diagnosed by optical coherence tomography angiography (see, e.g., Treister A et al. Transl Vis Sci Technol 2018 Sep; 7(5): 19).

Subclinical choroidal neovascularization (subclinical CNV) has originally been described by and is Green W et al. (Trans Am Ophthalmol Soc. 1977; 75: 180) and Sarks S et al. (Br J Ophthalmol 1973, 57:951) characterized by abnormal choroidal vessels passing through breaks in the Bruch's membrane in postmortem eyes that had not overlying hemorrhage or exudation. Yannuzzi et al. (Retina, 1992; 12:191-223) showed that indocyanine-green angiography (ICGA) could be used to detect subclinical CNV lesions as hyperfluorescence with late staining of choroidal vessels. ICGA was performed in conjunction with fluorescence angiography (FA); however, Fa was shown to be limited by lack of penetration into the choroid as well as more leakage due to lower affinity for large proteins.

It will be appreciated that a pharmaceutical composition comprises at least PEDF or an mRNA coding for PEDF and preferably a pharmaceutically acceptable excipient. Such excipient can be any excipient used and/or known in the art. More particularly such excipient is any excipient as discussed in connection with the manufacture of the medicament disclosed herein. In a further embodiment, the pharmaceutical composition comprises a further pharmaceutically active agent.

The preparation of a medicament and a pharmaceutical composition is known to a person skilled in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including eye drops, creams, lotions, salves, inhalants and the like. The use of sterile formulations, such as saline-based washes, by surgeons, physicians or health care workers to treat a particular area in the operating field may also be particularly useful. Compositions may also be delivered via microdevice, microparticle or sponge.

Upon formulation, a medicament will be administered in a manner compatible with the dosage formulation, and in such amount as is pharmacologically effective. The formulations are easily administered in a variety of dosage forms, such as the type of injectable solutions described above, but drug release capsules and the like can also be employed.

In this context, the quantity of active ingredient and volume of composition to be administered depends on the individual or the subject to be treated. Specific amounts of active compound required for administration depend on the judgment of the practitioner and are peculiar to each individual.

A minimal volume of a medicament required to disperse the active compounds is typically utilized. Suitable regimes for administration are also variable, but would be typified by initially administering the compound and monitoring the results and then giving further controlled doses at further intervals.

The pharmaceutical composition or medicament may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating, or coating methods, and typically contain about 0.1% to 75%, preferably about 1% to 50%, of the active ingredient.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form the injectable solution or suspension. Additionally, solid forms suitable for dissolving in liquid prior to injection can be formulated.

If desired, the pharmaceutical composition and medicament, respectively, to be administered may also contain minor amounts of non-toxic auxiliary substances such as wetting or emulsifying agents, pH buffering agents, and other substances such as for example, sodium acetate, and triethanolamine oleate.

The dosage regimen utilizing the nucleic acid molecules and medicaments, respectively, of the present invention is selected in accordance with a variety of factors including type, species, age, weight, sex and medical condition of the patient; the severity of the condition to be treated; the route of administration; the renal and hepatic function of the patient; and the particular aptamer or salt thereof employed. An ordinarily skilled physician or veterinarian can readily determine and prescribe the effective amount of the drug required to prevent, counter or arrest the progress of the condition.

The present invention is further illustrated by the figures, examples and sequence listing from which further features, embodiment and advantages may be taken. In connection therewith,
Fig. 1 is an electron micrograph showing a choriocapillaris of an untreated rat that was fixed directly after enucleation; the arrows mark the fenestrations in the endothelium towards Bruch's membrane; the indicated bar = 2 µm;
Fig. 2 is an electron micrograph taken fourteen hours after hypoxia; there were many filopodia-like projections within the capillary lumen which is largely reduced; the extracellular matrix surrounding the capillary was enhanced (arrowhead) and cells appeared within Bruch's membrane (arrow); the indicated bar = 2 µm;
Fig. 3 is an electron micrograph taken after hypoxia; individual filipodia of the endothelium projected into the capillary lumen were more than 10 µm long (arrows); the indicated bar = 2 µm;
Fig. 4 is an electron micrograph taken after hypoxia; were many open gaps between or within the endothelial cells (arrowhead) of the labyrinth capillaries; the indicated bar = 2 µm;
Fig. 5 is an electron micrograph taken after intravitreal PEDF injection and hypoxia; labyrinth capillaries did not develop and the lumen of the capillaries was maintained like in vivo (asterisk); the indicated bar = 5 µm;
Fig. 6 is an electron micrograph taken after hypoxia and without intravitreal PEDF injection; labyrinth capillaries developed and the lumen of the capillaries was collapsed (arrow); the indicated bar = 5 µm;
Fig. 7 is a bar diagram showing quantitative analysis of the areas occupied by the choricapillaris, by the choriocapillaris lumen and by the endothelium in ultrathin section after hypoxia and treatment with Avastin, PEDF or without treatment;
Fig. 8 is an electron micrograph of a CNV shown in a semithin section; the left and right arrow mark the extension of the CNV and the site where the RPE remains a monolayer; the photoreceptor nuclear layer is thinner and the outer segments are irregular facing the CNV;
Fig. 9 shows a representative SLO angiography image about 20 min after injection of dyes (left fluorescein angiography (FA), right (indocyanine green angiography (ICG)) for an eye six weeks after VEGF-vector injection;
Fig. 10 is a bar diagram showing the means of change of the maximal thickness of the CNV lesion area between the measurements six (pretreatment) and seven weeks after vector injection (one week after treatment) for each group; Mean standard deviations are shown * = p < 0.05, *** = p < 0.0001;
Fig. 11 is an electron micrograph showing a newly formed choriocapillaris located between Bruch's membrane (black arrowhead) and RPE; the vessel contains a red blood cell (RB); between RPE and the new vessel a new Bruch's membrane (white arrowhead) was been formed after PEDF treatment;
Fig. 12 is an electron micrograph showing a newly formed choriocapillaris located between Bruch's membrane (black arrowhead) and RPE; the vessel contains a red blood cell (RB); between RPE and the new vessel a new Bruch's membrane (white arrowhead) was been formed; a pericyte (P) is associated to this vessel which also is fenestrated (arrows) in the endothelium facing the RPE after PEDF treatment;

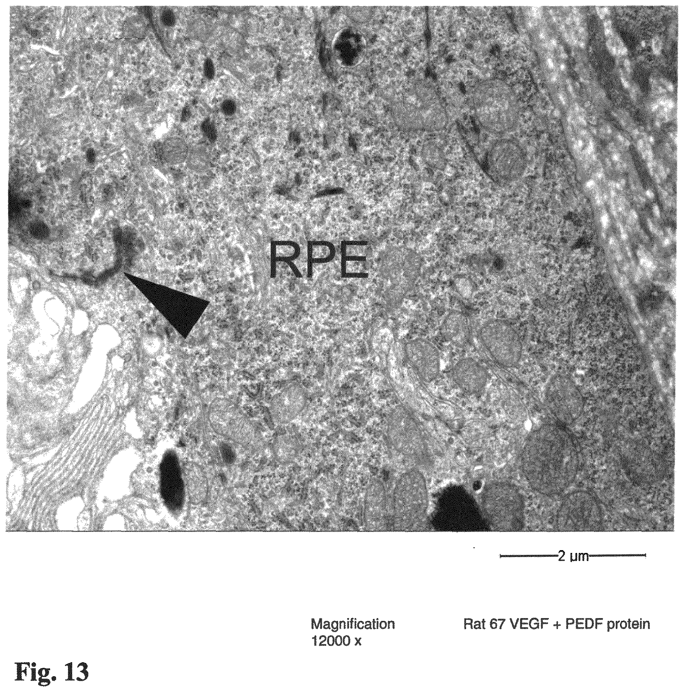

Fig. 13 is an electron micrograph showing extremely electron-dense tight junctions (arrowhead) between two RPE cells after PEDF treatment;
Fig. 14 is an electron micrograph showing several extremely electron dense and prominent junctions (arrowheads) between two endothelial of a choriocapillaris cells after PEDF treatment;

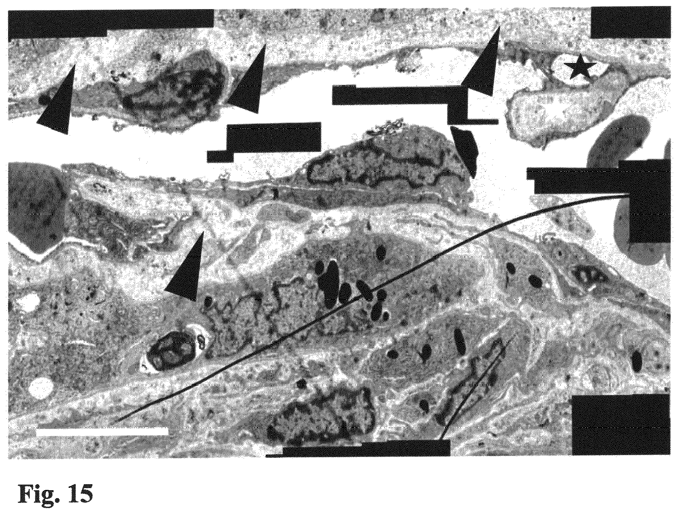

Fig. 15 is an electron micrograph shows a newly formed blood vessels which are surrounded by a thick layer of extracellular matrix (arrowheads) and separated by several layer of RPE cells from the original RPE monolayer in the absence of PEDF treatment; a protusion of extracellular matrix shifted an endothelium fold towards the vessel lumen (white asterisk); within the endothelium a large vacuole is formed (black asterisk) which is typical for pathological vessels in human CNV (Schraermeyer, Julien et al. 2015); such protrusions or vacuoles were not seen after PEDF treatment; the indicated bar = 5 µm;

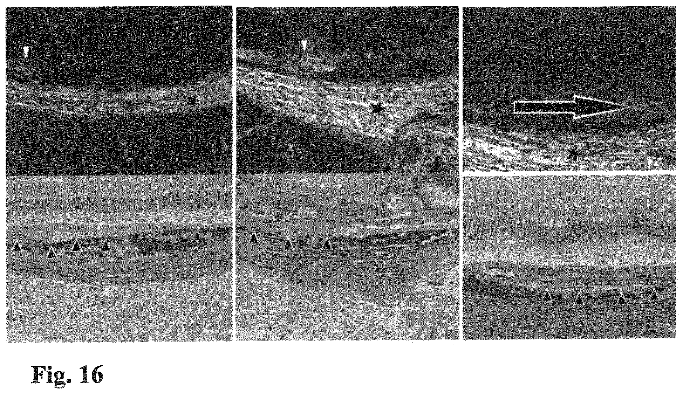

Fig. 16 is a panel of pictures taken by a polarizing microscope; the lower row shows sections from eyes with CNV's after picrosirius red staining; the upper row shows the same sections under polarized light; the black arrowheads mark the border between CNV and choroid. The white arrowheads indicate an immature collagen type III; the black arrow indicate the position of a ring of type I collagen surrounding a blood vessel after treatment with PEDF; the asterisks label the scleras which consist of mature collagen (type I); the left column shows an example from an eye after injection of PEDF and avastin; the middle column shows an eye that was only treated with avastin; and the right column shows an example from an eye treated with PEDF alone;
Figs. 17a-h show microscopic photographs of endothelial cell tube formation of HUVEC on growth factor reduced Matrigel; HUVEC were left untreated (a), or treated alone with 250 ng/mL PEDF (b), 500 ng/mL PEDF (c), 250 µg/mL Bevacizumab (d) 1 mg/mL Bevacizumab (e), 2 mg/mL Bevacizumab (f), or as a combination PEDF (250 ng/mL) + Bevacizumab (250 µg/mL) (g), PEDF (250 ng/mL) + Bevacizumab (1 mg/mL) (h); Photographs were taken after 5 hours of incubation at 37°C;
Fig. 18 is a panel of photomicrographs of a paraffin section prepared from eyes with CNVs after picrosirius red staining. The upper photomicrograph shows an overview of the section, the middle photomicrograph shows the total CNV area ("Total CNV area") and the lower photomicrograph shows the total area of VEGF expression ("VEGF positive area") imaged by means of an anti-VEGF antibody;
Fig. 19 is a box plot diagram indicating VEGF positive area as a percentage of total CNV are upon administration of AAV-VEGF vector alone, AAV.VEGF vector and vehicle, AAV.VEGF vector and PEDF protein, AAV.VEGF vector and Avastin, and a triple combination of AAV.VEGF vector, PEDF protein and Avastin;
Fig. 20 is a panel of photomicrographs of a paraffin section of eyes, more specifically of the retina upon, staining. The left photomicrograph shows a retina section prepared upon 14 hours of hypoxia and vehicle injection, and the right photomicrograph shows a retina section prepared upon 14 hours of hypoxia and injection of PEDF protein;
Fig. 21 is a box plot diagram illustrating the results shown in Fig. 20 and more specifically indicating the percentage (%) of dead ganglion cells/µl upon injection of vehicle or injection of PEFD protein.
Fig. 22 shows the amino acid sequence of vascular endothelial growth factor A, isoform a, of Homo sapiens (GenBank entry NP_001020537.2);
Fig. 23 shows that mRNA sequence of vascular endothelial growth factor A (VEGFA), transcript variant 1, of Homo sapiens (GenBank entry NM_001025366.3);
Fig. 24 shows the amino acid sequence of vascular endothelial growth factor A, isoform b, of Homo sapiens (GenBank entry NP_003367.4);
Fig. 25 shows the mRNA sequence of vascular endothelial growth factor A (VEGFA), transcript variant 2, of Homo sapiens (GenBank entry NM_003376.6);
Fig. 26 shows the amino acid sequence of vascular endothelial growth factor A, isoform c, of Homo sapiens (GenBank entry NP_001020538.2);
Fig. 27 shows the mRNA sequence of vascular endothelial growth factor A (VEGFA), transcript variant 3, of Homo sapiens (GenBank entry NM_001025367.3)
Fig. 28 shows the amino acid sequence of vascular endothelial growth factor A, isoform d, of Homo sapiens (GenBank entry NP_001020539.2);
Fig. 29 shows the mRNA sequence of vascular endothelial growth factor A (VEGFA), transcript variant 4, of Homo sapiens (GenBank entry NM_001025368.3);
Fig. 30 shows the amino acid sequence of vascular endothelial growth factor A, isoform e, of Homo sapiens (GenBank entry NP_001020540.2);
Fig. 31 shows the mRNA sequence of vascular endothelial growth factor A (VEGFA), transcript variant 5, of Homo sapiens (GenBank entry NM_001025369.3);
Fig. 32 shows the amino acid sequence of vascular endothelial growth factor A, isoform f, of Homo sapiens (GenBank entry NP_001020541.2);
Fig. 33 shows the mRNA sequence of vascular endothelial growth factor A (VEGFA), transcript variant 6, of Homo sapiens (GenBank entry NM_001025370.3);
Fig. 34 shows the amino acid sequence of vascular endothelial growth factor A, isoform g, of Homo sapiens;
Fig. 35 shows the mRNA sequence of vascular endothelial growth factor A (VEGFA), transcript variant 7, of Homo sapiens (GenBank entry NM_001033756.3);
Fig. 36 shows the amino acid sequence of vascular endothelial growth factor A, isoform h, of Homo sapiens (GenBank entry NP_001165093.1);
Fig. 37 shows that mRNA sequence of vascular endothelial growth factor A (VEGFA), transcript variant 8, of Homo sapiens (GenBank entry NM_001171622.);
Fig. 38 shows that amino acid sequence of vascular endothelial growth factor A, isoform i precursor, of Homo sapiens (GenBank entry NP_001165094.1);
Fig. 39 shows that mRNA sequence of vascular endothelial growth factor A (VEGFA), transcript variant 1, of Homo sapiens (GenBank entry NM_001171623.1);
Fig. 40 shows the amino acid sequence of vascular endothelial growth factor A, isoform j precursor, of Homo sapiens (GenBank entry NP_001165095.1);
Fig. 41 shows the mRNA sequence of vascular endothelial growth factor A (VEGFA), transcript variant 2, of Homo sapiens (GenBank entry NM_001171624.1);
Fig. 42 shows the amino acid sequence of vascular endothelial growth factor A, isoform k precursor, of Homo sapiens (GenBank entry NP_001165096.1);
Fig. 43 shows the mRNA sequence of vascular endothelial growth factor A (VEGFA), transcript variant 3, of Homo sapiens (GenBank entry NM_001171625.1);
Fig. 44 shows the amino acid sequence of vascular endothelial growth factor A, isoform m precursor, of Homo sapiens (GenBank entry NP_001165098.1);
Fig. 45 shows the mRNA sequence of vascular endothelial growth factor A (VEGFA), transcript variant 5, of Homo sapiens (GenBank entry NM_001171627.1);
Fig. 46 shows the amino acid sequence of vascular endothelial growth factor A isoform n precursor, of Homo sapiens (GenBank entry NP_001165099.1);
Fig. 47 shows the mRNA sequence of vascular endothelial growth factor A (VEGFA), transcript variant 6, of Homo sapiens (GenBank entry NM_001171628.1);
Fig. 48 shows the amino acid sequence of vascular endothelial growth factor A, isoform o precursor, of Homo sapiens (GenBank entry NP_001165100.1);
Fig. 49 shows that mRNA sequence of vascular endothelial growth factor A (VEGFA), transcript variant 7, of Homo sapiens (GenBank entry NM_001171629.1);
Fig. 50 shows the amino acid sequence of vascular endothelial growth factor A, isoform q precursor, of Homo sapiens (GenBank entry NP_001191313.1);
Fig. 51 shows that mRNA sequence of vascular endothelial growth factor A (VEGFA), transcript variant 9, of Homo sapiens (GenBank entry NM_001204384.1);
Fig. 52 shows that amino acid sequence of vascular endothelial growth factor A, isoform r, of Homo sapiens (GenBank entry NP_001191314.1);
Fig. 53 shows the mRNA sequence of vascular endothelial growth factor A (VEGFA), transcript variant 9, of Homo sapiens (GenBank entry NM_001204385.2);
Fig. 54 shows that amino acid sequence of vascular endothelial growth factor A, isoform s, of Homo sapiens (GenBank entry NP_001273973.1);
Fig. 55 shows the mRNA sequence of vascular endothelial growth factor A (VEGFA), transcript variant 10, of Homo sapiens (GenBank entry NM_001287044.2);
Fig. 56 shows the amino acid sequence of vascular endothelial growth factor A, isoform VEGF-Ax precursor, of Homo sapiens (GenBank entry NP_001303939.1);
Fig. 57 shows the mRNA sequence of vascular endothelial growth factor A (VEGFA), transcript variant 4, of Homo sapiens (GenBank entry NM_001317010.1);
Fig. 58 shows the amino acid sequence of vascular endothelial growth factor B, isoform VEGFB-167 precursor, of Homo sapiens (GenBank entry NP_001230662.1);
Fig. 59 shows that mRNA sequence of vascular endothelial growth factor B (VEGFB), transcript variant VEGFB-167, of Homo sapiens (GenBank entry NM_001243733.2);
Fig. 60 shows that amino acid sequence of vascular endothelial growth factor B, isoform VEGFB-186 precursor, of Homo sapiens (GenBank entry NP_003368.1);
Fig. 61 shows that mRNA sequence of vascular endothelial growth factor B (VEGFB), transcript variant VEGFB-186, of Homo sapiens (GenBank NM_003377.5);
Fig. 62 shows the amino acid sequence of vascular endothelial growth factor C preproprotein of Homo sapiens (GenBank entry NP_005420.1);
Fig. 63 shows the mRNA sequence of vascular endothelial growth factor C (VEGFC) of Homo sapiens (GenBank entry NM_005429.5);
Fig. 64 shows the amino acid sequence of vascular endothelial growth factor D preproprotein of Homo sapiens (GenBank entry NP_004460.1); and
Fig. 65 shows the mRNA sequence of vascular endothelial growth factor D (VEGFD) of Homo sapiens (GenBank entry NM_004469.5).

### Example 1: Exposure of eyes to hypoxia

Thirty-two eyes from 16 rats were exposed to mild hypoxia. Ischemia was modeled by incubating the eyes in DMEM at 4°C during fourteen hours after enucleation in 15 ml Falcon tubes (1 eye per tube). Hypothermia can prolong the tolerance time to an ischemic insult. The tubes were filled with 7 ml DMEM and air. They were stored horizontally to enhance the oxygen exchange between DMEM and air. After 14 hours half of the eyes were embedded into paraffin for immunocytochemistry or into Epon for electron microscopy. Twelve eyes were embedded directly after enucleation and served as controls.

The oxygen pressure was measured by a calibrated fiber optic oxygen sensor (WPI, Friedberg, Germany) which was inserted into the vitreous body of eyes in this ex vivo experiment and for comparison in eyes of living rats under anesthesia. Directly after enucleation the oxygen pressure dropped down to 2% of the in vivo concentration and then gradually increased and reached the in vivo concentration after 1 hour. After that the in vivo oxygen concentration was not undercut.

### Example 2: Measurement of the inner circumferential contour of the filipodia-like projections of the endothelial cells

Electron micrographs from choriocapillaris vessels from each plastic embedded eye were analyzed for the inner circumferential contour of the filopodia-like projections. Also, the length of the outer endothelial cell circumferential contour per sectioned vessel area was measured. The iTEM image analysis software (iTEM version 5.0; Olympus Soft Imaging Solutions, Münster, Germany) was used for the measurements. The results were analyzed in Microsoft Excel 2011 and IBM SPSS Statistics 22 software by using a nonparametric Mann-Whitney test. A p-value of less than 0.05 was considered significantly different between groups. The length of the inner endothelial cell circumferential contour increased by 58% (p<0.001) in comparison to the control group which indicates the formation of microvillar endothelial cell projections towards the vessel lumen. These vessels correspond exactly to the labyrinth capillaries in human CNV (Schraermeyer, Julien et al. 2015).

### Example 3: Effect of hypoxia on choriocapillaris

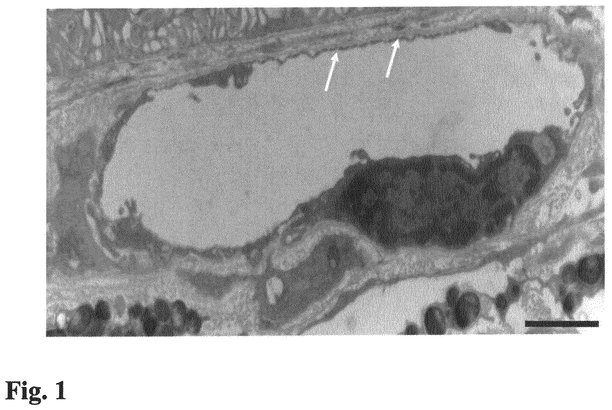

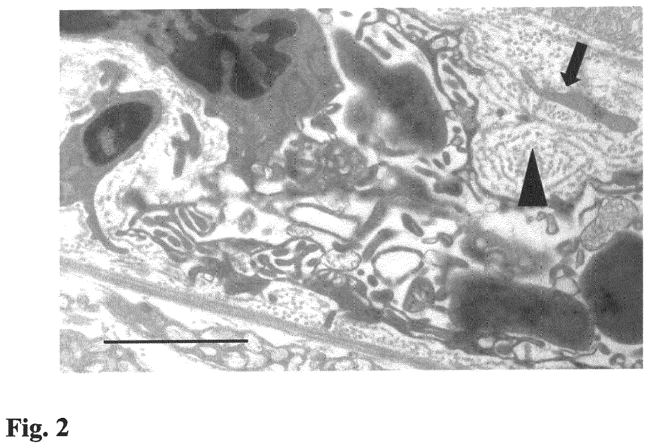

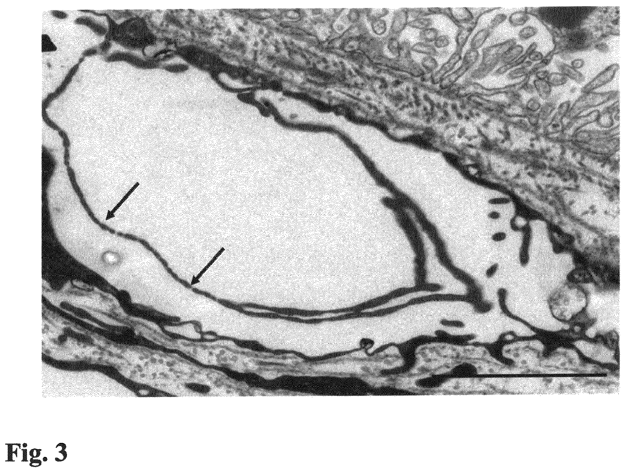

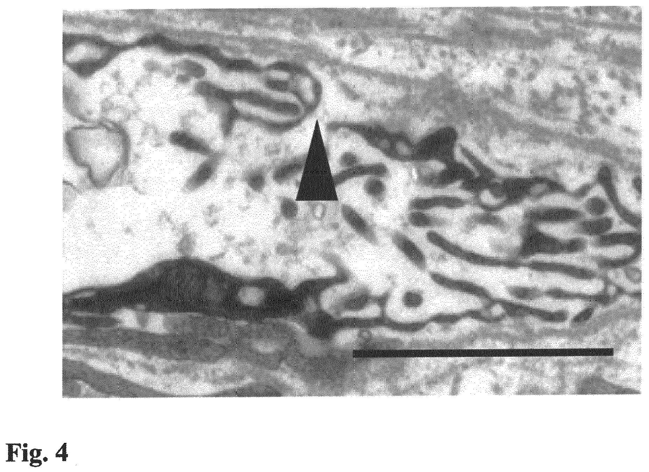

The choriocapillaris without exposure to hypoxia contains a regular thin endothelium with fenetrations towards the side of Bruch's membrane (see, Fig. 1 arrows). The lumen of the capillaries is lacking any cellular projections. Fourteen hours after hypoxia there were many filopodia-like projections within the capillary lumen. (see, Fig. 2). The extracellular matrix surrounding the capillary was enhanced (arrowhead) and cells appeared within Bruch's membrane (arrow). Individual filipodia within the capillary lumen were more than 10 µm long (see, Fig. 3 arrows). After hypoxia there were many open gaps between or within the endothelial cells (see, Fig. 4 arrowhead).

### Example 4: Expression of VEGF and HIF-1α after Hypoxia

Control and ischemic eyes were formalin-fixed and paraffin-embedded according to standard procedures. 4-µm thick sections were cut, deparaffinized and rehydrated, and boiled in a citrate buffer (pH=6.0). After three washing steps in TBS (pH=7.6), immuno-histochemical staining of **HIF-1α** and VEGF were performed according to the instructions provided by the manufacturer in a humid chamber. The slides were incubated for 120 min with the primary rabbit anti-HIF-la antibody (1:100, Abcam, Denmark) at 37°C and then processed using the DAKO REAL Detection System Alkaline Phosphatase/RED kit rabbit/mouse, then counterstained with hematoxylin and covered. The same procedure was performed for immune reactivity analysis against VEGF using a primary mouse antibody (1:50, Gene Tex, USA) and boiling in TBS buffer (pH=9.0).

In control rats, **HIF-1α** was not expressed in the choroid. After Hypoxia **HIF-1α** was detected in the choroid. VEGF in the control eyes was detected within the RPE. After 14 hours of ischemia the staining of VEGF appeared additionally in the retina and the choroid.

### Example 5: Inhibition of labyrinth capillary formation by PEDF

12 eyes were injected with 20 µg PEDF (BioVendor) and then exposed to hypoxia as described in example 1. Three eyes were injected with 0.8 µl Bevacizumab (Avastin). Six eyes were also exposed to hypoxia without any injection. Ultrathin sections of the eyes were investigated under the electron microscope.

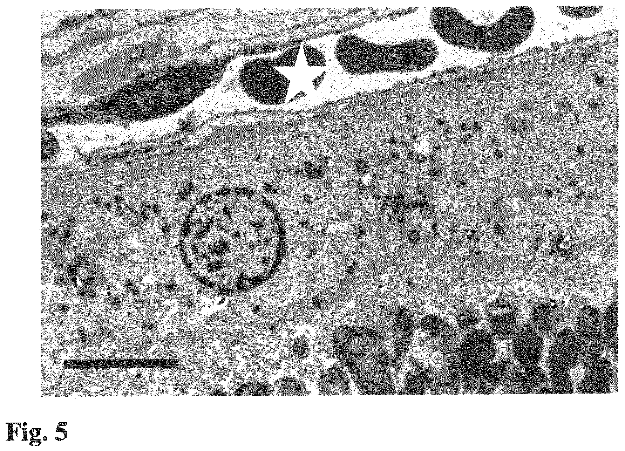

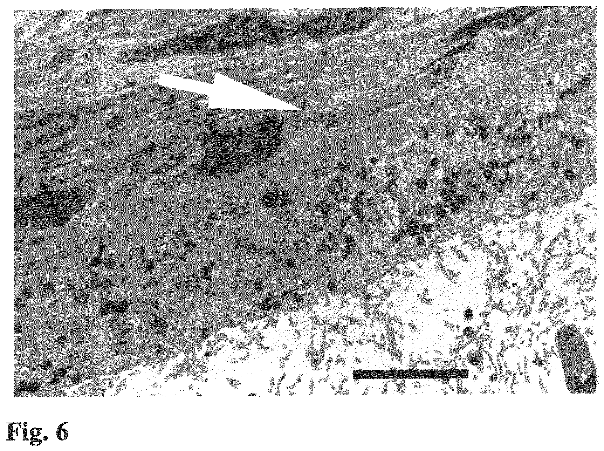

Without treatment the choriocapillaris changed into labyrinth capillaries with gaps between the endothelium as shown in Figs. 1 to 4 and collapsed leading to often complete loss of the capillary lumen (see, arrow in Fig. 5). In contrast the lumen of the choriocapillaris appeared like after in-vivo fixation and were well preserved (see, asterisk in Fig. 6).

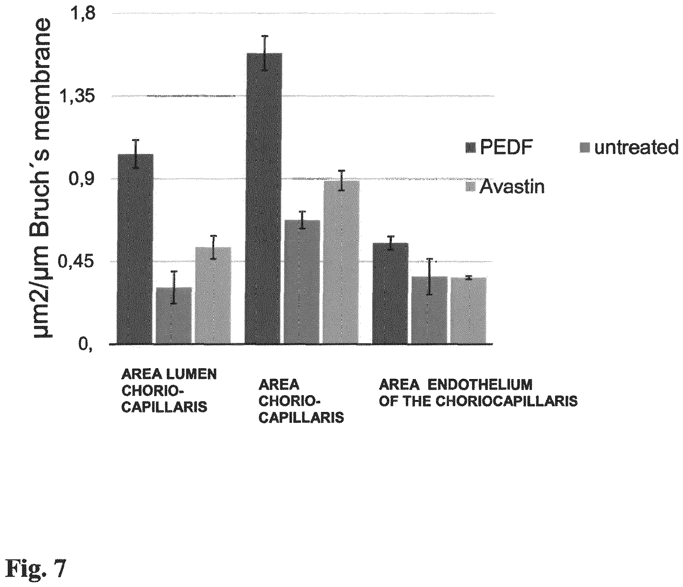

The areas enclosed by the inner and outer endothelial cell circumferential contour per sectioned vessel were measured in electron micrographs from all eyes. From these measurements the areas occupied by the entire choriocapillaris, by the lumen of the choriocapillaris and by the endothelium were calculated. PEDF not only inhibited the formation of endothelial filopodia towards the vessel lumen and gaps, it also preserved the vessel lumina significantly better than without treatment (see, Fig. 7) (p< 0.0000003) and compared to Avastin treatment (p < 0.023). Also, the area of the sectioned endothelial cells, which is likely proportional to the volume of the cells, was significantly larger compared to untreated (see, Fig. 7 right) (p < 0.03) whereas Avastin had no effect (p = 0.75).

Students t-test was performed to compare the results of the different experimental groups. For the analysis the excel software was used. The error probability was 5% (p < 0.05 statistically significant).

### Example 6: Formation of functional tight choriocapillaris and Bruch's membrane after VEGF overexpression and PEDF treatment

A new vector system was designed for this project, using the same VEGF cassette as in the adeno-vector studies before (Julien, Kreppel et al. 2008). Human VEGF-A165 cDNA, from the plasmid pBLAST49- hVEGF (Invivogen, San Diego, CA) was inserted in a state of the art AAV2 vector (subtype 4) backbone produced by Sirion Biotech GmbH (Munich, Germany). The new AAV vector has the benefit that it contains an RPE specific RPE65 promotor instead of the unspecific CMV promotor used before in adenoviral studies. These new AAV-vectors (e.g. AAV-VEGF) are less toxic and have a slower expression rate with longer expression time as compared to the adeno-vectors, favorable for long time expression studies dedicated for evaluation of drug candidates for treatment over a time frame of several months (Rolling, Le Meur et al. 2006).

### Subretinal injection of AAV.VEGF-A165 vector in rat eyes

2x109 virus particles of the AAV-VEGF vector, diluted in 2 µl PBS were sub-retinally injected in both eyes of 30 Long Evans rats. Briefly, after anaesthesia with an intraperitoneal injection of a three component narcosis (0.005 mg fentanyl, 2 mg midazolam and 0.15 mg of medetomidine/kg body weight), the pupils were dilated with 1 to 2 drops of Medriaticum drops (Pharmacy of the University of Tubingen, Germany) and a drop of topical anaesthetic Novesine (OmniVision, Puchheim, Germany) was applied. Methocel (OmniVision, Puchheim, Germany) eye drops were used to avoid drying of the eyes. Injections were performed using a surgical microscope. The sclera was first opened with a 25 G needle close to the limbus, then 2 µl of vector suspension (2 µl contain 2x109 virus particles AAV-VEGF, max. possible dose) were injected sub-retinally (pars plana) using a 10 µl NanoFil syringe with a NanoFil 34 G blunt needle (World Precision Instruments). Topical antibiotic eye drops Gentamicin-POS^{®} (Ursapharm, Saarbrücken, Germany) were applied after the injection. The anaesthesia was neutralized by subcutaneous injection of an antidote (0.12 mg naloxon, 0.2 mg flumazenil, 0.75 mg atipamezol/kg body weight).

### Intravitreal injection

### Intravitreal injection of the therapeutic substances was made 6 weeks after VEGF vector injection

For the intravitreal injections, a small incision was made into the conjunctiva at the outer corner of the eyes. The eyeball was rotated by grasping the conjunctiva with a pair of fine tweezers and gentle pulling. A volume of 5 µl was injected through the hole intravitreally using a 10 µl NanoFil syringe with a NanoFil 34 gauge bevelled needle (World Precision Instruments). After the injection, the needle remained in the eye for an additional 3 or 4 seconds to reduce reflux and was then drawn back. The eyeball was brought back into its normal position, and the antibiotic ointment was applied to the eye. The whole procedure was performed using a surgical microscope equipped with illumination. Three groups were investigated.
1) Avastin^{®} (bevacizumab; 25 mg/ml; Roche) was injected intravitreally into 20 eyes: It was purchased and aliquoted by the Pharmacy of the University Hospital of Tubingen. 100 mg of Avastin^{®} were diluted in four milliliters of the vehicle solution contains 240 mg a,a-trehalose 2 H2O, 23.2 mg Na2HPO4 H2O, 4.8 mg NaH2PO4, and 1.6 mg polysorbate 20.
2) PEDF human HEK293 recombinant protein (1 µg/µl; BioVendor) was injected intravitreally into 20 eyes.
   The pellet of the of the recombinant protein was filtered (0.4 µm) and lyophilized in 0.5 mg/mL in 20mM TRIS, 50mM NaCl, pH 7.5. According to the product data sheet, it was dissolved in deionized water (Ampuwa water) in order to obtain a working stock solution of 1 µg/µl.
3) 20 eyes were not treated

In vivo imaging (SLO/OCT, FA and ICG angiographies) and quantifications were performed according to (Wang, Rendahl et al. 2003). Subretinal AAV-VEGF leads to RPE proliferation 5 weeks to 20 months after injections, leakage can be observed starting from 2-12 months by fluorescein angiography. Therefore, scanning laser ophthalmoscopy (SLO), optical coherence tomography (OCT), fluorescein angiography (FA) and indocyanine green angiography (ICG) were performed 6 weeks after vector injection. The eyes were reinvestigated 7 weeks after injection of the VEGF vector using a SpectralisTM HRA+OCT (Heidelberg Engineering, Heidelberg, Germany) device modified for the use with animals according to protocols from (Fischer, Huber et al. 2009, Huber, Beck et al. 2009). A 78 dpt double aspheric lens (Volk Optical, Inc., Mentor, OH 44060, U.S.A.) was placed directly to the outlet of the device, an additional custom-made +3.5 dpt contact lens directly on the eyes of the rats. The rats were anaesthetized, the pupils dilated and treated with Methocel to avoid drying of the eyes and for better adherence of the 3.5 dpt lens. The ICG dye (250 µl (VERDYE, 5 mg/ml, Diagnostic Green) was injected into the tail vein, the fluorescein dye (Alcon 10% (1/10 dilution), 250 µl) was injected subcutaneously. SLO/OCT was performed ca. 2 to 5 minutes after injection for early phase and ca 15 to 20 minutes later for late phase angiography imaging. As the SLO/OCT machine is calibrated for the use with human eyes, the dimension in the x and y axis are not corrected for use in rats. Dimensions in the z axis, like retinal height, are displayed properly. Therefore, measurements of CNV hyper-fluorescent areas in the angiography measurements performed here are presented in arbitrary units (au) and not in µm using the original Heidelberg calibration. Quantification of the thickness measurements performed in OCT data sets is displayed in µm as they lay in the z direction of the beam.

### Processing of the eyes for histology

For electron microscopy (EM), whole eyes were fixed in 5 % glutaraldehyde in 0.1 M cacodylate buffer (pH 7.4) over night. Then, it will be possible to cut the lesion area, as it was visible in the angiography, and to embed it.

### Statistics

Students t-test was performed to compare the results of the treated animals with the control groups. For the analysis the excel software was used. The error probability was 5% (p < 0.05 statistically significant). To avoid the multiple comparisons problem, the results were corrected using the Holm-Bonferroni method.

### Investigation of CNV 6 weeks after subretinal injection of AAV.VEGF-A165 in rat eyes by angiography

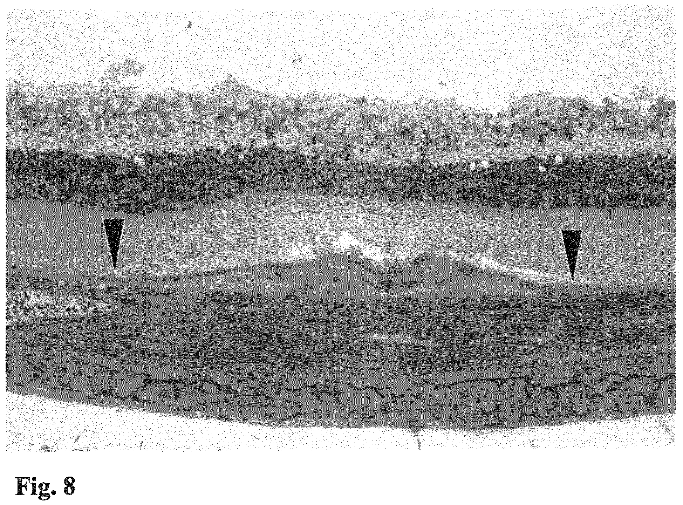

The AAV-VEGF triggered rat CNV model showed a fully grown CNV 6 weeks after VEGF transduction, as documented by in vivo imaging. A representative image is presented in (see, Fig. 8).

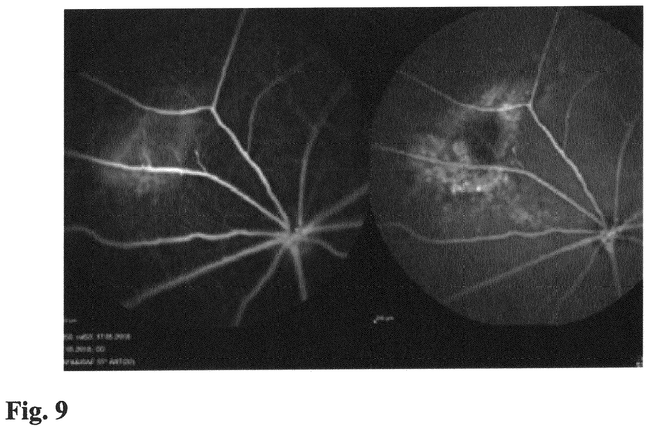

All 60 eyes overexpressing VEGF showed typical CNV lesion-like hyper-fluorescence in FA and ICG imaging (Fig. 9) meaning that the VEGF transduction efficacy was 100%.

In the following, eyes successfully transduced with VEGF vector and showing CNV-like lesions will be termed "CNV eyes", the CNV-like lesions "CNV lesion".

All eyes were investigated by angiography. Most CNV lesions showed a typical ring-shaped pattern in both the FA and ICG angiographies. A central hypo-fluorescent area was surrounded by a bright hyper-fluorescent ring especially in FA images (see, Fig. 9 left panel). This pattern correlated well with the OCT analyses showing pronounced subretinal lesions in the hyper-reflective areas. In contrast, the ICG signal showed a rather spotty pattern that usually stretched over a larger area around the hypo-fluorescent center of the lesion.
ICG is a dye that has a very long half-life and binds to luminal proteins. Therefore, it can be recorded at several time points after single intravenous injection if it is retained within the tissue. This occurs, e.g. with protein leakage from CNV vessels into the surrounding tissue. As shown in Fig. 9 (see, right panel, green channel) and in contrast to the FA signal, the ICG hyper-fluorescence shows a rather spotty pattern around the CNV lesion that spreads over time (within 20 minutes, but also at reinvestigation of ICG without additional dye injections at later time points, here one week after the first angiography session). Finally, this leads to formation of larger fields of single hyper-fluorescent highlights that can cover the whole background of the eye at late time points. These patterns, however, do not dramatically change directly after injection of additional ICG dye.

### Reduction of the thickness of the CNV lesion area by PEDF treatment

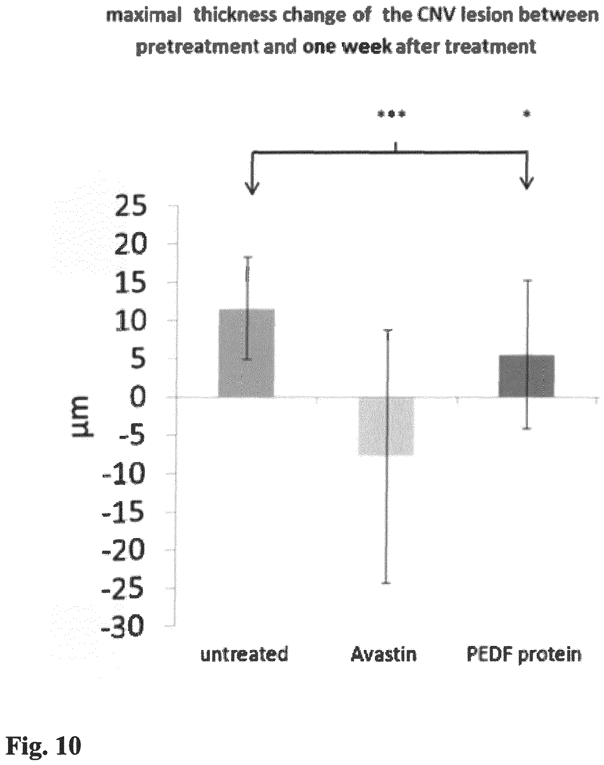

For each eye the area of the whole CNV lesion area (detected by SLO angiography) was screened by OCT. The area of maximal thickness of the lesion was determined and imaged. In these images the maximal thickness was measured. To analyze the changes caused by the treatment with the different agents the differences of the measurement values for each eye for the analysis seven weeks after the subretinal injection of the vector (one week after treatment) and the corresponding values for the six weeks analysis (before treatment) were determined. PEDF inhibited cellular proliferation and fibrosis and therefore reduced the thickness of the CNV significantly compared to the untreated group, but the blood vessels did not collapse completely as in the Avastin group. Thus, the CNVs became flatter in the Avastin group (see, Fig. 10).

### Effects of PEDF on new formation of a healthy choriocapillaris, Bruch's membrane and junctional complexes

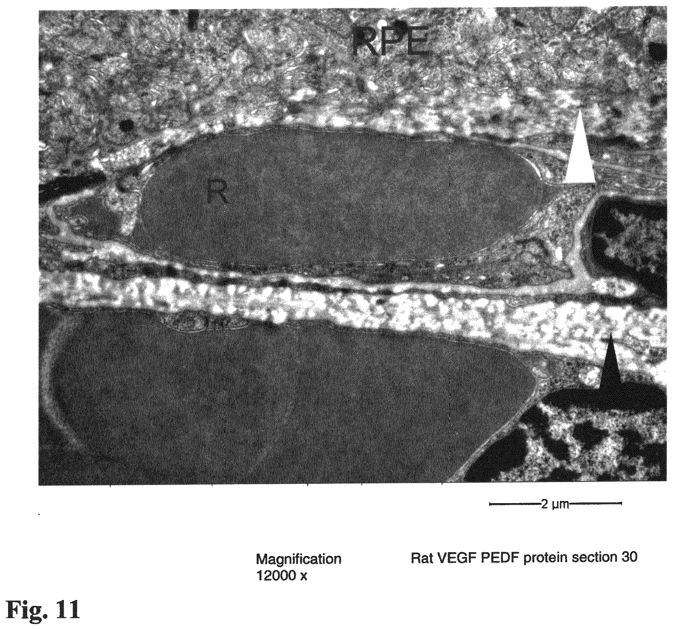

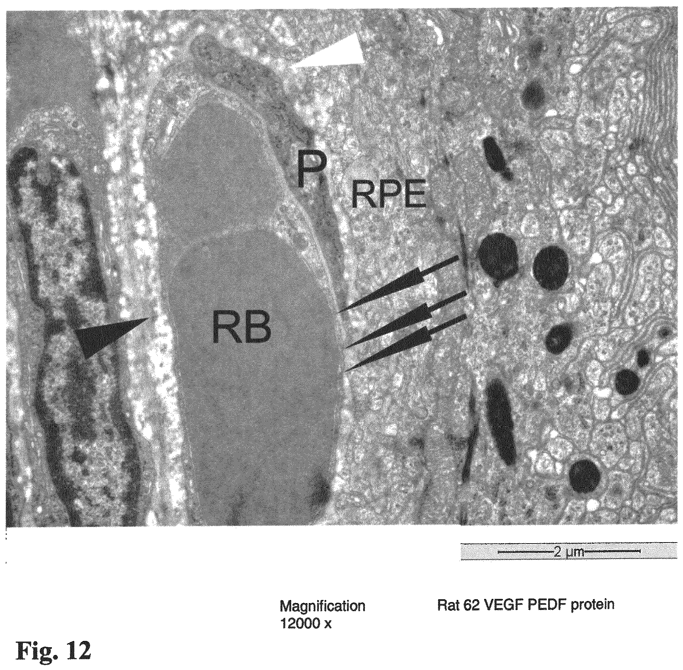

Eyes after PEDF protein treatment were investigated by electron microscopy and eyes after VEGF vector injection without PEDF treatment were used as controls. The most prominent effects of PEDF treatment were that the newly formed choriocapillaris was very similar to the healthy choriocapillaris without any treatment. The newly formed vessels were directly located below the RPE and formed a new Bruch's membrane (see, Fig. 11). The endothelial cells were thin as in healthy vessels, were associated with pericytes, developed fenestrations (see, Fig. 12) and did not grow into the subretinal space.

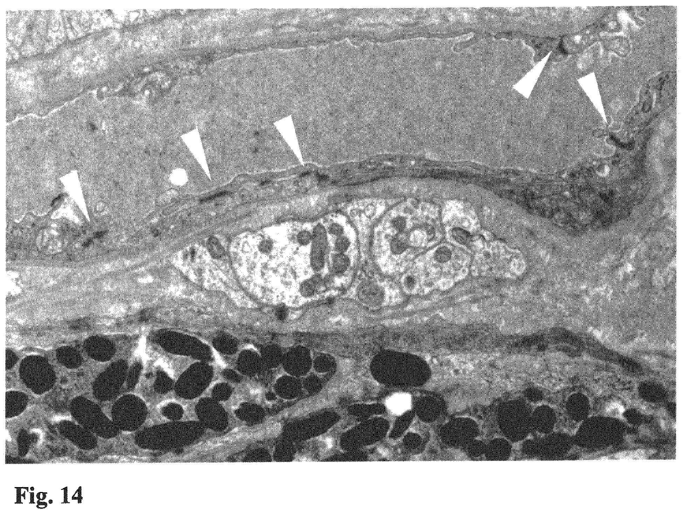

In addition, junctional complexes between retinal pigment epithelial cells (Fig. 14) and the endothelial cells of the choriocapillaris (Fig. 15) were dramatically enlarged and electron dense compared to only VEGF vector treated eyes. These complexes consist of adherent junctions and tight junctions. Tight junctions also appeared between endothelial cells of the choriocapillaris although they have not been reported in these vessels before. It is generally accepted that the blood retina barrier is built up by the tight junctions of the retinal vessels and the tight junctions of the retinal pigment epithelium. The effect on the junctions is mediated by PEDF in combination with VEGF over expression.

PEDF also reduced dividing of RPE cells and inhibited the formation of intravascular protrusions containing extracellular matrix (see, Fig. 2 and 15). This phenomenon was also described in a rabbit model of CNV (Julien, Kreppel et al. 2008). Such protrusions were not seen after PEDF treatment, which caused formation of monolayered basement membranes in newly formed vessel whereas without treatment the basement membranes were multilayered. Also, the breakthrough of newly formed blood vessels into the subretinal space and retina did not occur after PEDF treatment but were seen without PEDF injection.

### Example 7: Effect of a combination of PEDF and anti-VEGF drug

A combination of PEDF and an anti-VEGF drug, for example Bevacizumab (Avastin), acts synergistically and is supporting the coordinated growth of new functional vessels and also improves the formation of fenestrations in the newly formed choriocapillaris.

### Example 8: PEDF reduces formation of extracellular matrix in CNV

As shown in this example, PEDF reduced formation of extracelluar matrix in CNV. Therefore, scarring which is typical for CNV is minimized and therefore the distance for supply of oxygen and nutrition from the newly formed vessels towards the PRE and photoreceptors was shortened.

### Methods

A subretinal injection of 2 µl AAV.VEGF-A¹⁶⁵ (2x10⁹ virus particles of the AAV-VEGF vector, diluted in 2 µl PBS) was performed in 48 eyes of Long Evans rats in order to induce a CNV. After three weeks, the CNV development was checked by *in vivo* examinations and 100% of the eyes (n=48 eyes) showed a CNV.

Directly after the *in vivo* investigation, the eyes were intravitreally treated with 4 µl of PEDF protein (10 µg) "group 1" (n= 12 eyes) or avastin (50 µg) "group 2" (n= 12 eyes) or as combination therapy (PEDF protein (10 µg) + avastin (50 µg)) "group 3" (n= 12 eyes). Untreated eyes served as control "group 4" (n= 12 eyes).

At week 6 a second intravitreal treatment of PEDF protein or avastin or a combination of both proteins was performed as described for the week 3. One week later at week 7, the effect on maturation of the extracellular matrix in the CNV was evaluated by polarization microscopy.

Paraffin sections of the eyes were stained according to the following protocol.

### Picrosirius Red Stain Protocol

1. Deparaffinize and hydrate in distilled water
2. Stain in Weigerts Hematoxylin for 8 minutes
3. Rinse well in distilled water
4. Place in solution A for 2 minutes
5. Distilled water rinse
6. Place in solution B for 60 minutes
7. Place in solution C for 2 minutes
8. 70% ethanol for 45 seconds
9. Dehydrate, clear and mount
10. Slides are evaluated under the polarized microscope (Axioplan, Zeiss). This method allows to discriminate different types of collagen by colour. Type I (Red, Orange); type III (Yellow, Green).

### Results

The results are shown In Fig. 16.

Within the area of choroidal neovascularization collagen appeared green under the polarizing microscope after the injection of PEDF and Avastin (Fig. 16, left column). Also, after injection of avastin alone the collagen was greenish but the amount of collagen was largely enhanced (Fig. 16, middle column) compared to injection of both proteins. The green color indicated that the collagen was type III which is typical for fibrotic tissues. After injection of PEDF alone the collagen was orange and surrounded the blood vessels as a thin layer (Fig. 16, arrow, right column). This indicated that the collagen had matured and the new formation of the extracellular matrix and vessels had stopped. Greenish collagen was not seen after the specimen was turned by 360 degree after PEDF injection. Without treatment the collagen was greenish and occupied the majority of the CNV area (not shown) similar to the results after avastin injection (Fig. 16, middle column).

### Example 9: Mimicking human AMD by subretinal or intravitral injection of VEGF

Hundred ng VEGF protein (hVEGF Sigma) in 2 µl PBS were injected subretinally or intravitreally into eyes of Long Evans rats. For controls, only PBS was injected.

The eyes were investigated after 1 and 24 hours by electron microscopy and immunocytchemistry. The choriocapillaris changed into labyrinth capillaries as shown in Figs. 2 to 4 and an earlier publication in human CNV's (Schraermeyer, Julien et al. 2015). In addition, there was a prominent augmentation of the extracellular matrix within Bruch's membrane and around the choricapillaris. The protrusion of extracellular matrix which induced the endothelial invaginations into the vessel lumen as shown in Fig. 15 was also present. The synthesis of basement membranes of RPE and vessels was enhanced to multilayers. In addition, the RPE was highly activated and migrated out of the monolayer. Within the choriocapillaris, thrombocytes were activated red blood cells were lysed probably by complement activation and also developed stasis. All these findings were surprisingly already observed 1 - 24 hours after injection, lacked in the control group and mimicked the findings seen in human eyes suffering from AMD

### Example 10: In vitro effects of PEDF, Bevacizumab or a combination of both PEDF and Bevacizumab on angiogenesis

In vitro effects of PEDF, Bevacizumab (Avastin) or a combination of both PEDF and Bevacizumab (Avastin) on angiogenesis were determined in an endothelial cell tube formation assay. The endothelial cell tube formation assay is a classical in vitro assay to study angiogenesis and anti-angiogenic effects of potential drug candidates.

### Methods: Endothelial cell tube formation assay

96-well plates (Corning, USA) were pre-coated with 60 µL of growth factor reduced Matrigel (BD Biosciences, USA), and HUVEC cells (13000 cells/well) in ECGM Media (Promocell, Germany) were seeded onto the plates. The wells were supplemented with: PEDF alone (250 ng/ml, 500 ng/ml), Bevacizumab alone (Avastin; Genentech, Inc., South San Francisco, CA) (250 µg/mL, 1 mg/mL, 2 mg/mL) and together at a concentration of PEDF (250 ng/mL) + Bevacizumab (250 µg/mL) and PEDF (250 ng/mL) + Bevacizumab (1 mg/mL) to determine the effects of these molecules on endothelial cell tube formation. After incubation for 5 hrs at 37°C the tube formation was analysed in the wells using a Leica DM IL LED inverted phase contrast microscope.

### Results

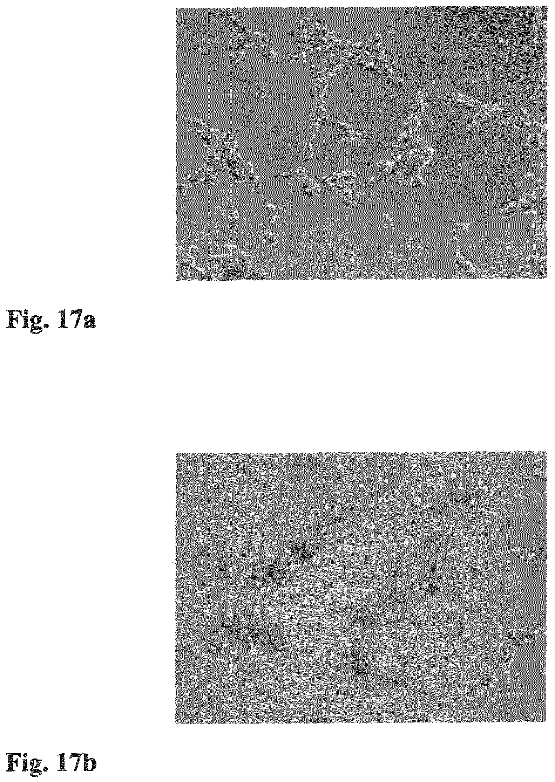

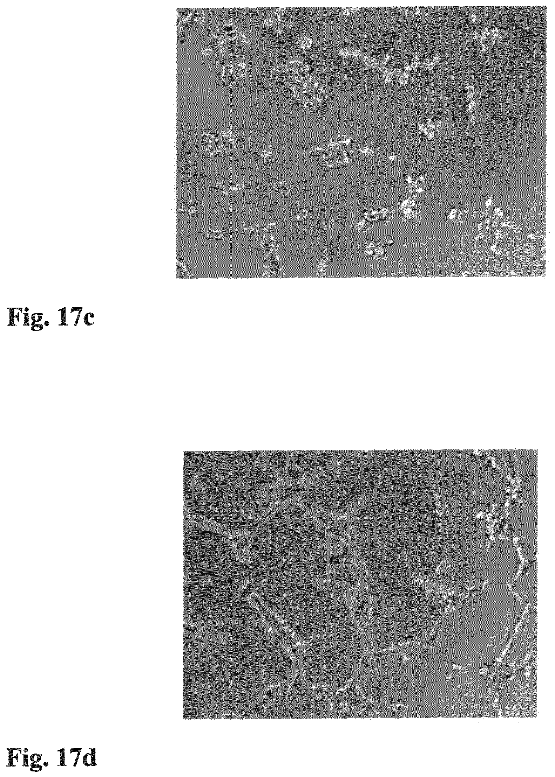

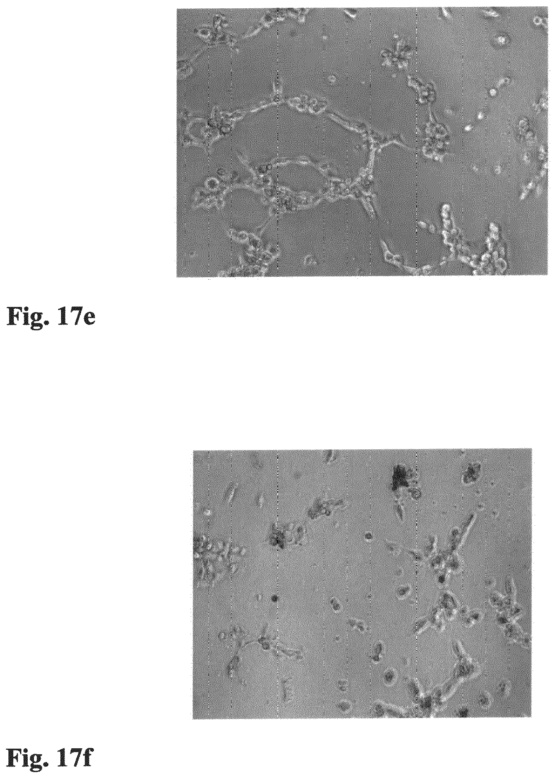

The results are shown in Figs. 17a-h.

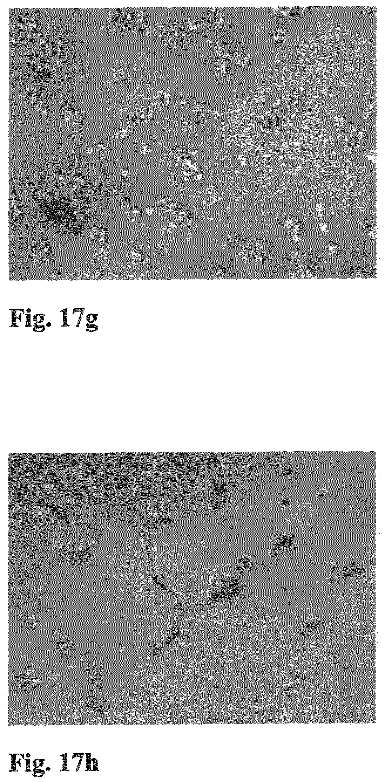

There was only little inhibition of endothelial tube formation with PEDF at a concentration of 250 ng/mL (Fig. 17b) with a complete inhibition observed at 500 ng/mL (Fig. 17c). Bevacizumab inhibited tube formation only at a concentration of 2 mg/mL (Fig. 17f). Coadministration of PEDF and Bevacizumab at concentrations of 250 ng/mL (PEDF) and 250 µg/mL (Bevacizumab), respectively, showed a much stronger inhibitory effect on tube formation (Fig. 17g) than when individually treated with PEDF or Bevacizumab at the same concentrations. This was particularly evident for Bevacizumab which, when used alone, inhibited endothelial tube formation only at a high concentration of 2 mg/mL. Thus, Bevacizumab was thus effective in inhibiting tube formation at a much lower concentration when treated in combination with PEDF (Figs. 17b and 17h). This data indicates a synergistic effect of PEDF and Bevacizumab with respect to the inhibition of endothelial tube formation and thus in the inhibition of angiogenesis.

### Example 11: PEDF induces growth of choriocapillaris

This example shows that PEDF induce growth of choriocapillaris as evidenced in a model for dry AMD using sodium iodate (NaIO3)⁻.

The sodium iodate (NaIO3)-induced model of retinal degeneration has been shown to display some dry AMD-associated features (Hanus, J., et al. (2016). "Retinal pigment epithelial cell necroptosis in response to sodium iodate." Cell Death Discov 2: 16054).

In LE rats, the intravenous dose of 40 mg/kg NaIO3 has been shown to be optimal to induce regional and time dependent alteration of the RPE. Here, the efficacy of the treatment of PEDF protein alone (11 µg) or in combination with an anti-VEGF agent such as Avastin (50 µg) following a single intravitreal injection given directly after the NaIO3 intoxication is shown.

In vivo examinations and flat-mounted RPE/choroid complexes show formation of new healthy choriocapillaries three days later.

### Example 12: PEDF in combination with VEGF induces growth of choriocapillaris

This example shows that PEDF in combination with VEGF induce growth of choriocapillaris as evidenced in a dry AMD model using sodium iodate (NaIO3).

The sodium iodate (NaIO3)-induced model of retinal degeneration has been shown to display some dry AMD-associated features. In LE rats, the intravenous dose of 40 mg/kg NaIO3 has been shown to be optimal to induce regional and time dependent alteration of the RPE. Here, the efficacy of the treatment of PEDF protein (11 µg) alone or in combination with VEGF (2-4 µg) protein following a single intravitreal injection given directly after the NaIO3 intoxication is shown. Both proteins are also injected separately.

In vivo examinations and flat-mounted RPE/choroid complexes show formation of new healthy choriocapillaries three days later. The new choriocapillaries are healthy, i.e. non-leaking, and functional when PEDF and VEGF are combined or PEDF is used alone.

### Example 13: PEDF in combination with an anti VEGF drug induces growth of healthy retinal vessels

This example shows that PEDF in combination with an anti-VEGF drug induce growth of healthy retinal vessels as evidenced in a neovascularization model using an oxygen-induced ischemic retinopathy model.

PEDF protein alone (11 µg) or combined with an anti-VEGF antibody (Avastin, 50 µg) in a newborn rat oxygen-induced ischemic retinopathy (OIR) induce formation of new retinal blood vessels.

Said model is considered representative of the retinopathy of prematurity (ROP) and was already described by Semkova I et al. (Semkova, I., et al. (2002)). "Autologous transplantation of genetically modified iris pigment epithelial cells: a promising concept for the treatment of age-related macular degeneration and other disorders of the eye." Proc Natl Acad Sci USA 99(20): 13090-13095).

Briefly, rats are maintained in room air for the normoxia group, whereas the rats in the hyperoxia group are exposed to 75% oxygen from postnatal day 7 (P7) to P12, then they are removed to room air and immediately treated.

The non-perfused areas and degree of vascularization is determined as described in Semkova et al (2002) (supra). The new retinal vessels are healthy, i.e. non-leaking, and functional when PEDF is used alone of used in combination with an anti-VEGF drug.

### Example 14: PEDF in combination with anti-VEGF reduced the amount of VEGF more effective than PEDF alone

Choroidal neovascularizations (CNVs) produced in the experiments described in Example 6 were stained with anti-VEGF antibodies in paraffin sections. 30 rats were subretinally injected with AAV-VEGF vectors. Three weeks after that, all of them developed CNV around the area of injection. The rats were divided in 5 groups. The total CNV area and the VEGF positive area were measured using the FIJI software, using a semi-automated machine learning based approach (WEKA segmentation).

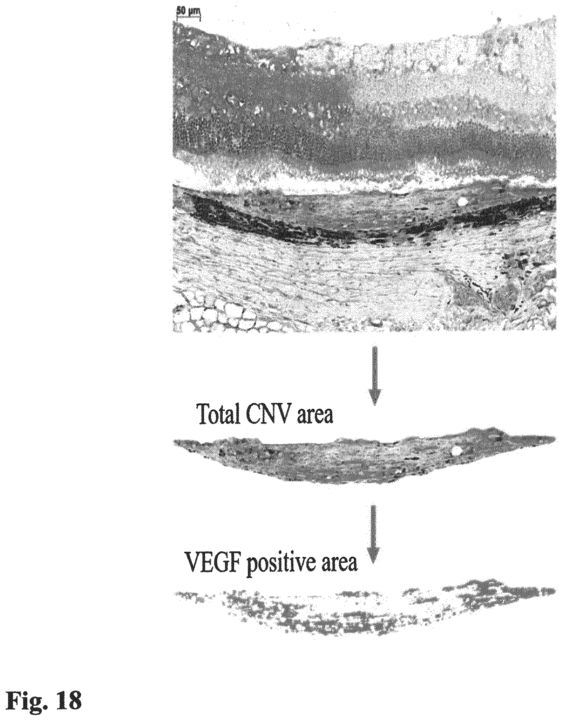

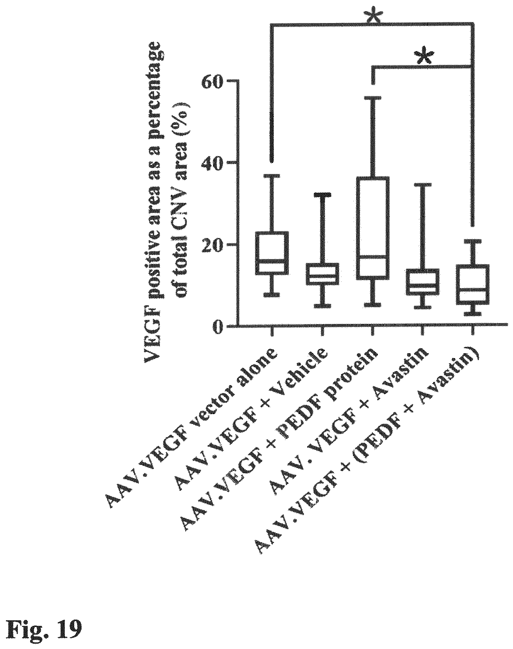

The results are shown in Figs. 18 and 19.

As illustrated in Fig. 19, treatment with PEDF (11 µg) + Avastin (50 µg) (n = 6) resulted in a significant reduction of VEGF in the CNV area in this group compared to the untreated control group (n = 5). Treatment with PEDF alone, resulted in strong intra-individual variation of VEGF in this group (n = 5). Compared to this PEDF group, the Avastin+PEDF group also showed a significant reduction. Data was analyzed by Wilcoxon test with Kruskal-Wallis post-test. (* p< 0.05). Although the combination of PEDF and Avastin was only minimal more effective than Avastin alone, the combination is of advantage due the anti-apoptotic effect of PEDF (see example 15).

### Example 15: Neuroprotective effect of PEDF

This example shows that PEDF is neuroprotective as evidenced in a hypoxia rat eye model.

Hypoxic retinae were produced as described in example 1. Rat eyes were isolated and immediately injected with PBS (vehicle) or PEDF solution (11,5µg/µl). After 14 h incubation in the refrigerator (4°C), the eyes were fixed and embedded in paraffin. The Tunnel-analysis was performed using the *in situ* cell death detection TMR red TUNEL kit (Roche Diagnostics, Mannheim, Germany) as recommended by the manufacturer. The apoptotic cell nuclei were shown in red; the outer segments of the photoreceptors also appeared red, but this was caused by retinal fluorophores.

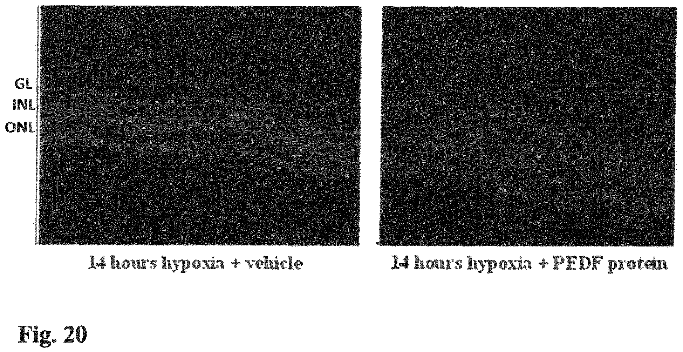

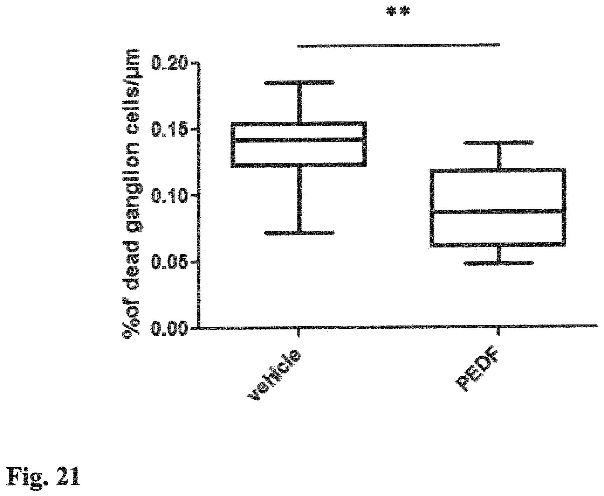

The results are shown in Figs. 20 and 21.

The treatment with PEDF reduced the number of apoptotic cells in the ex vivo retinal hypoxia model described in example 1 and this provided a significant neuroprotective effect on ganglion cells compared with vehicle treatment. (* * p< 0.05).

### References

The complete bibliographic data of the documents recited herein the disclosure of which is incorporated by reference is, if not indicated to the contrary, as follows.
Biesemeier, A., T. Taubitz, S. Julien, E. Yoeruek and U. Schraermeyer (2014). "Choriocapillaris breakdown precedes retinal degeneration in age-related macular degeneration." Neurobiol Aging 35(11): 2562-2573.
Biesemeier, A. K., S. Julien and U. Schraermeyer (2014a). "Choroidal neovascularization can help photoreceptors to survive in late AMD." Investigative Ophthalmology & Visual Science 55(13).
Browning, D. J., P. K. Kaiser, P. J. Rosenfeld and M. W. Stewart (2012). "Aflibercept for age-related macular degeneration: a game-changer or quiet addition?" Am J Ophthalmol 154(2): 222-226.
Daha, N. A., N. K. Banda, A. Roos, F. J. Beurskens, J. M. Bakker, M. R. Daha and L. A. Trouw (2011). "Complement activation by (auto-) antibodies." Mol Immunol 48(14): 1656-1665.
Ferrara, N., L. Damico, N. Shams, H. Lowman and R. Kim (2006). "Development of ranibizumab, an anti-vascular endothelial growth factor antigen binding fragment, as therapy for neovascular age-related macular degeneration." Retina 26(8): 859-870.
Fischer, M. D., G. Huber, S. C. Beck, N. Tanimoto, R. Muehlfriedel, E. Fahl, C. Grimm, A. Wenzel, C. E. Reme, S. A. van de Pavert, J. Wijnholds, M. Pacal, R. Bremner and M. W. Seeliger (2009). "Noninvasive, in vivo assessment of mouse retinal structure using optical coherence tomography." PLoS One 4(10): e7507.
Hanhart, J., D. S. Comaneshter, Y. Freier Dror and S. Vinker (2017). "Mortality in patients treated with intravitreal bevacizumab for age-related macular degeneration." BMC Ophthalmol 17(1): 189.
Hanhart, J., D. S. Comaneshter, Y. Freier-Dror and S. Vinker (2018). "Mortality associated with bevacizumab intravitreal injections in age-related macular degeneration patients after acute myocardial infarct: a retrospective population-based survival analysis." Graefes Arch Clin Exp Ophthalmol 256(4): 651-663.
Hanhart, J., D. S. Comaneshter and S. Vinker (2018). "Mortality after a cerebrovascular event in age-related macular degeneration patients treated with bevacizumab ocular injections." Acta Ophthalmol 96(6): e732-e739.
Hanus, J., et al. (2016). "Retinal pigment epithelial cell necroptosis in response to sodium iodate." Cell Death Discov 2: 16054.
He, T., J. Hu, G. Yan, L. Li, D. Zhang, Q. Zhang, B. Chen and Y. Huang (2015). "Pigment epithelium-derived factor regulates microvascular permeability through adipose triglyceride lipase in sepsis." Clin Sci (Lond) 129(1): 49-61.
Huber, G., S. C. Beck, C. Grimm, A. Sahaboglu-Tekgoz, F. Paquet-Durand, A. Wenzel, P. Humphries, T. M. Redmond, M. W. Seeliger and M. D. Fischer (2009). "Spectral domain optical coherence tomography in mouse models of retinal degeneration." Invest Ophthalmol Vis Sci 50(12): 5888-5895.
Julien, S., A. Biesemeier and U. Schraermeyer (2013). "In vitro induction of protein complexes between bevacizumab, VEGF-A(1)(6)(5) and heparin: explanation for deposits observed on endothelial veins in monkey eyes." Br J Ophthalmol 97(4): 511-517.
Julien, S., A. Biesemeier, T. Taubitz and U. Schraermeyer (2014). "Different effects of intravitreally injected ranibizumab and aflibercept on retinal and choroidal tissues of monkey eyes." Br J Ophthalmol 98(6): 813-825.
Julien, S., F. Kreppel, S. Beck, P. Heiduschka, V. Brito, S. Schnichels, S. Kochanek and U. Schraermeyer (2008). "A reproducible and quantifiable model of choroidal neovascularization induced by VEGF A165 after subretinal adenoviral gene transfer in the rabbit." Mol Vis 14: 1358-1372.
King, G. L. and K. Suzuma (2000). "Pigment-epithelium-derived factor - A key coordinator of retinal neuronal and vascular functions." New England Journal of Medicine 342(5): 349-351.
Mentes, J. and S. Yildirim (2019). "Optical Coherence Tomography Characteristics of Quiescent Type 1 Neovascularization in Eyes with Nonexudative Age-related Macular Degeneration." Turk J Ophthalmol 49(2): 84-88.
Meyer, C. H. and F. G. Holz (2011). "Preclinical aspects of anti-VEGF agents for the treatment of wet AMD: ranibizumab and bevacizumab." Eye (Lond) 25(6): 661-672.
Meyer, T., L. Robles-Carrillo, T. Robson, F. Langer, H. Desai, M. Davila, M. Amaya, J. L. Francis and A. Amirkhosravi (2009). "Bevacizumab immune complexes activate platelets and induce thrombosis in FCGR2A transgenic mice." J Thromb Haemost 7(1): 171-181.
Muller, P. L., et al. (2020). OCT-Angiography in Geographic Atrophy. Ophthalmologica ;doi: 10.1159/000510727
Papadopoulos, N., J. Martin, Q. Ruan, A. Rafique, M. P. Rosconi, E. Shi, E. A. Pyles, G. D. Yancopoulos, N. Stahl and S. J. Wiegand (2012). "Binding and neutralization of vascular endothelial growth factor (VEGF) and related ligands by VEGF Trap, ranibizumab and bevacizumab." Angiogenesis 15(2): 171-185.
Peters, S., P. Heiduschka, S. Julien, F. Ziemssen, H. Fietz, K. U. Bartz-Schmidt, G. Tubingen Bevacizumab Study and U. Schraermeyer (2007). "Ultrastructural findings in the primate eye after intravitreal injection of bevacizumab." Am J Ophthalmol 143(6): 995-1002.
Querques et al. (2013) Functional Characterization and Multimodal Imaging of Treatment-Na¨ve "Quiescent" Choroidal Neovascularization IOVS j October j Vol. 54 j No. 10 j 6887.
Rolling, F., G. Le Meur, K. Stieger, A. J. Smith, M. Weber, J. Y. Deschamps, D. Nivard, A. Mendes-Madeira, N. Provost, Y. Pereon, Y. Cherel, R. R. Ali, C. Hamel, P. Moullier and F. Rolling (2006). "Gene therapeutic prospects in early onset of severe retinal dystrophy: restoration of vision in RPE65 Briard dogs using an AAV serotype 4 vector that specifically targets the retinal pigmented epithelium." Bull Mem Acad R Med Belg 161(10-12): 497-508; discussion 508-499.
Schraermeyer, U. and S. Julien (2012). "Formation of immune complexes and thrombotic microangiopathy after intravitreal injection of bevacizumab in the primate eye." Graefes Arch Clin Exp Ophthalmol 250(9): 1303-1313.
Schraermeyer, U. and S. Julien (2013). "Effects of bevacizumab in retina and choroid after intravitreal injection into monkey eyes." Expert Opin Biol Ther 13(2): 157-167.
Schraermeyer, U., S. Julien, A. Biesemeier, K. U. Bartz-Schmidt and H. Wolburg (2015). "A new kind of labyrinth-like capillary is responsible for leakage from human choroidal neovascular endothelium, as investigated by high-resolution electron microscopy." Graefes Arch Clin Exp Ophthalmol 253(5): 681-689.
Schutze, C., M. Wedl, B. Baumann, M. Pircher, C. K. Hitzenberger and U. Schmidt-Erfurth (2015). "Progression of retinal pigment epithelial atrophy in antiangiogenic therapy of neovascular age-related macular degeneration." Am J Ophthalmol 159(6): 1100-1114 e1101.
Semkova, I., et al. (2002)). "Autologous transplantation of genetically modified iris pigment epithelial cells: a promising concept for the treatment of age-related macular degeneration and other disorders of the eye." Proc Natl Acad Sci U S A 99(20): 13090-13095.
Spaide RF, Klancnik JM, Cooney MJ. Retinal Vascular Layers Imaged by Fluorescein Angiography and Optical Coherence Tomography Angiography. JAMA Ophthalmol. 2015; 133(1):45.
Treister, A. D., P. L. Nesper, A. E. Fayed, M. K. Gill, R. G. Mirza and A. A. Fawzi (2018). "Prevalence of Subclinical CNV and Choriocapillaris Nonperfusion in Fellow Eyes of Unilateral Exudative AMD on OCT Angiography." Transl Vis Sci Technol 7(5): 19.
Wang, F., K. G. Rendahl, W. C. Manning, D. Quiroz, M. Coyne and S. S. Miller (2003). "AAV-mediated expression of vascular endothelial growth factor induces choroidal neovascularization in rat." Invest Ophthalmol Vis Sci 44(2): 781-790.

The features of the present invention disclosed in the specification, the claims, the sequence listing and/or the drawings may both separately and in any combination thereof be material for realizing the invention in various forms thereof.

## Claims

1. A pigment epithelium-derived factor (PEDF) for use in a method for the treatment and/or prevention of dry macular degeneration in a subject, preferably dry age-related macular degeneration, wherein the method comprises administering PEDF and VEGF to the subj ect.

2. The pigment epithelium-derived factor (PEDF) for use of claim 1, wherein the subject is suffering from treatment-naive quiescent choroidal neovascularization.

3. The pigment epithelium-derived factor (PEDF) for use of any one of claims 1 and 2, wherein the subject is suffering from geographic atrophy.

4. The pigment epithelium-derived factor (PEDF) for use of claim 3, wherein geographic atrophy is the result of anti-VEGF therapy.

5. The pigment epithelium-derived factor (PEDF) for use of any one of claims 1, 2, 3 and 4, wherein PEDF induces growth of choriocapillaris.

6. The pigment epithelium-derived factor (PEDF) for use of claim 5, wherein choriocapillaris are non-leaking choriocapillaris.

7. The pigment epithelium-derived factor (PEDF) for use of any one of claims 1 to 6, preferably any one of claims 3 to 6, wherein PEDF inhibits growth and/or formation of geographic atrophy.

8. A pigment epithelium-derived factor (PEDF) for use in a method for the treatment and/or prevention of wet macular degeneration in a subject, preferably wet age-related macular degeneration, wherein the method comprises administering PEDF and an anti-VEGF therapy to the subject.

9. The pigment epithelium-derived factor (PEDF) for use of claim 8, wherein PEDF induces growth of non-leaking choriocapillaris.

10. The pigment epithelium-derived factor (PEDF) for use for use of any one of claims 8 and 9, wherein the anti-VEGF therapy comprises administration to the subject of an anti-VEGF drug.

11. The pigment epithelium-derived factor (PEDF) for use of any one of claims 8, 9 and 10, wherein the anti-VEGF drug is selected from the group comprising pegaptanib, ranibizumab, bevacizumab and aflibercept.

12. A pigment epithelium-derived factor (PEDF) for use in a method for the treatment and/or prevention of treatment-naive quiescent choroidal neovascularization in a subject, wherein the method comprises administering PEDF and an VEGF to the subject.

13. The pigment epithelium-derived factor (PEDF) for use of claim 12, wherein the subject is suffering from dry macular degeneration, preferably dry age-related macular degeneration.

14. The pigment epithelium-derived factor (PEDF) for use of 12 and 13, wherein the subject is suffering from geographic atrophy.

15. The pigment epithelium-derived factor (PEDF) for use of claim 14, wherein geographic atrophy is the result of anti-VEGF therapy.

16. The pigment epithelium-derived factor (PEDF) for use of any one of claims 12 to 15, wherein PEDF induces growth of choriocapillaris.

17. The pigment epithelium-derived factor (PEDF) for use of claim 16, wherein choriocapillaris are non-leaking choriocapillaris.

18. The pigment epithelium-derived factor (PEDF) for use of any one of claims 12 to 17, preferably any one of claims 14 to 17, wherein PEDF inhibits growth and/or formation of geographic atrophy.


SEQUENCE LISTING

<110>  Curebiotec GmbH

<120>  Method for the treatment of a disease using pigment
       epithelium-derived factor (PEDF)

<130>  S 10126 EP

<140>  20195140.7
<141>  2020-09-08

<160>  47

<170>  PatentIn version 3.5

<210>  1
<211>  399
<212>  PRT
<213>  Homo sapiens

<400>  1

Gln Asn Pro Ala Ser Pro Pro Glu Glu Gly Ser Pro Asp Pro Asp Ser
1               5                   10                  15

Thr Gly Ala Leu Val Glu Glu Glu Asp Pro Phe Phe Lys Val Pro Val
            20                  25                  30

Asn Lys Leu Ala Ala Ala Val Ser Asn Phe Gly Tyr Asp Leu Tyr Arg
        35                  40                  45

Val Arg Ser Ser Thr Ser Pro Thr Thr Asn Val Leu Leu Ser Pro Leu
    50                  55                  60

Ser Val Ala Thr Ala Leu Ser Ala Leu Ser Leu Gly Ala Glu Gln Arg
65                  70                  75                  80

Thr Glu Ser Ile Ile His Arg Ala Leu Tyr Tyr Asp Leu Ile Ser Ser
                85                  90                  95

Pro Asp Ile His Gly Thr Tyr Lys Glu Leu Leu Asp Thr Val Thr Ala
            100                 105                 110

Pro Gln Lys Asn Leu Lys Ser Ala Ser Arg Ile Val Phe Glu Lys Lys
        115                 120                 125

Leu Arg Ile Lys Ser Ser Phe Val Ala Pro Leu Glu Lys Ser Tyr Gly
    130                 135                 140

Thr Arg Pro Arg Val Leu Thr Gly Asn Pro Arg Leu Asp Leu Gln Glu
145                 150                 155                 160

Ile Asn Asn Trp Val Gln Ala Gln Met Lys Gly Lys Leu Ala Arg Ser
                165                 170                 175

Thr Lys Glu Ile Pro Asp Glu Ile Ser Ile Leu Leu Leu Gly Val Ala
            180                 185                 190

His Phe Lys Gly Gln Trp Val Thr Lys Phe Asp Ser Arg Lys Thr Ser
        195                 200                 205

Leu Glu Asp Phe Tyr Leu Asp Glu Glu Arg Thr Val Arg Val Pro Met
    210                 215                 220

Met Ser Asp Pro Lys Ala Val Leu Arg Tyr Gly Leu Asp Ser Asp Leu
225                 230                 235                 240

Ser Cys Lys Ile Ala Gln Leu Pro Leu Thr Gly Ser Met Ser Ile Ile
                245                 250                 255

Phe Phe Leu Pro Leu Lys Val Thr Gln Asn Leu Thr Leu Ile Glu Glu
            260                 265                 270

Ser Leu Thr Ser Glu Phe Ile His Asp Ile Asp Arg Glu Leu Lys Thr
        275                 280                 285

Val Gln Ala Val Leu Thr Val Pro Lys Leu Lys Leu Ser Tyr Glu Gly
    290                 295                 300

Glu Val Thr Lys Ser Leu Gln Glu Met Lys Leu Gln Ser Leu Phe Asp
305                 310                 315                 320

Ser Pro Asp Phe Ser Lys Ile Thr Gly Lys Pro Ile Lys Leu Thr Gln
                325                 330                 335

Val Glu His Arg Ala Gly Phe Glu Trp Asn Glu Asp Gly Ala Gly Thr
            340                 345                 350

Thr Pro Ser Pro Gly Leu Gln Pro Ala His Leu Thr Phe Pro Leu Asp
        355                 360                 365

Tyr His Leu Asn Gln Pro Phe Ile Phe Val Leu Arg Asp Thr Asp Thr
    370                 375                 380

Gly Ala Leu Leu Phe Ile Gly Lys Ile Leu Asp Pro Arg Gly Pro
385                 390                 395

<210>  2
<211>  1257
<212>  RNA

<213>  Homo sapiens

<400>  2
   augcaggccc uggugcuacu ccucugcauu ggagcccucc ucgggcacag cagcugccag      60
   aacccugcca gcccccggga ggagggcucc ccagaccccg acagcacagg ggcgcuggug     120
   gaggaggagg auccuuucuu caaagucccc gugaacaagc uggcagcggc ugucuccaac     180
   uucggcuaug accuguaccg ggugcgaucc agcacgagcc ccacgaccaa cgugcuccug     240
   ucuccucuca guguggccac ggcccucucg gcccucucgc ugggagcgga gcagcgaaca     300
   gaauccauca uucaccgggc ucucuacuau gacuugauca gcagcccaga cauccauggu     360
   accuauaagg agcuccuuga cacggucacc gccccccaga agaaccucaa gagugccucc     420
   cggaucgucu uugagaagaa gcugcgcaua aaauccagcu uuguggcacc ucuggaaaag     480
   ucauauggga ccaggcccag aguccugacg ggcaacccuc gcuuggaccu gcaagagauc     540
   aacaacuggg ugcaggcgca gaugaaaggg aagcucgcca gguccacaaa ggaaauuccc     600
   gaugagauca gcauucuccu ucucggugug gcgcacuuca aggggcagug gguaacaaag     660
   uuugacucca gaaagacuuc ccucgaggau uucuacuugg augaagagag gaccgugagg     720
   guccccauga ugucggaccc uaaggcuguu uuacgcuaug gcuuggauuc agaucucagc     780
   ugcaagauug cccagcugcc cuugaccgga agcaugagua ucaucuucuu ccugccccug     840
   aaagugaccc agaauuugac cuugauagag gagagccuca ccuccgaguu cauucaugac     900
   auagaccgag aacugaagac cgugcaggcg guccucacug uccccaagcu gaagcugagu     960
   uacgaaggcg aagucaccaa gucccugcag gagaugaagc ugcaauccuu guuugauuca    1020
   ccagacuuua gcaagaucac aggcaaaccc aucaagcuga cucaggugga acaccgggcu    1080
   ggcuuugagu ggaacgagga uggggcggga accaccccca gcccagggcu gcagccugcc    1140
   caccucaccu ucccgcugga cuaucaccuu aaccagccuu ucaucuucgu acugagggac    1200
   acagacacag gggcccuucu cuucauuggc aagauucugg accccagggg ccccuaa       1257

<210>  3
<211>  1000
<212>  RNA
<213>  Homo sapiens

<400>  3
   ggacgcugga uuagaaggca gcaaaaaaag aucugugcug gcuggagccc ccucagugug      60
   caggcuuaga gggacuaggc ugggugugga gcugcagcgu auccacaggc cccaggaugc     120
   aggcccuggu gcuacuccuc ugcauuggag cccuccucgg gcacagcagc ugccagaacc     180
   cugccagccc cccggaggag ggcuccccag accccgacag cacaggggcg cugguggagg     240
   aggaggaucc uuucuucaaa guccccguga acaagcuggc agcggcuguc uccaacuucg     300
   gcuaugaccu guaccgggug cgauccagca ugagccccac gaccaacgug cuccugucuc     360
   cucucagugu ggccacggcc cucucggccc ucucgcuggg agcggacgag cgaacagaau 420
   ccaucauuca ccgggcucuc uacuaugacu ugaucagcag cccagacauc caugguaccu 480
   auaaggagcu ccuugacacg gucacugccc cccagaagaa ccucaagagu gccucccgga 540
   ucgucuuuga gaagaagcur cgcauaaaau ccagcuuugu ggcaccucug gaaaagucau 600
   augggaccag gcccagaguc cugacgggca acccucgcuu ggaccugcaa gagaucaaca 660
   acugggugca ggcgcagaug aaagggaagc ucgccagguc cacaaaggaa auucccgaug 720
   agaucagcau ucuccuucuc gguguggcgc acuucaaggg gcagugggua acaaaguuug 780
   acuccagaaa gacuucccuc gaggauuucu acuuggauga agagaggacc gugagggucc 840
   ccaugauguc ggacccuaag gcuguuuuac gcuauggcuu ggauucagau cucagcugca 900
   agauugccca gcugcccuug accggaagca ugaguaucau cuucuuccug ccccugaaag 960
   ugacccagaa uuugaccuug auagaggaga gccucaccuc 1000

<210> 4
<211> 412
<212> PRT
<213> Homo sapiens

<400> 4

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Lys Lys Ser Val Arg Gly Lys Gly Lys Gly Gln Lys Arg Lys Arg Lys
                325                 330                 335

Lys Ser Arg Tyr Lys Ser Trp Ser Val Tyr Val Gly Ala Arg Cys Cys
            340                 345                 350

Leu Met Pro Trp Ser Leu Pro Gly Pro His Pro Cys Gly Pro Cys Ser
        355                 360                 365

Glu Arg Arg Lys His Leu Phe Val Gln Asp Pro Gln Thr Cys Lys Cys
    370                 375                 380

Ser Cys Lys Asn Thr Asp Ser Arg Cys Lys Ala Arg Gln Leu Glu Leu
385                 390                 395                 400

Asn Glu Arg Thr Cys Arg Cys Asp Lys Pro Arg Arg
                405                 410

<210>  5
<211>  3660
<212>  RNA
<213>  Homo sapiens

<400>  5
gcggaggcuu ggggcagccg gguagcucgg aggucguggc gcugggggcu agcaccagcg      60

cucugucggg aggcgcagcg guuaggugga ccggucagcg gacucaccgg ccagggcgcu     120

cggugcugga auuugauauu cauugauccg gguuuuaucc cucuucuuuu uucuuaaaca     180

uuuuuuuuua aaacuguauu guuucucguu uuaauuuauu uuugcuugcc auuccccacu     240

ugaaucgggc cgacggcuug gggagauugc ucuacuuccc caaaucacug uggauuuugg     300

aaaccagcag aaagaggaaa gagguagcaa gagcuccaga gagaagucga ggaagagaga     360

gacgggguca gagagagcgc gcgggcgugc gagcagcgaa agcgacaggg gcaaagugag     420

ugaccugcuu uuggggguga ccgccggagc gcggcgugag cccucccccu ugggaucccg     480

cagcugacca gucgcgcuga cggacagaca gacagacacc gcccccagcc ccagcuacca     540

ccuccucccc ggccggcggc ggacagugga cgcggcggcg agccgcgggc aggggccgga     600

gcccgcgccc ggaggcgggg uggagggggu cggggcucgc ggcgucgcac ugaaacuuuu     660

cguccaacuu cugggcuguu cucgcuucgg aggagccgug guccgcgcgg gggaagccga     720

gccgagcgga gccgcgagaa gugcuagcuc gggccgggag gagccgcagc cggaggaggg     780

ggaggaggaa gaagagaagg aagaggagag gggccgcagu ggcgacucgg gcgcucggaa     840

gccgggcuca uggacgggug aggcggcggu gugcgcagac agugcuccag ccgcgcgcgc     900

uccccaggcc cuggcccggg ccucgggccg gggaggaaga guagcucgcc gaggcgccga     960

ggagagcggg ccgccccaca gcccgagccg gagagggagc gcgagccgcg ccggccccgg    1020

ucgggccucc gaaaccauga acuuucugcu gucuugggug cauuggagcc uugccuugcu    1080

gcucuaccuc caccaugcca agugguccca ggcugcaccc auggcagaag gaggagggca    1140

gaaucaucac gaagugguga aguucaugga ugucuaucag cgcagcuacu gccauccaau    1200

cgagacccug guggacaucu uccaggagua cccugaugag aucgaguaca ucuucaagcc    1260

auccugugug ccccugaugc gaugcggggg cugcugcaau gacgagggcc uggagugugu    1320

gcccacugag gaguccaaca ucaccaugca gauuaugcgg aucaaaccuc accaaggcca    1380

gcacauagga gagaugagcu uccuacagca caacaaaugu gaaugcagac caaagaaaga    1440

uagagcaaga caagaaaaaa aaucaguucg aggaaaggga aaggggcaaa aacgaaagcg 1500

caagaaaucc cgguauaagu ccuggagcgu guacguuggu gcccgcugcu gucuaaugcc 1560

cuggagccuc ccuggccccc aucccugugg gccuugcuca gagcggagaa agcauuuguu 1620

uguacaagau ccgcagacgu guaaauguuc cugcaaaaac acagacucgc guugcaaggc 1680

gaggcagcuu gaguuaaacg aacguacuug cagaugugac aagccgaggc ggugagccgg 1740

gcaggaggaa ggagccuccc ucaggguuuc gggaaccaga ucucucacca ggaaagacug 1800

auacagaacg aucgauacag aaaccacgcu gccgccacca caccaucacc aucgacagaa 1860

caguccuuaa uccagaaacc ugaaaugaag gaagaggaga cucugcgcag agcacuuugg 1920

guccggaggg cgagacuccg gcggaagcau ucccggcggg gugacccagc acggucccuc 1980

uuggaauugg auucgccauu uuauuuuucu ugcugcuaaa ucaccgagcc cggaagauua 2040

gagaguuuua uuucugggau uccuguagac acacccaccc acauacauac auuuauauau 2100

auauauauua uauauauaua aaaauaaaua ucucuauuuu auauauauaa aauauauaua 2160

uucuuuuuuu aaauuaacag ugcuaauguu auuggugucu ucacuggaug uauuugacug 2220

cuguggacuu gaguugggag gggaauguuc ccacucagau ccugacaggg aagaggagga 2280

gaugagagac ucuggcauga ucuuuuuuuu gucccacuug guggggccag gguccucucc 2340

ccugcccagg aaugugcaag gccagggcau gggggcaaau augacccagu uuugggaaca 2400

ccgacaaacc cagcccuggc gcugagccuc ucuaccccag gucagacgga cagaaagaca 2460

gaucacaggu acaggaugag ggacaccggc ucugaccagg aguuggggag gcuucaggac 2520

auugcugugc uuugggauuu cccuccacau gcugcacgcg caucucgccc ccagggcacc 2580

ugccuggaag auucaggagc cugggcggcc uucgcuuacu cucaccugcu ucugaguugc 2640

ccaggagacc acuggcagau gucccggcga agagaagaga cacauuguug gaagaagcag 2700

cccaugacag cuccccuucc ugggacucgc ccucauccuc uuccugcucc ccuuccuggg 2760

gugcagccua aaaggaccua uguccucaca ccauugaaac cacuaguucu guccccccag 2820

gagaccuggu ugugugugug ugagugguug accuuccucc auccccuggu ccuucccuuc 2880

ccuucccgag gcacagagag acagggcagg auccacgugc ccauugugga ggcagagaaa 2940

agagaaagug uuuuauauac gguacuuauu uaauaucccu uuuuaauuag aaauuaaaac 3000

aguuaauuua auuaaagagu aggguuuuuu uucaguauuc uugguuaaua uuuaauuuca 3060

acuauuuaug agauguaucu uuugcucucu cuugcucucu uauuuguacc gguuuuugua 3120

uauaaaauuc auguuuccaa ucucucucuc ccugaucggu gacagucacu agcuuaucuu 3180

gaacagauau uuaauuuugc uaacacucag cucugcccuc cccgaucccc uggcucccca 3240

gcacacauuc cuuugaaaua agguuucaau auacaucuac auacuauaua uauauuuggc 3300

aacuuguauu ugugugua ua uauauauaua uauguuuaug uauauaugug auucugauaa 3360

aauagacauu gcuauucugu uuuuuauaug uaaaaacaaa acaagaaaaa auagagaauu 3420

cuacauacua aaucucucuc cuuuuuuaau uuuaauauuu guuaucauuu auuuauuggu 3480

gcuacuguuu auccguaaua auugugggga aaagauauua acaucacguc uuugucucua 3540

gugcaguuuu ucgagauauu ccguaguaca uauuuauuuu uaaacaacga caaagaaaua 3600

cagauauauc uuaaaaaaaa aaaagcauuu uguauuaaag aauuuaauuc ugaucucaaa 3660

<210> 6
<211> 395
<212> PRT
<213> Homo sapiens

<400> 6

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Lys Lys Ser Val Arg Gly Lys Gly Lys Gly Gln Lys Arg Lys Arg Lys
                325                 330                 335

Lys Ser Arg Tyr Lys Ser Trp Ser Val Pro Cys Gly Pro Cys Ser Glu
            340                 345                 350

Arg Arg Lys His Leu Phe Val Gln Asp Pro Gln Thr Cys Lys Cys Ser
        355                 360                 365

Cys Lys Asn Thr Asp Ser Arg Cys Lys Ala Arg Gln Leu Glu Leu Asn
    370                 375                 380

Glu Arg Thr Cys Arg Cys Asp Lys Pro Arg Arg
385                 390                 395

<210>  7
<211>  3609
<212>  RNA
<213>  Homo sapiens

<400>  7
   gcggaggcuu ggggcagccg gguagcucgg aggucguggc gcugggggcu agcaccagcg        60
   cucugucggg aggcgcagcg guuaggugga ccggucagcg gacucaccgg ccagggcgcu 120
   cggugcugga auuugauauu cauugauccg gguuuauccc cucuucuuuu uucuuaaaca 180
   uuuuuuuuua aaacuguauu guuucucguu uuaauuuauu uuugcuugcc auuccccacu 240
   ugaaucgggc cgacggcuug gggagauugc ucuacuuccc caaaucacug uggauuuugg 300
   aaaccagcag aaagaggaaa gagguagcaa gagcuccaga gagaagucga ggaagagaga 360
   gacgggguca gagagagcgc gcgggcgugc gagcagcgaa agcgacaggg gcaaagugag 420
   ugaccugcuu uuggggguga ccgccggagc gcggcgugag cccucccccu ugggaucccg 480
   cagcugacca gucgcgcuga cggacagaca gacagacacc gcccccagcc ccagcuacca 540
   ccuccucccc ggccggcggc ggacagugga cgcggcggcg agccgcgggc aggggccgga 600
   gcccgcgccc ggaggcgggg uggagggggu cggggcucgc ggcgucgcac ugaaacuuuu 660
   cguccaacuu cugggcuguu cucgcuucgg aggagccgug guccgcgcgg gggaagccga 720
   gccgagcgga gccgcgagaa gugcuagcuc gggccgggag gagccgcagc cggaggaggg 780
   ggaggaggaa gaagagaagg aagaggagag gggccgcagu uggcgacucg gcgcucggaa 840
   gccgggcuca uggacgggug aggcggcggu gugcgcagac agugcuccag ccgcgcgcgc 900
   uccccaggcc cuggcccggg ccucgggccg gggaggaaga guagcucgcc gaggcgccga 960
   ggagagcggg ccgccccaca gcccgagccg gagagggagc gcgagccgcg ccggccccgg 1020
   ucgggccucc gaaaccauga acuuucugcu gucuugggug cauuggagcc uugccuugcu 1080
   gcucuaccuc caccaugcca agugguccca ggcugcaccc auggcagaag gaggagggca 1140
   gaaucaucac gaagugguga aguucaugga ugucuaucag cgcagcuacu gccauccaau 1200
   cgagacccug guggacaucu uccaggagua cccugaugag aucgaguaca ucuucaagcc 1260
   auccugugug ccccugaugc gaugcggggg cugcugcaau gacgagggcc uggagugugu 1320
   gcccacugag gaguccaaca ucaccaugca gauuaugcgg aucaaaccuc accaaggcca 1380
   gcacauagga gagaugagcu uccuacagca caacaaaugu gaaugcagac caaagaaaga 1440
   uagagcaaga caagaaaaaa aaucaguucg aggaaaggga aaggggcaaa aacgaaagcg 1500
   caagaaaucc cgguauaagu ccuggagcgu ucccuguggg ccuugcucag agcggagaaa 1560
   gcauuuguuu guacaagauc cgcagacgug uaaauguucc ugcaaaaaca cagacucgcg 1620
   uugcaaggcg aggcagcuug aguuaaacga acguacuugc agaugugaca agccgaggcg 1680
   gugagccggg caggaggaag gagccucccu caggguuucg ggaaccagau cucucaccag 1740
   gaaagacuga uacagaacga ucgauacaga aaccacgcug ccgccaccac accaucacca 1800
   ucgacagaac aguccuuaau ccagaaaccu gaaaugaagg aagaggagac ucugcgcaga 1860
   gcacuuuggg uccggagggc gagacuccgg cggaagcauu cccgggcggg ugacccagca 1920
   cggucccucu uggaauugga uucgccauuu uauuuuucuu gcugcuaaau caccgagccc 1980
   ggaagauuag agaguuuuau uucugggauu ccuguagaca cacccaccca cauacauaca 2040
   uuuauauaua uauauauuau auauauauaa aaauaaauau cucuauuuua uauauauaaa 2100
   auauauauau ucuuuuuuua aauuaacagu gcuaauguua uuggugucuu cacuggaugu 2160
   auuugacugc uguggacuug aguugggagg ggaauguucc cacucagauc cugacaggga 2220
   agaggaggag augagagacu cuggcaugau cuuuuuuuug ucccacuugg uggggccagg 2280
   guccucuccc cugcccagga augugcaagg ccagggcaug ggggcaaaua ugacccaguu 2340
   uugggaacac cgacaaaccc agcccuggcg cugagccucu cuaccccagg ucagacggac 2400
   agaaagacag aucacaggua cagggaugag gacaccggcu cugaccagga guuuggggag 2460
   cuucaggaca uugcugugcu uuggggauuc ccuccacaug cugcacgcgc aucucgcccc 2520
   caggggcacu gccuggaaga uucaggagcc ugggcggccu ucgcuuacuc ucaccugcuu 2580
   cugaguugcc caggagacca cuggcagaug ucccggcgaa gagaagagac acauuguugg 2640
   aagaagcagc ccaugacagc uccccuuccu gggacucgcc cucauccucu uccugcuccc 2700
   cuuccugggg ugcagccuaa aaggaccuau guccucacac cauugaaacc acuaguucug 2760
   uccccccagg agaccugguu gugugugugu gagugguuga ccuuccucca uccccugguc 2820
   cuucccuucc cuucccgagg cacagagaga cagggcagga uccacgugcc cauuguggag 2880
   gcagagaaaa gagaaagugu uuuauauacg guacuuauuu aauaucccuu uuuaauuaga 2940
   aauuaaaaca guuaauuuaa uuaaagagua ggguuuuuuu ucaguauucu ugguuaauau 3000
   uuaauuucaa cuauuuauga gauguaucuu uugcucucuc uugcucucuu auuuguaccg 3060
   guuuuuguau auaaaauuca uguuuccaau cucucucucc cugaucggug acagucacua 3120
   gcuuaucuug aacagauauu uaauuuugcu aacacucagc ucugcccucc ccgauccccu 3180
   ggcuccccag cacacauucc uuugaaauaa gguuucaaua uacaucuaca uacuauauau 3240
   auauuuggca acuuguauuu guguguauau auauauauau auguuuaugu auauauguga 3300
   uucugauaaa auagacauug cuauucuguu uuuuauaugu aaaaacaaaa caagaaaaaa 3360
   uagagaauuc uacauacuaa aucucucucc uuuuuuaauu uuaauauuug uuaucauuua 3420
   uuuauuggug cuacuguuua uccguaauaa uuguggggaa aagauauuaa caucacgucu 3480
   uugucucuag ugcaguuuuu cgagauauuc cguaguacau auuuauuuuu aaacaacgac 3540
   aaagaaauac agauauaucu uaaaaaaaaa aaagcauuuu guauuaaaga auuuaauucu 3600
   gaucucaaa 3609

<210> 8
<211> 389
<212> PRT
<213> Homo sapiens

<400>  8

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Lys Lys Ser Val Arg Gly Lys Gly Lys Gly Gln Lys Arg Lys Arg Lys
                325                 330                 335

Lys Ser Arg Pro Cys Gly Pro Cys Ser Glu Arg Arg Lys His Leu Phe
            340                 345                 350

Val Gln Asp Pro Gln Thr Cys Lys Cys Ser Cys Lys Asn Thr Asp Ser
        355                 360                 365

Arg Cys Lys Ala Arg Gln Leu Glu Leu Asn Glu Arg Thr Cys Arg Cys
    370                 375                 380

Asp Lys Pro Arg Arg
385

<210>  9
<211>  3591
<212>  RNA
<213>  Homo sapiens

<400>  9
gcggaggcuu ggggcagccg gguagcucgg aggucguggc gcugggggcu agcaccagcg      60

cucugucggg aggcgcagcg guuaggugga ccggucagcg gacucaccgg ccagggcgcu     120

cggugcugga auuugauauu cauugauccg gguuuuaucc cucuucuuuu uucuuaaaca     180

uuuuuuuuua aaacuguauu guuucucguu uuaauuuauu uuugcuugcc auuccccacu     240

ugaaucgggc cgacggcuug gggagauugc ucuacuuccc caaaucacug uggauuuugg     300

aaaccagcag aaagaggaaa gagguagcaa gagcuccaga gagaagucga ggaagagaga     360

gacgggguca gagagagcgc gcgggcgugc gagcagcgaa agcgacaggg gcaaagugag     420

ugaccugcuu uuggggguga ccgccggagc gcggcgugag cccucccccu ugggaucccg     480

cagcugacca gucgcgcuga cggacagaca gacagacacc gcccccagcc ccagcuacca     540

ccuccucccc ggccggcggc ggacagugga cgcggcggcg agccgcgggc aggggccgga 600

gcccgcgccc ggaggcgggg uggagggggu cggggcucgc ggcgucgcac ugaaacuuuu 660

cguccaacuu cugggcuguu cucgcuucgg aggagccgug guccgcgcgg gggaagccga 720

gccgagcgga gccgcgagaa gugcuagcuc gggccgggag gagccgcagc cggaggaggg 780

ggaggaggaa gaagagaagg aagaggagag ggggccgcag uggcgacucg gcgcucggaa 840

gccgggcuca uggacgggug aggcggcggu gugcgcagac agugcuccag ccgcgcgcgc 900

uccccaggcc cuggcccggg ccucgggccg gggaggaaga guagcucgcc gaggcgccga 960

ggagagcggg ccgccccaca gcccgagccg gagagggagc gcgagccgcg ccggccccgg 1020

ucgggccucc gaaaccauga acuuucugcu gucuugggug cauuggagcc uugccuugcu 1080

gcucuaccuc caccaugcca agugguccca ggcugcaccc auggcagaag gaggagggca 1140

gaaucaucac gaagugguga aguucaugga ugucuaucag cgcagcuacu gccauccaau 1200

cgagacccug guggacaucu uccaggagua cccugaugag aucgaguaca ucuucaagcc 1260

auccugugug ccccugaugc gaugcggggg cugcugcaau gacgagggcc uggagugugu 1320

gcccacugag gaguccaaca ucaccaugca gauuaugcgg aucaaaccuc accaaggcca 1380

gcacauagga gagaugagcu uccuacagca caacaaaugu gaaugcagac caaagaaaga 1440

uagagcaaga caagaaaaaa aaucaguucg aggaaaggga aaggggcaaa aacgaaagcg 1500

caagaaaucc cgucccugug ggccuugcuc agagcggaga aagcauuugu uuguacaaga 1560

uccgcagacg uguaaauguu ccugcaaaaa cacagacucg cguugcaagg cgaggcagcu 1620

ugaguuaaac gaacguacuu gcagauguga caagccgagg cggugagccg ggcaggagga 1680

aggagccucc cucaggguuu cgggaaccag aucucucacc aggaaagacu gauacagaac 1740

gaucgauaca gaaaccacgc ugccgccacc acaccaucac caucgacaga acaguccuua 1800

auccagaaac cugaaaugaa ggaagaggag acucugcgca gagcacuuug gguccggagg 1860

gcgagacucc ggcggaagca uucccgggcg ggugacccag cacggucccu cuuggaauug 1920

gauucgccau uuuauuuuuc uugcugcuaa aucaccgagc ccggaagauu agagaguuuu 1980

auuucuggga uuccuguaga cacacccacc cacauacaua cauuuauaua uauauauauu 2040

auauauauau aaaaauaaau aucucuauuu uauauauaua aaauauauau auucuuuuuu 2100

uaaauuaaca gugcuaaugu uauugguguc uucacuggau guauuugacu gcuguggacu 2160

ugaguuggga ggggaauguu cccacucaga uccugacagg gaagaggagg agaugagaga 2220

cucuggcaug aucuuuuuuu ugucccacuu gguggggcca ggguccucuc cccugcccag 2280

gaaugugcaa ggccagggca ugggggcaaa uaugacccag uuuugggaac accgacaaac 2340

ccagcccugg cgcugagccu cucuacccca ggucagacgg acagaaagac agaucacagg 2400

uacagggaug aggacaccgg cucugaccag gaguuugggg agcuucagga cauugcugug 2460

cuuuggggau ucccuccaca ugcugcacgc gcaucucgcc cccaggggca cugccuggaa 2520

gauucaggag ccugggcggc cuucgcuuac ucucaccugc uucugaguug cccaggagac 2580

cacuggcaga ugucccggcg aagagaagag acacauuguu ggaagaagca gcccaugaca 2640

gcuccccuuc cugggacucg cccucauccu cuuccugcuc cccuuccugg ggugcagccu 2700

aaaaggaccu auguccucac accauugaaa ccacuaguuc ugucccccca ggagaccugg 2760

uugugugugu gugagugguu gaccuuccuc cauccccugg uccuucccuu cccuucccga 2820

ggcacagaga gacagggcag gauccacgug cccauugugg aggcagagaa aagagaaagu 2880

guuuuauaua cgguacuuau uuaauauccc uuuuuaauua gaaauuaaaa caguuaauuu 2940

aauuaaagag uaggguuuuu uuucaguauu cuugguuaau auuuaauuuc aacuauuuau 3000

gagauguauc uuuugcucuc ucuugcucuc uuauuuguac cgguuuuugu auauaaaauu 3060

cauguuucca aucucucucu cccugaucgg ugacagucac uagcuuaucu ugaacagaua 3120

uuuaauuuug cuaacacuca gcucugcccu ccccgauccc cuggcucccc agcacacauu 3180

ccuuugaaau aagguuucaa uauacaucua cauacuauau auauauuugg caacuuguau 3240

uuguguguau auauauauau auauguuuau guauauaugu gauucugaua aaauagacau 3300

ugcuauucug uuuuuuauau guaaaaacaa aacaagaaaa aauagagaau ucuacauacu 3360

aaaucucucu ccuuuuuuaa uuuuaauauu uguuaucauu uauuuauugg ugcuacuguu 3420

uauccguaau aauugugggg aaaagauauu aacaucacgu cuuugucucu agugcaguuu 3480

uucgagauau uccguaguac auauuuauuu uuaaacaacg acaaagaaau acagauauau 3540

cuuaaaaaaa aaaaagcauu uuguauuaaa gaauuuaauu cugaucucaa a 3591

<210> 10
<211> 371
<212> PRT
<213> Homo sapiens

<400> 10

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Asn Pro Cys Gly Pro Cys Ser Glu Arg Arg Lys His Leu Phe Val Gln
                325                 330                 335

Asp Pro Gln Thr Cys Lys Cys Ser Cys Lys Asn Thr Asp Ser Arg Cys
            340                 345                 350

Lys Ala Arg Gln Leu Glu Leu Asn Glu Arg Thr Cys Arg Cys Asp Lys
        355                 360                 365

Pro Arg Arg
    370

<210>  11
<211>  3537
<212>  RNA
<213>  Homo sapiens

<400>  11
gcggaggcuu ggggcagccg gguagcucgg aggucguggc gcugggggcu agcaccagcg      60

cucugucggg aggcgcagcg guuaggugga ccggucagcg gacucaccgg ccagggcgcu     120

cggugcugga auuugauauu cauugauccg gguuuuaucc cucuucuuuu uucuuaaaca     180

uuuuuuuuua aaacuguauu guuucucguu uuaauuuauu uuugcuugcc auuccccacu     240

ugaaucgggc cgacggcuug gggagauugc ucuacuuccc caaaucacug uggauuuugg     300

aaaccagcag aaagaggaaa gagguagcaa gagcuccaga gagaagucga ggaagagaga     360

gacgggguca gagagagcgc gcgggcgugc gagcagcgaa agcgacaggg gcaaagugag     420

ugaccugcuu uuggggguga ccgccggagc gcggcgugag cccucccccu ugggaucccg     480

cagcugacca gucgcgcuga cggacagaca gacagacacc gcccccagcc ccagcuacca     540

ccuccucccc ggccggcggc ggacagugga cgcggcggcg agccgcgggc aggggccgga     600

gcccgcgccc ggaggcgggg uggagggggu cggggcucgc ggcgucgcac ugaaacuuuu     660

cguccaacuu cugggcuguu cucgcuucgg aggagccgug guccgcgcgg gggaagccga     720

gccgagcgga gccgcgagaa gugcuagcuc gggccgggag gagccgcagc cggaggaggg     780

ggaggaggaa gaagagaagg aagaggagag ggggccgcag uggcgacucg gcgcucggaa     840

gccgggcuca uggacgggug aggcggcggu gugcgcagac agugcuccag ccgcgcgcgc     900

uccccaggcc cuggcccggg ccucgggccg gggaggaaga guagcucgcc gaggcgccga     960

ggagagcggg ccgccccaca gcccgagccg gagagggagc gcgagccgcg ccggccccgg    1020

ucgggccucc gaaaccauga acuuucugcu gucuugggug cauuggagcc uugccuugcu    1080

gcucuaccuc caccaugcca agugguccca ggcugcaccc auggcagaag gaggagggca    1140

gaaucaucac gaagugguga aguucaugga ugucuaucag cgcagcuacu gccauccaau    1200

cgagacccug guggacaucu uccaggagua cccugaugag aucgaguaca ucuucaagcc 1260

auccugugug ccccugaugc gaugcggggg cugcugcaau gacgagggcc uggagugugu 1320

gcccacugag gaguccaaca ucaccaugca gauuaugcgg aucaaaccuc accaaggcca 1380

gcacauagga gagaugagcu uccuacagca caacaaaugu gaaugcagac caaagaaaga 1440

uagagcaaga caagaaaauc ccugugggcc uugcucagag cggagaaagc auuuguuugu 1500

acaagauccg cagacgugua aauguuccug caaaaacaca gacucgcguu gcaaggcgag 1560

gcagcuugag uuaaacgaac guacuugcag augugacaag ccgaggcggu gagccgggca 1620

ggaggaagga gccucccuca ggguuucggg aaccagaucu cucaccagga aagacugaua 1680

cagaacgauc gauacagaaa ccacgcugcc gccaccacac caucaccauc gacagaacag 1740

uccuuaaucc agaaaccuga aaugaaggaa gaggagacuc ugcgcagagc acuuuggguc 1800

cggagggcga gacuccggcg gaagcauucc cgggcgggug acccagcacg gucccucuug 1860

gaauuggauu cgccauuuua uuuuucuugc ugcuaaauca ccgagcccgg aagauuagag 1920

aguuuuauuu cugggauucc uguagacaca cccacccaca uacauacauu uauauauaua 1980

uauauuauau auauauaaaa auaaauaucu cuauuuuaua uauauaaaau auauauauuc 2040

uuuuuuuaaa uuaacagugc uaauguuauu ggugucuuca cuggauguau uugacugcug 2100

uggacuugag uugggagggg aauguuccca cucagauccu gacagggaag aggaggagau 2160

gagagacucu ggcaugaucu uuuuuuuguc ccacuuggug gggccagggu ccucuccccu 2220

gcccaggaau gugcaaggcc agggcauggg ggcaaauaug acccaguuuu gggaacaccg 2280

acaaacccag cccuggcgcu gagccucucu accccagguc agacggacag aaagacagau 2340

cacagguaca gggaugagga caccggcucu gaccaggagu uuggggagcu ucaggacauu 2400

gcugugcuuu ggggauuccc uccacaugcu gcacgcgcau cucgccccca ggggcacugc 2460

cuggaagauu caggagccug ggcggccuuc gcuuacucuc accugcuucu gaguugccca 2520

ggagaccacu ggcagauguc ccggcgaaga gaagagacac auuguuggaa gaagcagccc 2580

augacagcuc ccuuccuggg acucgcccu cauccucuuc cugcuccccu uccugggug 2640

cagccuaaaa ggaccuaugu ccucacacca uugaaaccac uaguucuguc cccccaggag 2700

accugguugu guguguguga gugguugacc uuccuccauc cccugguccu ucccuucccu 2760

ucccgaggca cagagagaca gggcaggauc cacgugccca uuguggaggc agagaaaaga 2820

gaaaguguuu uauauacggu acuuauuuaa uaucccuuuu uaauuagaaa uuaaaacagu 2880

uaauuuaauu aaagaguagg guuuuuuuuc aguauucuug guuaauauuu aauuucaacu 2940

auuuaugaga uguaucuuuu gcucucucuu gcucucuuau uuguaccggu uuuuguauau 3000

aaaauucaug uuuccaaucu cucucucccu gaucggugac agucacuagc uuaucuugaa 3060

cagauauuua auuuugcuaa cacucagcuc ugcccucccc gauccccugg cuccccagca 3120

cacauuccuu ugaaauaagg uuucaauaua caucuacaua cuauauauau auuuggcaac 3180

uuguauuugu guguauauau auauauauau guuuauguau auaugugauu cugauaaaau 3240

agacauugcu auucuguuuu uuauauguaa aaacaaaaca agaaaaaaua gagaauucua 3300

cauacuaaau cucucuccuu uuuuaauuuu aauauuuguu aucauuuauu uauuggugcu 3360

acuguuuauc cguaauaauu guggggaaaa gauauuaaca ucacgucuuu gucucuagug 3420

caguuuuucg agauauuccg uaguacauau uuauuuuuaa acaacgacaa agaaauacag 3480

auauaucuua aaaaaaaaaa agcauuuugu auuaaagaau uuaauucuga ucucaaa 3537

<210> 12
<211> 354
<212> PRT
<213> Homo sapiens

<400> 12

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Asn Pro Cys Gly Pro Cys Ser Glu Arg Arg Lys His Leu Phe Val Gln
                325                 330                 335

Asp Pro Gln Thr Cys Lys Cys Ser Cys Lys Asn Thr Asp Ser Arg Cys
            340                 345                 350

Lys Met

<210>  13
<211>  3502
<212>  RNA
<213>  Homo sapiens

<400>  13
gcggaggcuu ggggcagccg gguagcucgg aggucguggc gcugggggcu agcaccagcg      60

cucugucggg aggcgcagcg guuaggugga ccggucagcg gacucaccgg ccagggcgcu     120

cggugcugga auuugauauu cauugauccg gguuuuaucc cucuucuuuu uucuuaaaca 180

uuuuuuuuua aaacuguauu guuucucguu uuaauuuauu uuugcuugcc auuccccacu 240

ugaaucgggc cgacggcuug gggagauugc ucuacuuccc caaaucacug uggauuuugg 300

aaaccagcag aaagaggaaa gagguagcaa gagcuccaga gagaagucga ggaagagaga 360

gacgggguca gagagagcgc gcgggcgugc gagcagcgaa agcgacaggg gcaaagugag 420

ugaccugcuu uuggggguga ccgccggagc gcggcgugag cccucccccu ugggaucccg 480

cagcugacca gucgcgcuga cggacagaca gacagacacc gcccccagcc ccagcuacca 540

ccuccucccc ggccggcggc ggacagugga cgcggcggcg agccgcgggc aggggccgga 600

gcccgcgccc ggaggcgggg uggagggggu cggggcucgc ggcgucgcac ugaaacuuuu 660

cguccaacuu cugggcuguu cucgcuucgg aggagccgug guccgcgcgg gggaagccga 720

gccgagcgga gccgcgagaa gugcuagcuc gggccgggag gagccgcagc cggaggaggg 780

ggaggaggaa gaagagaagg aagaggagag ggggccgcag uggcgacucg gcgcucggaa 840

gccgggcuca uggacgggug aggcggcggu gugcgcagac agugcuccag ccgcgcgcgc 900

uccccaggcc cuggcccggg ccucgggccg gggaggaaga guagcucgcc gaggcgccga 960

ggagagcggg ccgccccaca gcccgagccg gagagggagc gcgagccgcg ccggccccgg 1020

ucgggccucc gaaaccauga acuuucugcu gucuugggug cauuggagcc uugccuugcu 1080

gcucuaccuc caccaugcca agugguccca ggcugcaccc auggcagaag gaggagggca 1140

gaaucaucac gaagugguga aguucaugga ugucuaucag cgcagcuacu gccauccaau 1200

cgagacccug guggacaucu uccaggagua cccugaugag aucgaguaca ucuucaagcc 1260

auccugugug ccccugaugc gaugcggggg cugcugcaau gacgagggcc uggagugugu 1320

gcccacugag gaguccaaca ucaccaugca gauuaugcgg aucaaaccuc accaaggcca 1380

gcacauagga gagaugagcu uccuacagca caacaaaugu gaaugcagac caaagaaaga 1440

uagagcaaga caagaaaauc ccugugggcc uugcucagag cggagaaagc auuuguuugu 1500

acaagauccg cagacgugua aauguuccug caaaaacaca gacucgcguu gcaagaugug 1560

acaagccgag gcggugagcc gggcaggagg aaggagccuc ccucaggguu ucgggaacca 1620

gaucucucac caggaaagac ugauacagaa cgaucgauac agaaaccacg cugccgccac 1680

cacaccauca ccaucgacag aacaguccuu aauccagaaa ccugaaauga aggaagagga 1740

gacucugcgc agagcacuuu ggguccggag ggcgagacuc cggcggaagc auucccgggc 1800

gggugaccca gcacgguccc ucuuggaauu ggauucgcca uuuuauuuuu cuugcugcua 1860

aaucaccgag cccggaagau uagagaguuu uauuucuggg auuccuguag acacacccac 1920

ccacauacau acauuuauau auauauauau uauauauaua uaaaaauaaa uaucucuauu 1980

uuauauauau aaaauauaua uauucuuuuu uuaaauuaac agugcuaaug uuauuggugu 2040

cuucacugga uguauuugac ugcuguggac uugaguuggg aggggaaugu ucccacucag 2100

auccugacag ggaagaggag gagaugagag acucuggcau gaucuuuuuu uugucccacu 2160

ugguggggcc aggguccucu ccccugccca ggaaugugca aggccagggc augggggcaa 2220

auaugaccca guuuugggaa caccgacaaa cccagcccug gcgcugagcc ucucuacccc 2280

aggucagacg gacagaaaga cagaucacag guacagggau gaggacaccg gcucugacca 2340

ggaguuuggg gagcuucagg acauugcugu gcuuugggga uucccuccac augcugcacg 2400

cgcaucucgc ccccaggggc acugccugga agauucagga gccuggcggg ccuucgcuua 2460

cucucaccug cuucugaguu gcccaggaga ccacuggcag augucccggc gaagagaaga 2520

gacacauugu uggaagaagc agcccaugac agcuccccuu ccugggacuc gcccucaucc 2580

ucuuccugcu ccccuuccug gggugcagcc uaaaaggacc uauguccuca caccauugaa 2640

accacuaguu cugucccccc aggagaccug guugugugug ugugaguggu ugaccuuccu 2700

ccauccccug guccuucccu ucccuucccg aggcacagag agacagggca ggauccacgu 2760

gcccauugug gaggcagaga aaagagaaag uguuuuauau acgguacuua uuuaauaucc 2820

cuuuuuaauu agaaauuaaa acaguuaauu uaauuaaaga guaggguuuu uuuucaguau 2880

ucuugguuaa uauuuaauuu caacuauuua ugagauguau cuuuugcucu cucuugcucu 2940

cuuauuugua ccgguuuuug uauauaaaau ucauguuucc aaucucucuc ucccugaucg 3000

gugacaguca cuagcuuauc uugaacagau auuuaauuuu gcuaacacuc agcucugccc 3060

uccccgaucc ccuggcuccc cagcacacau uccuuugaaa uaagguuuca auauacaucu 3120

acauacuaua uauauauuug gcaacuugua uuugugugua uauauauaua uauauguuua 3180

uguauauaug ugauucugau aaaauagaca uugcuauucu guuuuuuaua uguaaaaaca 3240

aaacaagaaa aaauagagaa uucuacauac uaaaucucuc uccuuuuuua auuuuaauau 3300

uuguuaucau uuauuuauug gugcuacugu uuauccguaa uaauuguggg gaaaagauau 3360

uaacaucacg ucuuugucuc uagugcaguu uuucgagaua uuccguagua cauauuuauu 3420

uuuaaacaac gacaaagaaa uacagauaua ucuuaaaaaa aaaaaagcau uuuguauuaa 3480

agaauuuaau ucugaucuca aa 3502

<210> 14
<211> 327
<212> PRT
<213> Homo sapiens

<400> 14

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Lys Cys Asp Lys Pro Arg Arg
                325

<210>  15
<211>  3405
<212>  RNA
<213>  Homo sapiens

<400>  15
gcggaggcuu ggggcagccg gguagcucgg aggucguggc gcugggggcu agcaccagcg      60

cucugucggg aggcgcagcg guuaggugga ccggucagcg gacucaccgg ccagggcgcu     120

cggugcugga auuugauauu cauugauccg gguuuuaucc cucuucuuuu uucuuaaaca     180

uuuuuuuuua aaacuguauu guuucucguu uuaauuuauu uuugcuugcc auuccccacu     240

ugaaucgggc cgacggcuug gggagauugc ucuacuuccc caaaucacug uggauuuugg     300

aaaccagcag aaagaggaaa gagguagcaa gagcuccaga gagaagucga ggaagagaga     360

gacgggguca gagagagcgc gcgggcgugc gagcagcgaa agcgacaggg gcaaagugag     420

ugaccugcuu uuggggguga ccgccggagc gcggcgugag cccucccccu ugggaucccg     480

cagcugacca gucgcgcuga cggacagaca gacagacacc gcccccagcc ccagcuacca     540

ccuccucccc ggccggcggc ggacagugga cgcggcggcg agccgcgggc aggggccgga     600

gcccgcgccc ggaggcgggg uggagggggu cggggcucgc ggcgucgcac ugaaacuuuu     660

cguccaacuu cugggcuguu cucgcuucgg aggagccgug guccgcgcgg gggaagccga     720

gccgagcgga gccgcgagaa gugcuagcuc gggccgggag gagccgcagc cggaggaggg     780

ggaggaggaa gaagagaagg aagaggagag ggggccgcag uggcgacucg gcgcucggaa     840

gccgggcuca uggacgggug aggcggcggu gugcgcagac agugcuccag ccgcgcgcgc     900

uccccaggcc cuggcccggg ccucgggccg gggaggaaga guagcucgcc gaggcgccga     960

ggagagcggg ccgccccaca gcccgagccg gagagggagc gcgagccgcg ccggccccgg    1020

ucgggccucc gaaaccauga acuuucugcu gucuugggug cauuggagcc uugccuugcu    1080

gcucuaccuc caccaugcca agugguccca ggcugcaccc auggcagaag gaggagggca    1140

gaaucaucac gaagugguga aguucaugga ugucuaucag cgcagcuacu gccauccaau    1200

cgagacccug guggacaucu uccaggagua cccugaugag aucgaguaca ucuucaagcc 1260

auccugugug ccccugaugc gaugcggggg cugcugcaau gacgagggcc uggagugugu 1320

gcccacugag gaguccaaca ucaccaugca gauuaugcgg aucaaaccuc accaaggcca 1380

gcacauagga gagaugagcu uccuacagca caacaaaugu gaaugcagac caaagaaaga 1440

uagagcaaga caagaaaaau gugacaagcc gaggcgguga gccgggcagg aggaaggagc 1500

cucccucagg guuucgggaa ccagaucucu caccaggaaa gacugauaca gaacgaucga 1560

uacagaaacc acgcugccgc caccacacca ucaccaucga cagaacaguc cuuaauccag 1620

aaaccugaaa ugaaggaaga ggagacucug cgcagagcac uuugggucog gagggcgaga 1680

cuccggcgga agcauucccg ggcgggugac ccagcacggu cccucuugga auuggauucg 1740

ccauuuuauu uuucuugcug cuaaaucacc gagcccggaa gauuagagag uuuuauuucu 1800

gggauuccug uagacacacc cacccacaua cauacauuua uauauauaua uauuauauau 1860

auauaaaaau aaauaucucu auuuuauaua uauaaaauau auauauucuu uuuuuaaauu 1920

aacagugcua auguuauugg ugucuucacu ggauguauuu gacugcugug gacuugaguu 1980

gggaggggaa uguucccacu cagauccuga cagggaagag gaggagauga gagacucugg 2040

caugaucuuu uuuuuguccc acuugguggg gccagggucc ucuccccugc ccaggaaugu 2100

gcaaggccag ggcauggggg caaauaugac ccaguuuugg gaacaccgac aaacccagcc 2160

cuggcgcuga gccucucuac cccaggucag acggacagaa agacagauca cagguacagg 2220

gaugaggaca ccggcucuga ccaggaguuu ggggagcuuc aggacauugc ugugcuuugg 2280

ggauucccuc cacaugcugc acgcgcaucu cgcccccagg ggcacugccu ggaagauuca 2340

ggagccuggg cggccuucgc uuacucucac cugcuucuga guugcccagg agaccacugg 2400

cagauguccc ggcgaagaga agagacacau uguuggaaga agcagcccau gacagcuccc 2460

cuuccuggga cucgcccuca uccucuuccu gcuccccuuc cuggggugca gccuaaaagg 2520

accuaugucc ucacaccauu gaaaccacua guucuguccc cccaggagac cugguugugu 2580

gugugugagu gguugaccuu ccuccauccc cugguccuuc ccuucccuuc ccgaggcaca 2640

gagagacagg gcaggaucca cgugcccauu guggaggcag agaaaagaga aaguguuuua 2700

uauacgguac uuauuuaaua ucccuuuuua auuagaaauu aaaacaguua auuuaauuaa 2760

agaguagggu uuuuuuucag uauucuuggu uaauauuuaa uuucaacuau uuaugagaug 2820

uaucuuuugc ucucucuugc ucucuuauuu guaccgguuu uuguauauaa aauucauguu 2880

uccaaucucu cucucccuga ucggugacag ucacuagcuu aucuugaaca gauauuuaau 2940

uuugcuaaca cucagcucug cccuccccga uccccuggcu ccccagcaca cauuccuuug 3000

aaauaagguu ucaauauaca ucuacauacu auauauauau uuggcaacuu guauuugugu 3060

guauauauau auauauaugu uuauguauau augugauucu gauaaaauag acauugcuau 3120

ucuguuuuuu auauguaaaa acaaaacaag aaaaaauaga gaauucuaca uacuaaaucu 3180

cucuccuuuu uuaauuuuaa uauuuguuau cauuuauuua uuggugcuac uguuuauccg 3240

uaauaauugu ggggaaaaga uauuaacauc acgucuuugu cucuagugca guuuuucgag 3300

auauuccgua guacauauuu auuuuuaaac aacgacaaag aaauacagau auaucuuaaa 3360

aaaaaaaaag cauuuuguau uaaagaauuu aauucugauc ucaaa 3405

<210> 16
<211> 371
<212> PRT
<213> Homo sapiens

<400> 16

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Asn Pro Cys Gly Pro Cys Ser Glu Arg Arg Lys His Leu Phe Val Gln
                325                 330                 335

Asp Pro Gln Thr Cys Lys Cys Ser Cys Lys Asn Thr Asp Ser Arg Cys
            340                 345                 350

Lys Ala Arg Gln Leu Glu Leu Asn Glu Arg Thr Cys Arg Ser Leu Thr
        355                 360                 365

Arg Lys Asp
    370

<210>  17
<211>  3471
<212>  RNA
<213>  Homo sapiens

<400>  17
gcggaggcuu ggggcagccg gguagcucgg aggucguggc gcugggggcu agcaccagcg      60

cucugucggg aggcgcagcg guuaggugga ccggucagcg gacucaccgg ccagggcgcu     120

cggugcugga auuugauauu cauugauccg gguuuuaucc cucuucuuuu uucuuaaaca     180

uuuuuuuuua aaacuguauu guuucucguu uuaauuuauu uuugcuugcc auuccccacu 240

ugaaucgggc cgacggcuug gggagauugc ucuacuuccc caaaucacug uggauuuugg 300

aaaccagcag aaagaggaaa gagguagcaa gagcuccaga gagaagucga ggaagagaga 360

gacgggguca gagagagcgc gcgggcgugc gagcagcgaa agcgacaggg gcaaagugag 420

ugaccugcuu uuggggguga ccgccggagc gcggcgugag cccucccccu ugggaucccg 480

cagcugacca gucgcgcuga cggacagaca gacagacacc gcccccagcc ccagcuacca 540

ccuccucccc ggccggcggc ggacagugga cgcggcggcg agccgcgggc aggggccgga 600

gcccgcgccc ggaggcgggg uggagggggu cggggcucgc ggcgucgcac ugaaacuuuu 660

cguccaacuu cugggcuguu cucgcuucgg aggagccgug guccgcgcgg gggaagccga 720

gccgagcgga gccgcgagaa gugcuagcuc gggccgggag gagccgcagc cggaggaggg 780

ggaggaggaa gaagagaagg aagaggagag ggggccgcag uggcgacucg gcgcucggaa 840

gccgggcuca uggacgggug aggcggcggu gugcgcagac agugcuccag ccgcgcgcgc 900

uccccaggcc cuggcccggg ccucgggccg gggaggaaga guagcucgcc gaggcgccga 960

ggagagcggg ccgccccaca gcccgagccg gagagggagc gcgagccgcg ccggccccgg 1020

ucgggccucc gaaaccauga acuuucugcu gucuugggug cauuggagcc uugccuugcu 1080

gcucuaccuc caccaugcca agugguccca ggcugcaccc auggcagaag gaggagggca 1140

gaaucaucac gaagugguga aguucaugga ugucuaucag cgcagcuacu gccauccaau 1200

cgagacccug guggacaucu uccaggagua cccugaugag aucgaguaca ucuucaagcc 1260

auccugugug ccccugaugc gaugcggggg cugcugcaau gacgagggcc uggagugugu 1320

gcccacugag gaguccaaca ucaccaugca gauuaugcgg aucaaaccuc accaaggcca 1380

gcacauagga gagaugagcu uccuacagca caacaaaugu gaaugcagac caaagaaaga 1440

uagagcaaga caagaaaauc ccugugggcc uugcucagag cggagaaagc auuuguuugu 1500

acaagauccg cagacgugua aauguuccug caaaaacaca gacucgcguu gcaaggcgag 1560

gcagcuugag uuaaacgaac guacuugcag aucucucacc aggaaagacu gauacagaac 1620

gaucgauaca gaaaccacgc ugccgccacc acaccaucac caucgacaga acaguccuua 1680

auccagaaac cugaaaugaa ggaagaggag acucugcgca gagcacuuug gguccggagg 1740

gcgagacucc ggcggaagca uucccgggcg ggugacccag cacggucccu cuuggaauug 1800

gauucgccau uuuauuuuuc uugcugcuaa aucaccgagc ccggaagauu agagaguuuu 1860

auuucuggga uuccuguaga cacacccacc cacauacaua cauuuauaua uauauauauu 1920

auauauauau aaaaauaaau aucucuauuu uauauauaua aaauauauau auucuuuuuu 1980

uaaauuaaca gugcuaaugu uauuggugu c uucacuggau guauuugacu gcuguggacu 2040

ugaguuggga ggggaauguu cccacucaga uccugacagg gaagaggagg agaugagaga 2100

cucuggcaug aucuuuuuuu ugucccacuu gguggggcca ggguccucuc cccugcccag 2160

gaaugugcaa ggccagggca ugggggcaaa uaugacccag uuuugggaac accgacaaac 2220

ccagcccugg cgcugagccu cucuacccca ggucagacgg acagaaagac agaucacagg 2280

uacagggaug aggacaccgg cucugaccag gaguuugggg agcuucagga cauugcugug 2340

cuuuggggau ucccuccaca ugcugcacgc gcaucucgcc cccaggggca cugccuggaa 2400

gauucaggag ccugggcggc cuucgcuuac ucucaccugc uucugaguug cccaggagac 2460

cacuggcaga ugucccggcg aagagaagag acacauuguu ggaagaagca gcccaugaca 2520

gcuccccuuc cugggacucg cccucauccu cuuccugcuc cccuuccugg ggugcagccu 2580

aaaaggaccu auguccucac accauugaaa ccacuaguuc ugucccccca ggagaccugg 2640

uugugugugu gugagugguu gaccuuccuc cauccccugg uccuucccuu cccuucccga 2700

ggcacagaga gacagggcag gauccacgug cccauugugg aggcagagaa aagagaaagu 2760

guuuuauaua cgguacuuau uuaauauccc uuuuuaauua gaaauuaaaa caguuaauuu 2820

aauuaaagag uaggguuuuu uuucaguauu cuugguuaau auuuaauuuc aacuauuuau 2880

gagauguauc uuuugcucuc ucuugcucuc uuauuuguac cgguuuuugu auauaaaauu 2940

cauguuucca aucucucucu cccugaucgg ugacagucac uagcuuaucu ugaacagaua 3000

uuuaauuuug cuaacacuca gcucugcccu ccccgauccc cuggcucccc agcacacauu 3060

ccuuugaaau aagguuucaa uauacaucua cauacuauau auauauuugg caacuuguau 3120

uuguguguau auauauauau auauguuuau guauauaugu gauucugaua aaauagacau 3180

ugcuauucug uuuuuuauau guaaaaacaa aacaagaaaa aauagagaau ucuacauacu 3240

aaaucucucu ccuuuuuuaa uuuuaauauu uguuaucauu uauuuauugg ugcuacuguu 3300

uauccguaau aauugugggg aaaagauauu aacaucacgu cuuugucucu agugcaguuu 3360

uucgagauau uccguaguac auauuuauuu uuaaacaacg acaaagaaau acagauauau 3420

cuuaaaaaaa aaaaagcauu uuguauuaaa gaauuuaauu cugaucucaa a 3471

<210> 18
<211> 317
<212> PRT
<213> Homo sapiens

<400> 18

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Cys Asp Lys Pro Arg Arg
305                 310                 315

<210>  19
<211>  3375
<212>  RNA
<213>  Homo sapiens

<400>  19
   gcggaggcuu ggggcagccg gguagcucgg aggucguggc gcugggggcu agcaccagcg      60
   cucugucggg aggcgcagcg guuaggugga ccggucagcg gacucaccgg ccagggcgcu     120
   cggugcugga auuugauauu cauugauccg gguuuuaucc cucuucuuuu uucuuaaaca     180
   uuuuuuuuua aaacuguauu guuucucguu uuaauuuauu uuugcuugcc auuccccacu     240
   ugaaucgggc cgacggcuug gggagauugc ucuacuuccc caaaucacug uggauuuugg      300
   aaaccagcag aaagaggaaa gagguagcaa gagcuccaga gagaagucga ggaagagaga     360
   gacgggguca gagagagcgc gcgggcgugc gagcagcgaa agcgacaggg gcaaagugag     420
   ugaccugcuu uuggggguga ccgccggagc gcggcgugag cccucccccu ugggaucccg     480
   cagcugacca gucgcgcuga cggacagaca gacagacacc gcccccagcc ccagcuacca     540
   ccuccucccc ggccggcggc ggacagugga cgcggcggcg agccgcgggc aggggccgga     600
   gcccgcgccc ggaggcgggg uggagggggu cggggcucgc ggcgucgcac ugaaacuuuu     660
   cguccaacuu cugggcuguu cucgcuucgg aggagccgug guccgcgcgg gggaagccga     720
   gccgagcgga gccgcgagaa gugcuagcuc gggccgggag gagccgcagc cggaggaggg     780
   ggaggaggaa gaagagaagg aagaggagag ggggccgcag uggcgacucg gcgcucggaa      840
   gccgggcuca uggacgggug aggcggcggu gugcgcagac agugcuccag ccgcgcgcgc     900
   uccccaggcc cuggcccggg ccucgggccg gggaggaaga guagcucgcc gaggcgccga     960
   ggagagcggg ccgccccaca gcccgagccg gagagggagc gcgagccgcg ccggccccgg    1020
   ucgggccucc gaaaccauga acuuucugcu gucuugggug cauuggagcc uugccuugcu    1080
   gcucuaccuc caccaugcca agugguccca ggcugcaccc auggcagaag gaggagggca     1140
   gaaucaucac gaagugguga aguucaugga ugucuaucag cgcagcuacu gccauccaau    1200
   cgagacccug guggacaucu uccaggagua cccugaugag aucgaguaca ucuucaagcc    1260
   auccugugug ccccugaugc gaugcggggg cugcugcaau gacgagggcc uggagugugu    1320
   gcccacugag gaguccaaca ucaccaugca gauuaugcgg aucaaaccuc accaaggcca    1380
   gcacauagga gagaugagcu uccuacagca caacaaaugu gaaugcagau gugacaagcc    1440
   gaggcgguga gccgggcagg aggaaggagc cucccucagg guuucgggaa ccagaucucu 1500
   caccaggaaa gacugauaca gaacgaucga uacagaaacc acgcugccgc caccacacca 1560
   ucaccaucga cagaacaguc cuuaauccag aaaccugaaa ugaaggaaga ggagacucug 1620
   cgcagagcac uuuggguccg gagggcgaga cuccggcgga agcauucccg ggcgggugac 1680
   ccagcacggu cccucuugga auuggauucg ccauuuuauu uuucuugcug cuaaaucacc 1740
   gagcccggaa gauuagagag uuuuauuucu gggauuccug uagacacacc cacccacaua 1800
   cauacauuua uauauauaua uauuauauau auauaaaaau aaauaucucu auuuuauaua 1860
   uauaaaauau auauauucuu uuuuuaaauu aacagugcua auguuauugg ugucuucacu 1920
   ggauguauuu gacugcugug gacuugaguu gggaggggaa uguucccacu cagauccuga 1980
   cagggaagag gaggagauga gagacucugg caugaucuuu uuuuuguccc acuugguggg 2040
   gccagguccc ucuccccugc ccaggaaugu gcaaggccag ggcauggggg caaauaugac 2100
   ccaguuuugg gaacaccgac aaacccagcc cuggcgcuga gccucucuac cccaggucag 2160
   acggacagaa agacagauca cagguacagg gaugaggaca ccggcucuga ccaggaguuu 2220
   ggggagcuuc aggacauugc ugugcuuugg ggauucccuc cacaugcugc acgcgcaucu 2280
   cgcccccagg ggcacugccu ggaagauuca ggagccuggg cggccuucgc uuacucucac 2340
   cugcuucuga guugcccagg agaccacugg cagauguccc ggcgaagaga agagacacau 2400
   uguuggaaga agcagcccau gacagcuccc cuuccuggga cucgcccuca uccucuuccu 2460
   gcuccccuuc cuggggugca gccuaaaagg accuaugucc ucacaccauu gaaaccacua 2520
   guucuguccc cccaggagac cugguugugu gugugugagu gguugaccuu ccuccauccc 2580
   cugguccuuc ccuucccuuc ccgaggcaca gagagacagg gcaggaucca cgugcccauu 2640
   guggaggcag agaaaagaga aaguguuuua uauacgguac uuauuuaaua ucccuuuuua 2700
   auuagaaauu aaaacaguua auuuaauuaa agaguagggu uuuuuuucag uauucuuggu 2760
   uaauauuuaa uuucaacuau uuaugagaug uaucuuuugc ucucucuugc ucucuuauuu 2820
   guaccgguuu uuguauauaa aauucauguu uccaaucucu cucucccuga ucggugacag 2880
   ucacuagcuu aucuugaaca gauauuuaau uuugcuaaca cucagcucug cccuccccga 2940
   uccccuggcu ccccagcaca cauuccuuug aaauaagguu ucaauauaca ucuacauacu 3000
   auauauauau uuggcaacuu guauuugugu guauauauau auauauaugu uuauguauau 3060
   augugauucu gauaaaauag acauugcuau ucuguuuuuu auauguaaaa acaaaacaag 3120
   aaaaaauaga gaauucuaca uacuaaaucu cucuccuuuu uuaauuuuaa uauuuguuau 3180
   cauuuauuua uuggugcuac uguuuauccg uaauaauugu ggggaaaaga uauuaacauc 3240
   acgucuuugu cucuagugca guuuuucgag auauuccgua guacauauuu auuuuuaaac 3300
   aacgacaaag aaauacagau auaucuuaaa aaaaaaaaag cauuuuguau uaaagaauuu     3360
   aauucugauc ucaaa                                                      3375

<210>  20
<211>  232
<212>  PRT
<213>  Homo sapiens

<400>  20

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Lys Ser Val
    130                 135                 140

Arg Gly Lys Gly Lys Gly Gln Lys Arg Lys Arg Lys Lys Ser Arg Tyr
145                 150                 155                 160

Lys Ser Trp Ser Val Tyr Val Gly Ala Arg Cys Cys Leu Met Pro Trp
                165                 170                 175

Ser Leu Pro Gly Pro His Pro Cys Gly Pro Cys Ser Glu Arg Arg Lys
            180                 185                 190

His Leu Phe Val Gln Asp Pro Gln Thr Cys Lys Cys Ser Cys Lys Asn
        195                 200                 205

Thr Asp Ser Arg Cys Lys Ala Arg Gln Leu Glu Leu Asn Glu Arg Thr
    210                 215                 220

Cys Arg Cys Asp Lys Pro Arg Arg
225                 230

<210>  21
<211>  3677
<212>  RNA
<213>  Homo sapiens

<400>  21
ucgcggaggc uuggggcagc cggguagcuc ggaggucgug gcgcuggggg cuagcaccag      60

cgcucugucg ggaggcgcag cgguuaggug gaccggucag cggacucacc ggccagggcg     120

cucggugcug gaauuugaua uucauugauc cggguuuuau cccucuucuu uuuucuuaaa     180

cauuuuuuuu uaaaacugua uuguuucucg uuuuaauuua uuuuugcuug ccauucccca     240

cuugaaucgg gccgacggcu uggggagauu gcucuacuuc cccaaaucac uguggauuuu     300

ggaaaccagc agaaagagga aagagguagc aagagcucca gagagaaguc gaggaagaga     360

gagacggggu cagagagagc gcgcgggcgu gcgagcagcg aaagcgacag gggcaaagug     420

agugaccugc uuuugggggu gaccgccgga gcgcggcgug agcccucccc cuugggaucc     480

cgcagcugac cagucgcgcu gacggacaga cagacagaca ccgcccccag ccccagcuac     540

caccuccucc ccggccggcg gcggacagug gacgcggcgg cgagccgcgg gcaggggccg     600

gagcccgcgc ccggaggcgg gguggagggg gucggggcuc gcggcgucgc acugaaacuu     660

uucguccaac uucugggcug uucucgcuuc ggaggagccg ugguccgcgc gggggaagcc     720

gagccgagcg gagccgcgag aagugcuagc ucgggccggg aggagccgca gccggaggag     780

ggggaggagg aagaagagaa ggaagaggag agggggccgc aguggcgacu cggcgcucgg     840

aagccgggcu cauggacggg ugaggcggcg gugugcgcag acagugcucc agccgcgcgc     900

gcuccccagg cccuggcccg ggccucgggc cggggaggaa gaguagcucg ccgaggcgcc     960

gaggagagcg ggccgcccca cagcccgagc cggagaggga gcgcgagccg cgccggcccc    1020

ggucgggccu ccgaaaccau gaacuuucug cugucuuggg ugcauuggag ccuugccuug    1080

cugcucuacc uccaccaugc caaguggucc caggcugcac ccauggcaga aggaggaggg    1140

cagaaucauc acgaaguggu gaaguucaug gaugucuauc agcgcagcua cugccaucca    1200

aucgagaccc ugguggacau cuuccaggag uacccugaug agaucgagua caucuucaag    1260

ccauccugug ugccccugau gcgaugcggg ggcugcugca augacgaggg ccuggagugu    1320

gugcccacug aggaguccaa caucaccaug cagauuaugc ggaucaaacc ucaccaaggc    1380

cagcacauag gagagaugag cuuccuacag cacaacaaau gugaaugcag accaaagaaa    1440

gauagagcaa gacaagaaaa aaaaucaguu cgaggaaagg gaaaggggca aaaacgaaag 1500

cgcaagaaau cccgguauaa guccuggagc guguacguug gugcccgcug cugucuaaug 1560

cccuggagcc ucccuggccc ccaucccugu gggccuugcu cagagcggag aaagcauuug 1620

uuuguacaag auccgcagac guguaaaugu uccugcaaaa acacagacuc gcguugcaag 1680

gcgaggcagc uugaguuaaa cgaacguacu ugcagaugug acaagccgag gcggugagcc 1740

gggcaggagg aaggagccuc ccucaggguu ucgggaacca gaucucucac caggaaagac 1800

ugauacagaa cgaucgauac agaaaccacg cugccgccac cacaccauca ccaucgacag 1860

aacaguccuu aauccagaaa ccugaaauga aggaagagga gacucugcgc agagcacuuu 1920

ggguccggag ggcgagacuc cggcggaagc auucccgggc gggugaccca gcacgguccc 1980

ucuuggaauu ggauucgcca uuuuauuuuu cuugcugcua aaucaccgag cccggaagau 2040

uagagaguuu uauuucuggg auuccuguag acacacccac ccacauacau acauuuauau 2100

auauauauau uauauauaua uaaaaauaaa uaucucuauu uuauauauau aaaauauaua 2160

uauucuuuuu uuaaauuaac agugcuaaug uuauuggugu cuucacugga uguauuugac 2220

ugcuguggac uugaguuggg aggggaaugu ucccacucag auccugacag ggaagaggag 2280

gagaugagag acucuggcau gaucuuuuuu uuguccacuu ugguggggcc aggguccucu 2340

ccccugccca ggaaugugca aggccagggc augggggcaa auaugaccca guuuuuggaa 2400

caccgacaaa cccagcccug gcgcugagcc ucucuacccc aggucagacg gacagaaaga 2460

cagaucacag guacagggau gaggacaccg gcucugacca ggaguuuggg gagcuucagg 2520

acauugcugu gcuuugggga uucccuccac augcugcacg cgcaucucgc ccccaggggc 2580

acugccugga agauucagga gccugggcgg ccuucgcuua cucucaccug cuucugaguu 2640

gcccaggaga ccacuggcag augucccggc gaagagaaga gacacauugu uggaagaagc 2700

agcccaugac agcuccccuu ccugggacuc gcccucaucc ucuuccugcu ccccuuccug 2760

gggugcagcc uaaaaggacc uauguccuca caccauugaa accacuaguu cugucccccc 2820

aggagaccug guugugugug ugugaguggu ugaccuuccu ccauccccug guccuucccu 2880

ucccuucccg aggcacagag agacagggca ggauccacgu gcccauugug gaggcagaga 2940

aaagagaaag uguuuuauau acgguacuua uuuaauaucc cuuuuuaauu agaaauuaaa 3000

acaguuaauu uaauuaaaga guaggguuuu uuuucaguau ucuugguuaa uauuuaauuu 3060

caacuauuua ugagauguau cuuuugcucu cucuugcucu cuuauuugua ccgguuuuug 3120

uauauaaaau ucauguuucc aaucucucuc ucccugaucg gugacaguca cuagcuuauc 3180

uugaacagau auuuaauuuu gcuaacacuc agcucugccc uccccgaucc ccuggcuccc 3240

cagcacacau uccuuugaaa uaagguuuca auauacaucu acauacuaua uauauauuug 3300

gcaacuugua uuugugugua uauauauaua uauauguuua uguauauaug ugauucugau 3360

aaaauagaca uugcuauucu guuuuuuaua uguaaaaaca aaacaagaaa aaauagagaa 3420

uucuacauac uaaaucucuc uccuuuuuua auuuuaauau uuguuaucau uuauuuauug 3480

gugcuacugu uuauccguaa uaauuguggg gaaaagauau uaacaucacg ucuuugucuc 3540

uagugcaguu uuucgagaua uuccguagua cauauuuauu uuuaaacaac gacaaagaaa 3600

uacagauaua ucuuaaaaaa aaaaaagcau uuuguauuaa agaauuuaau ucugaucuca 3660

aaaaaaaaaa aaaaaaa 3677

<210> 22
<211> 215
<212> PRT
<213> Homo sapiens

<400> 22

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Lys Ser Val
    130                 135                 140

Arg Gly Lys Gly Lys Gly Gln Lys Arg Lys Arg Lys Lys Ser Arg Tyr
145                 150                 155                 160

Lys Ser Trp Ser Val Pro Cys Gly Pro Cys Ser Glu Arg Arg Lys His
                165                 170                 175

Leu Phe Val Gln Asp Pro Gln Thr Cys Lys Cys Ser Cys Lys Asn Thr
            180                 185                 190

Asp Ser Arg Cys Lys Ala Arg Gln Leu Glu Leu Asn Glu Arg Thr Cys
        195                 200                 205

Arg Cys Asp Lys Pro Arg Arg
    210                 215

<210>  23
<211>  3626
<212>  RNA
<213>  Homo sapiens

<400>  23
ucgcggaggc uuggggcagc cggguagcuc ggaggucgug gcgcuggggg cuagcaccag      60

cgcucugucg ggaggcgcag cgguuaggug gaccggucag cggacucacc ggccagggcg     120

cucggugcug gaauuugaua uucauugauc cggguuuuau cccucuucuu uuuucuuaaa     180

cauuuuuuuu uaaaacugua uuguuucucg uuuuaauuua uuuuugcuug ccauucccca     240

cuugaaucgg gccgacggcu uggggagauu gcucuacuuc cccaaaucac uguggauuuu     300

ggaaaccagc agaaagagga aagagguagc aagagcucca gagagaaguc gaggaagaga     360

gagacggggu cagagagagc gcgcgggcgu gcgagcagcg aaagcgacag gggcaaagug     420

agugaccugc uuuugggggu gaccgccgga gcgcggcgug agcccucccc cuugggaucc     480

cgcagcugac cagucgcgcu gacggacaga cagacagaca ccgcccccag ccccagcuac     540

caccuccucc ccggccggcg gcggacagug gacgcggcgg cgagccgcgg gcaggggccg     600

gagcccgcgc ccggaggcgg gguggagggg gucggggcuc gcggcgucgc acugaaacuu     660

uucguccaac uucugggcug uucucgcuuc ggaggagccg ugguccgcgc gggggaagcc     720

gagccgagcg gagccgcgag aagugcuagc ucgggccggg aggagccgca gccggaggag     780

ggggaggagg aagaagagaa ggaagaggag agggggccgc aguggcgacu cggcgcucgg     840

aagccgggcu cauggacggg ugaggcggcg gugugcgcag acagugcucc agccgcgcgc     900

gcuccccagg cccuggcccg ggccucgggc cggggaggaa gaguagcucg ccgaggcgcc     960

gaggagagcg ggccgcccca cagcccgagc cggagaggga gcgcgagccg cgccggcccc    1020

ggucgggccu ccgaaaccau gaacuuucug cugucuuggg ugcauuggag ccuugccuug    1080

cugcucuacc uccaccaugc caaguggucc caggcugcac ccauggcaga aggaggaggg    1140

cagaaucauc acgaaguggu gaaguucaug gaugucuauc agcgcagcua cugccaucca    1200

aucgagaccc ugguggacau cuuccaggag uacccugaug agaucgagua caucuucaag    1260

ccauccugug ugccccugau gcgaugcggg ggcugcugca augacgaggg ccuggagugu    1320

gugcccacug aggaguccaa caucaccaug cagauuaugc ggaucaaacc ucaccaaggc 1380

cagcacauag gagagaugag cuuccuacag cacaacaaau gugaaugcag accaaagaaa 1440

gauagagcaa gacaagaaaa aaaaucaguu cgaggaaagg gaaaggggca aaaacgaaag 1500

cgcaagaaau cccgguauaa guccuggagc guucccugug ggccuugcuc agagcggaga 1560

aagcauuugu uuguacaaga uccgcagacg uguaaauguu ccugcaaaaa cacagacucg 1620

cguugcaagg cgaggcagcu ugaguuaaac gaacguacuu gcagauguga caagccgagg 1680

cggugagccg ggcaggagga aggagccucc cucaggguuu cgggaaccag aucucucacc 1740

aggaaagacu gauacagaac gaucgauaca gaaaccacgc ugccgccacc acaccaucac 1800

caucgacaga acaguccuua auccagaaac cugaaaugaa ggaagaggag acucugcgca 1860

gagcacuuug gguccggagg gcgagacucc ggcggaagca uucccgggcg ggugacccag 1920

cacggucccu cuuggaauug gauucgccau uuuauuuuuc uugcugcuaa aucaccgagc 1980

ccggaagauu agagaguuuu auuucuggga uuccuguaga cacacccacc cacauacaua 2040

cauuuauaua uauauauauu auauauauau aaaaauaaau aucucuauuu uauauauaua 2100

aaauauauau auucuuuuuu uaaauuaaca gugcuaaugu uauugguguc uucacuggau 2160

guauuugacu gcuguggacu ugaguuggga ggggaauguu cccacucaga uccugacagg 2220

gaagaggagg agaugagaga cucuggcaug aucuuuuuuu ugucccacuu gguggggcca 2280

ggguccucuc cccugcccag gaaugugcaa ggccagggca ugggggcaaa uaugacccag 2340

uuuugggaac accgacaaac ccagcccugg cgcugagccu cucuacccca ggucagacgg 2400

acagaaagac agaucacagg uacagggaug aggacaccgg cucugaccag gaguuugggg 2460

agcuucagga cauugcugug cuuuggggau ucccuccaca ugcugcacgc gcaucucgcc 2520

cccaggggca cugccuggaa gauucaggag ccugggcggc cuucgcuuac ucucaccugc 2580

uucugaguug cccaggagac cacuggcaga ugucccggcg aagagaagag acacauuguu 2640

ggaagaagca gcccaugaca gcuccccuuc cugggacucg cccucauccu cuuccugcuc 2700

cccuuccugg ggugcagccu aaaaggaccu auguccucac accauugaaa ccacuaguuc 2760

ugucccccca ggagaccugg uugugugugu gugagugguu gaccuuccuc cauccccugg 2820

uccuucccuu cccuucccga ggcacagaga gacagggcag gauccacgug cccauugugg 2880

aggcagagaa aagagaaagu guuuuauaua cgguacuuau uuaauauccc uuuuuaauua 2940

gaaauuaaaa caguuaauuu aauuaaagag uaggguuuuu uuucaguauu cuugguuaau 3000

auuuaauuuc aacuauuuau gagauguauc uuuugcucuc ucuugcucuc uuauuuguac 3060

cgguuuuugu auauaaaauu cauguuucca aucucucucu cccugaucgg ugacagucac 3120

uagcuuaucu ugaacagaua uuuaauuuug cuaacacuca gcucugcccu ccccgauccc 3180

cuggcucccc agcacacauu ccuuugaaau aagguuucaa uauacaucua cauacuauau 3240

auauauuugg caacuuguau uugugugua u auauauauau auauguuuau guauauaugu 3300

gauucugaua aaauagacau ugcuauucug uuuuuuauau guaaaaacaa aacaagaaaa 3360

aauagagaau ucuacauacu aaaucucucu ccuuuuuuaa uuuuaauauu uguuaucauu 3420

uauuuauugg ugcuacuguu uauccguaau aauugugggg aaaagauauu aacaucacgu 3480

cuuugucucu agugcaguuu uucgagauau uccguaguac auauuuauuu uuaaacaacg 3540

acaaagaaau acagauauau cuuaaaaaaa aaaaagcauu uuguauuaaa gaauuuaauu 3600

cugaucucaa aaaaaaaaaa aaaaaa 3626

<210> 24
<211> 209
<212> PRT
<213> Homo sapiens

<400> 24

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Lys Ser Val
    130                 135                 140

Arg Gly Lys Gly Lys Gly Gln Lys Arg Lys Arg Lys Lys Ser Arg Pro
145                 150                 155                 160

Cys Gly Pro Cys Ser Glu Arg Arg Lys His Leu Phe Val Gln Asp Pro
                165                 170                 175

Gln Thr Cys Lys Cys Ser Cys Lys Asn Thr Asp Ser Arg Cys Lys Ala
            180                 185                 190

Arg Gln Leu Glu Leu Asn Glu Arg Thr Cys Arg Cys Asp Lys Pro Arg
        195                 200                 205

Arg

<210>  25
<211>  3608
<212>  RNA
<213>  Homo sapiens

<400>  25
   ucgcggaggc uuggggcagc cggguagcuc ggaggucgug gcgcuggggg cuagcaccag      60
   cgcucugucg ggaggcgcag cgguuaggug gaccggucag cggacucacc ggccagggcg     120
   cucggugcug gaauuugaua uucauugauc cggguuuuau cccucuucuu uuuucuuaaa     180
   cauuuuuuuu uaaaacugua uuguuucucg uuuuaauuua uuuuugcuug ccauucccca     240
   cuugaaucgg gccgacggcu uggggagauu gcucuacuuc cccaaaucac uguggauuuu     300
   ggaaaccagc agaaagagga aagagguagc aagagcucca gagagaaguc gaggaagaga     360
   gagacggggu cagagagagc gcgcgggcgu gcgagcagcg aaagcgacag gggcaaagug     420
   agugaccugc uuuugggggu gaccgccgga gcgcggcgug agcccucccc cuugggaucc     480
   cgcagcugac cagucgcgcu gacggacaga cagacagaca ccgcccccag ccccagcuac     540
   caccuccucc ccggccggcg gcggacagug gacgcggcgg cgagccgcgg gcaggggccg     600
   gagcccgcgc ccggaggcgg gguggagggg gucggggcuc gcggcgucgc acugaaacuu     660
   uucguccaac uucugggcug uucucgcuuc ggaggagccg ugguccgcgc gggggaagcc     720
   gagccgagcg gagccgcgag aagugcuagc ucgggccggg aggagccgca gccggaggag     780
   ggggaggagg aagaagagaa ggaagaggag agggggccgc aguggcgacu cggcgcucgg     840
   aagccgggcu cauggacggg ugaggcggcg gugugcgcag acagugcucc agccgcgcgc     900
   gcuccccagg cccuggcccg ggccucgggc cggggaggaa gaguagcucg ccgaggcgcc     960
   gaggagagcg ggccgcccca cagcccgagc cggagaggga gcgcgagccg cgccggcccc    1020
   ggucgggccu ccgaaaccau gaacuuucug cugucuuggg ugcauuggag ccuugccuug    1080
   cugcucuacc uccaccaugc caaguggucc caggcugcac ccauggcaga aggaggaggg    1140
   cagaaucauc acgaaguggu gaaguucaug gaugucuauc agcgcagcua cugccaucca    1200
   aucgagaccc ugguggacau cuuccaggag uacccugaug agaucgagua caucuucaag 1260
   ccauccugug ugccccugau gcgaugcggg ggcugcugca augacgaggg ccuggagugu 1320
   gugcccacug aggaguccaa caucaccaug cagauuaugc ggaucaaacc ucaccaaggc 1380
   cagcacauag gagagaugag cuuccuacag cacaacaaau gugaaugcag accaaagaaa 1440
   gauagagcaa gacaagaaaa aaaaucaguu cgaggaaagg gaaaggggca aaaacgaaag 1500
   cgcaagaaau cccgucccug ugggccuugc ucagagcgga gaaagcauuu guuuguacaa 1560
   gauccgcaga cguguaaaug uuccugcaaa aacacagacu cgcguugcaa ggcgaggcag 1620
   cuugaguuaa acgaacguac uugcagaugu gacaagccga ggcggugagc cgggcaggag 1680
   gaaggagccu cccucagggu uucgggaacc agaucucuca ccaggaaaga cugauacaga 1740
   acgaucgaua cagaaaccac gcugccgcca ccacaccauc accaucgaca gaacaguccu 1800
   uaauccagaa accugaaaug aaggaagagg agacucugcg cagagcacuu ugguccggga 1860
   gggcgagacu ccggcggaag cauucccggg cgggugaccc agcacggucc cucuuggaau 1920
   uggauucgcc auuuuauuuu ucuugcugcu aaaucaccga gcccggaaga uuagagaguu 1980
   uuauuucugg gauuccugua gacacaccca cccacauaca uacauuuaua uauauauaua 2040
   uuauauauau auaaaaauaa auaucucuau uuuauauaua uaaaauauau auauucuuuu 2100
   uuuaaauuaa cagugcuaau guuauuggug ucuucacugg auguauuuga cugcugugga 2160
   cuugaguugg gaggggaaug uucccacuca gauccugaca gggaagagga ggagaugaga 2220
   gacucuggca ugaucuuuuu uuugucccac uugguggggc caggguccuc uccccugccc 2280
   aggaaugugc aaggccaggg caugggggca aauaugaccc aguuuuggga acaccgacaa 2340
   acccagcccu ggcgcugagc cucucuaccc caggucagac ggacagaaag acagaucaca 2400
   gguacaggga ugaggacacc ggcucugacc aggaguuugg ggagcuucag gacauugcug 2460
   ugcuuugggg auucccucca caugcugcac gcgcaucucg cccccagggg cacugccugg 2520
   aagauucagg agccugggcg gccuucgcuu acucucaccu gcuucugagu ugcccaggag 2580
   accacuggca gaugucccgg cgaagagaag agacacauug uuggaagaag cagcccauga 2640
   cagcuccccu uccugggacu cgcccucauc cucuuccugc uccccuuccu ggggugcagc 2700
   cuaaaaggac cuauguccuc acaccauuga aaccacuagu ucuguccccc caggagaccu 2760
   gguugugugu gugugagugg uugaccuucc uccauccccu gguccuuccc uucccuuccc 2820
   gaggcacaga gagacagggc aggauccacg ugcccauugu ggaggcagag aaaagagaaa 2880
   guguuuuaua uacgguacuu auuuaauauc ccuuuuuaau uagaaauuaa aacaguuaau 2940
   uuaauuaaag aguaggguuu uuuuucagua uucuugguua auauuuaauu ucaacuauuu 3000
   augagaugua ucuuuugcuc ucucuugcuc ucuuauuugu accgguuuuu guauauaaaa 3060
   uucauguuuc caaucucucu cucccugauc ggugacaguc acuagcuuau cuugaacaga 3120
   uauuuaauuu ugcuaacacu cagcucugcc cuccccgauc cccuggcucc ccagcacaca 3180
   uuccuuugaa auaagguuuc aauauacauc uacauacuau auauauauuu ggcaacuugu 3240
   auuugugugu auauauauau auauauguuu auguauauau gugauucuga uaaaauagac 3300
   auugcuauuc uguuuuuuau auguaaaaac aaaacaagaa aaaauagaga auucuacaua 3360
   cuaaaucucu cuccuuuuuu aauuuuaaua uuuguuauca uuuauuuauu ggugcuacug 3420
   uuuauccgua auaauugugg ggaaaagaua uuaacaucac gucuuugucu cuagugcagu 3480
   uuuucgagau auuccguagu acauauuuau uuuuaaacaa cgacaaagaa auacagauau 3540
   aucuuaaaaa aaaaaaagca uuuuguauua aagaauuuaa uucugaucuc aaaaaaaaaa 3600
   aaaaaaaa 3608

<210> 26
<211> 174
<212> PRT
<213> Homo sapiens

<400> 26

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Asn Pro Cys Gly
    130                 135                 140

Pro Cys Ser Glu Arg Arg Lys His Leu Phe Val Gln Asp Pro Gln Thr
145                 150                 155                 160

Cys Lys Cys Ser Cys Lys Asn Thr Asp Ser Arg Cys Lys Met
                165                 170

<210>  27
<211>  3519
<212>  RNA
<213>  Homo sapiens

<400>  27
ucgcggaggc uuggggcagc cggguagcuc ggaggucgug gcgcuggggg cuagcaccag      60

cgcucugucg ggaggcgcag cgguuaggug gaccggucag cggacucacc ggccagggcg     120

cucggugcug gaauuugaua uucauugauc cggguuuuau cccucuucuu uuuucuuaaa     180

cauuuuuuuu uaaaacugua uuguuucucg uuuuaauuua uuuuugcuug ccauucccca     240

cuugaaucgg gccgacggcu uggggagauu gcucuacuuc cccaaaucac uguggauuuu     300

ggaaaccagc agaaagagga aagagguagc aagagcucca gagagaaguc gaggaagaga     360

gagacggggu cagagagagc gcgcgggcgu gcgagcagcg aaagcgacag gggcaaagug     420

agugaccugc uuuugggggu gaccgccgga gcgcggcgug agcccucccc cuugggaucc     480

cgcagcugac cagucgcgcu gacggacaga cagacagaca ccgcccccag ccccagcuac     540

caccuccucc ccggccggcg gcggacagug gacgcggcgg cgagccgcgg gcaggggccg     600

gagcccgcgc ccggaggcgg gguggagggg gucggggcuc gcggcgucgc acugaaacuu     660

uucguccaac uucugggcug uucucgcuuc ggaggagccg ugguccgcgc gggggaagcc     720

gagccgagcg gagccgcgag aagugcuagc ucgggccggg aggagccgca gccggaggag     780

ggggaggagg aagaagagaa ggaagaggag agggggccgc aguggcgacu cggcgcucgg     840

aagccgggcu cauggacggg ugaggcggcg gugugcgcag acagugcucc agccgcgcgc     900

gcuccccagg cccuggcccg ggccucgggc cggggaggaa gaguagcucg ccgaggcgcc     960

gaggagagcg ggccgcccca cagcccgagc cggagaggga gcgcgagccg cgccggcccc    1020

ggucgggccu ccgaaaccau gaacuuucug cugucuuggg ugcauuggag ccuugccuug    1080

cugcucuacc uccaccaugc caaguggucc caggcugcac ccauggcaga aggaggaggg    1140

cagaaucauc acgaaguggu gaaguucaug gaugucuauc agcgcagcua cugccaucca    1200

aucgagaccc ugguggacau cuuccaggag uacccugaug agaucgagua caucuucaag    1260

ccauccugug ugccccugau gcgaugcggg ggcugcugca augacgaggg ccuggagugu    1320

gugcccacug aggaguccaa caucaccaug cagauuaugc ggaucaaacc ucaccaaggc    1380

cagcacauag gagagaugag cuuccuacag cacaacaaau gugaaugcag accaaagaaa    1440

gauagagcaa gacaagaaaa ucccuguggg ccuugcucag agcggagaaa gcauuuguuu    1500

guacaagauc cgcagacgug uaaauguucc ugcaaaaaca cagacucgcg uugcaagaug 1560

ugacaagccg aggcggugag ccgggcagga ggaaggagcc ucccucaggg uuucgggaac 1620

cagaucucuc accaggaaag acugauacag aacgaucgau acagaaacca cgcugccgcc 1680

accacaccau caccaucgac agaacagucc uuaauccaga aaccugaaau gaaggaagag 1740

gagacucugc gcagagcacu uuggguccgg agggcgagac uccggcggaa gcauucccgg 1800

gcgggugacc cagcacgguc ccucuuggaa uuggauucgc cauuuuauuu uucuugcugc 1860

uaaaucaccg agcccggaag auuagagagu uuuauuucug ggauuccugu agacacaccc 1920

acccacauac auacauuuau auauauauau auuauauaua uauaaaaaua aauaucucua 1980

uuuuauauau auaaaauaua uauauucuuu uuuuaaauua acagugcuaa uguuauuggu 2040

gucuucacug gauguauuug acugcugugg acuugaguug ggaggggaau guucccacuc 2100

agauccugac agggaagagg aggagaugag agacucuggc augaucuuuu uuuuguccca 2160

cuuggugggg ccaggguccu cuccccugcc caggaaugug caaggccagg gcaugggggc 2220

aaauaugacc caguuuuggg aacaccgaca aacccagccc uggcgcugag ccucucuacc 2280

ccaggucaga cggacagaaa gacagaucac agguacaggg augaggacac cggcucugac 2340

caggaguuug gggagcuuca ggacauugcu gugcuuuggg gauucccucc acaugcugca 2400

cgcgcaucuc gcccccaggg gcacugccug gaagauucag gagccugggc ggccuucgcu 2460

uacucucacc ugcuucugag uugcccagga gaccacuggc agaugucccg gcgaagagaa 2520

gagacacauu guuggaagaa gcagcccaug acagcucccc uuccugggac ucgcccucau 2580

ccucuuccug cuccccuucc ugggugcagc ccuaaaagga ccuauguccu cacaccauug 2640

aaaccacuag uucugucccc ccaggagacc ugguugugug ugugugagug guugaccuuc 2700

cuccaucccc ugguccuucc cuucccuucc cgaggcacag agagacaggg caggauccac 2760

gugcccauug uggaggcaga gaaaagagaa aguguuuuau auacgguacu uauuuaauau 2820

cccuuuuuaa uuagaaauua aaacaguuaa uuuaauuaaa gaguaggguu uuuuuucagu 2880

auucuugguu aauauuuaau uucaacuauu uaugagaugu aucuuuugcu cucucuugcu 2940

cucuuauuug uaccgguuuu uguauauaaa auucauguuu ccaaucucuc ucucccugau 3000

cggugacagu cacuagcuua ucuugaacag auauuuaauu uugcuaacac ucagcucugc 3060

ccuccccgau ccccuggcuc cccagcacac auuccuuuga aauaagguuu caauauacau 3120

cuacauacua uauauauauu uggcaacuug uauuugugug uauauauaua uauauauguu 3180

uauguauaua ugugauucug auaaaauaga cauugcuauu cuguuuuuua uauguaaaaa 3240

caaaacaaga aaaaauagag aauucuacau acuaaaucuc ucuccuuuuu uaauuuuaau 3300

auuuguuauc auuuauuuau uggugcuacu guuuauccgu aauaauugug gggaaaagau 3360

auuaacauca cgucuuuguc ucuagugcag uuuuucgaga uauuccguag uacauauuua 3420

uuuuuaaaca acgacaaaga aauacagaua uaucuuaaaa aaaaaaaagc auuuuguauu 3480

aaagaauuua auucugaucu caaaaaaaaa aaaaaaaaa 3519

<210> 28
<211> 147
<212> PRT
<213> Homo sapiens

<400> 28

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Cys Asp Lys
    130                 135                 140

Pro Arg Arg
145

<210> 29
<211> 3422
<212> RNA
<213> Homo sapiens

<400> 29
ucgcggaggc uuggggcagc cggguagcuc ggaggucgug gcgcuggggg cuagcaccag 60

cgcucugucg ggaggcgcag cgguuaggug gaccggucag cggacucacc ggccagggcg 120

cucggugcug gaauuugaua uucauugauc cggguuuuau cccucuucuu uuuucuuaaa 180

cauuuuuuuu uaaaacugua uuguuucucg uuuuaauuua uuuuugcuug ccauucccca 240

cuugaaucgg gccgacggcu uggggagauu gcucuacuuc cccaaaucac uguggauuuu 300

ggaaaccagc agaaagagga aagagguagc aagagcucca gagagaaguc gaggaagaga 360

gagacggggu cagagagagc gcgcgggcgu gcgagcagcg aaagcgacag gggcaaagug 420

agugaccugc uuuugggggu gaccgccgga gcgcggcgug agcccucccc cuugggaucc 480

cgcagcugac cagucgcgcu gacggacaga cagacagaca ccgcccccag ccccagcuac 540

caccuccucc ccggccggcg gcggacagug gacgcggcgg cgagccgcgg gcaggggccg 600

gagcccgcgc ccggaggcgg gguggagggg gucggggcuc gcggcgucgc acugaaacuu 660

uucguccaac uucugggcug uucucgcuuc ggaggagccg ugguccgcgc gggggaagcc 720

gagccgagcg gagccgcgag aagugcuagc ucgggccggg aggagccgca gccggaggag 780

ggggaggagg aagaagagaa ggaagaggag agggggccgc aguggcgacu cggcgcucgg 840

aagccgggcu cauggacggg ugaggcggcg gugugcgcag acagugcucc agccgcgcgc 900

gcuccccagg cccuggcccg ggccucgggc cggggaggaa gaguagcucg ccgaggcgcc 960

gaggagagcg ggccgcccca cagcccgagc cggagaggga gcgcgagccg cgccggcccc 1020

ggucgggccu ccgaaaccau gaacuuucug cugucuuggg ugcauuggag ccuugccuug 1080

cugcucuacc uccaccaugc caaguggucc caggcugcac ccauggcaga aggaggaggg 1140

cagaaucauc acgaaguggu gaaguucaug gaugucuauc agcgcagcua cugccaucca 1200

aucgagaccc ugguggacau cuuccaggag uacccugaug agaucgagua caucuucaag 1260

ccauccugug ugccccugau gcgaugcggg ggcugcugca augacgaggg ccuggagugu 1320

gugcccacug aggaguccaa caucaccaug cagauuaugc ggaucaaacc ucaccaaggc 1380

cagcacauag gagagaugag cuuccuacag cacaacaaau gugaaugcag accaaagaaa 1440

gauagagcaa gacaagaaaa augugacaag ccgaggcggu gagccgggca ggaggaagga 1500

gccucccuca ggguuucggg aaccagaucu cucaccagga aagacugaua cagaacgauc 1560

gauacagaaa ccacgcugcc gccaccacac caucaccauc gacagaacag uccuuaaucc 1620

agaaaccuga aaugaaggaa gaggagacuc ugcgcagagc acuuuggguc cggagggcga 1680

gacuccggcg gaagcauucc cgggcgggug acccagcacg gucccucuug gaauuggauu 1740

cgccauuuua uuuuucuugc ugcuaaauca ccgagcccgg aagauuagag aguuuuauuu 1800

cugggauucc uguagacaca cccacccaca uacauacauu uauauauaua uauauuauau 1860

auauauaaaa auaaauaucu cuauuuuaua uauauaaaau auauauauuc uuuuuuuaaa 1920

uuaacagugc uaauguuauu ggugucuuca cuggauguau uugacugcug uggacuugag 1980

uugggagggg aauguuccca cucagauccu gacagggaag aggaggagau gagagacucu 2040

ggcaugaucu uuuuuuuguc ccacuuggug gggccagggu ccucuccccu gcccaggaau 2100

gugcaaggcc agggcauggg ggcaaauaug acccaguuuu gggaacaccg acaaacccag 2160

cccuggcgcu gagccucucu accccagguc agacggacag aaagacagau cacagguaca 2220

gggaugagga caccggcucu gaccaggagu uuggggagcu ucaggacauu gcugugcuuu 2280

ggggauuccc uccacaugcu gcacgcgcau cucgccccca ggggcacugc cuggaagauu 2340

caggagccug ggcggccuuc gcuuacucuc accugcuucu gaguugccca ggagaccacu 2400

ggcagauguc ccggcgaaga gaagagacac auuguuggaa gaagcagccc augacagcuc 2460

cccuuccugg gacucgcccu cauccucuuc cugcuccccu uccuggggug cagccuaaaa 2520

ggaccuaugu ccucacacca uugaaaccac uaguucuguc cccccaggag accugguugu 2580

guguguguga gugguugacc uuccuccauc cccugguccu ucccuucccu ucccgaggca 2640

cagagagaca gggcaggauc cacgugccca uuguggaggc agagaaaaga gaaaguguuu 2700

uauauacggu acuuauuuaa uaucccuuuu uaauuagaaa uuaaaacagu uaauuuaauu 2760

aaagaguagg guuuuuuuuc aguauucuug guuaauauuu aauuucaacu auuuaugaga 2820

uguaucuuuu gcucucucuu gcucucuuau uuguaccggu uuuuguauau aaaauucaug 2880

uuuccaaucu cucucucccu gaucggugac agucacuagc uuaucuugaa cagauauuua 2940

auuuugcuaa cacucagcuc ugcccucccc gauccccugg cuccccagca cacauuccuu 3000

ugaaauaagg uuucaauaua caucuacaua cuauauauau auuuggcaac uuguauuugu 3060

guguauauau auauauauau guuuauguau auaugugauu cugauaaaau agacauugcu 3120

auucuguuuu uuauauguaa aaacaaaaca agaaaaaaua gagaauucua cauacuaaau 3180

cucucuccuu uuuuaauuuu aauauuuguu aucauuuauu uauuggugcu acuguuuauc 3240

cguaauaauu guggggaaaa gauauuaaca ucacgucuuu gucucuagug caguuuuucg 3300

agauauuccg uaguacauau uuauuuuuaa acaacgacaa agaaauacag auauaucuua 3360

aaaaaaaaaa agcauuuugu auuaaagaau uuaauucuga ucucaaaaaa aaaaaaaaaa 3420

aa 3422

<210> 30
<211> 191
<212> PRT
<213> Homo sapiens

<400> 30

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly

20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Asn Pro Cys Gly
    130                 135                 140

Pro Cys Ser Glu Arg Arg Lys His Leu Phe Val Gln Asp Pro Gln Thr
145                 150                 155                 160

Cys Lys Cys Ser Cys Lys Asn Thr Asp Ser Arg Cys Lys Ala Arg Gln
                165                 170                 175

Leu Glu Leu Asn Glu Arg Thr Cys Arg Ser Leu Thr Arg Lys Asp
            180                 185                 190

<210>  31
<211>  3488
<212>  RNA
<213>  Homo sapiens

<400>  31
ucgcggaggc uuggggcagc cggguagcuc ggaggucgug gcgcuggggg cuagcaccag      60

cgcucugucg ggaggcgcag cgguuaggug gaccggucag cggacucacc ggccagggcg     120

cucggugcug gaauuugaua uucauugauc cggguuuuau cccucuucuu uuuucuuaaa     180

cauuuuuuuu uaaaacugua uuguuucucg uuuuaauuua uuuuugcuug ccauucccca     240

cuugaaucgg gccgacggcu uggggagauu gcucuacuuc cccaaaucac uguggauuuu     300

ggaaaccagc agaaagagga aagagguagc aagagcucca gagagaaguc gaggaagaga     360

gagacggggu cagagagagc gcgcgggcgu gcgagcagcg aaagcgacag gggcaaagug     420

agugaccugc uuuugggggu gaccgccgga gcgcggcgug agcccucccc cuugggaucc 480

cgcagcugac cagucgcgcu gacggacaga cagacagaca ccgcccccag ccccagcuac 540

caccuccucc ccggccggcg gcggacagug gacgcggcgg cgagccgcgg gcaggggccg 600

gagcccgcgc ccggaggcgg gguggagggg gucggggcuc gcggcgucgc acugaaacuu 660

uucguccaac uucugggcug uucucgcuuc ggaggagccg ugguccgcgc gggggaagcc 720

gagccgagcg gagccgcgag aagugcuagc ucgggccggg aggagccgca gccggaggag 780

ggggaggagg aagaagagaa ggaagaggag agggggccgc aguggcgacu cggcgcucgg 840

aagccgggcu cauggacggg ugaggcggcg gugugcgcag acagugcucc agccgcgcgc 900

gcuccccagg cccuggcccg ggccucgggc cggggaggaa gaguagcucg ccgaggcgcc 960

gaggagagcg ggccgcccca cagcccgagc cggagaggga gcgcgagccg cgccggcccc 1020

ggucgggccu ccgaaaccau gaacuuucug cugucuuggg ugcauuggag ccuugccuug 1080

cugcucuacc uccaccaugc caaguggucc caggcugcac ccauggcaga aggaggaggg 1140

cagaaucauc acgaaguggu gaaguucaug gaugucuauc agcgcagcua cugccaucca 1200

aucgagaccc ugguggacau cuuccaggag uacccugaug agaucgagua caucuucaag 1260

ccauccugug ugccccugau gcgaugcggg ggcugcugca augacgaggg ccuggagugu 1320

gugcccacug aggaguccaa caucaccaug cagauuaugc ggaucaaacc ucaccaaggc 1380

cagcacauag gagagaugag cuuccuacag cacaacaaau gugaaugcag accaaagaaa 1440

gauagagcaa gacaagaaaa ucccuguggg ccuugcucag agcggagaaa gcauuuguuu 1500

guacaagauc cgcagacgug uaaauguucc ugcaaaaaca cagacucgcg uugcaaggcg 1560

aggcagcuug aguuaaacga acguacuugc agaucucuca ccaggaaaga cugauacaga 1620

acgaucgaua cagaaaccac gcugccgcca ccacaccauc accaucgaca gaacaguccu 1680

uaauccagaa accugaaaug aaggaagagg agacucugcg cagagcacuu uggguccgga 1740

gggcgagacu ccggcggaag cauucccggg cgggugaccc agcacggucc cucuuggaau 1800

uggauucgcc auuuuauuuu ucuugcugcu aaaucaccga gcccggaaga uuagagaguu 1860

uuauuucugg gauuccugua gacacaccca cccacauaca uacauuuaua uauauauaua 1920

uuauauauau auaaaaauaa auaucucuau uuuauauaua uaaaauauau auauucuuuu 1980

uuuaaauuaa cagugcuaau guuauuggug ucuucacugg auguauuuga cugcugugga 2040

cuugaguugg gaggggaaug uucccacuca gauccugaca gggaagagga ggagaugaga 2100

gacucuggca ugaucuuuuu uuugucccac uugguggggc caggguccuc uccccugccc 2160

aggaaugugc aaggccaggg caugggggca aauaugaccc aguuuuggga acaccgacaa 2220

acccagcccu ggcgcugagc cucucuaccc caggucagac ggacagaaag acagaucaca 2280

gguacaggga ugaggacacc ggcucugacc aggaguuugg ggagcuucag gacauugcug 2340

ugcuuugggg auucccucca caugcugcac gcgcaucucg cccccagggg cacugccugg 2400

aagauucagg agccugggcg gccuucgcuu acucucaccu gcuucugagu ugcccaggag 2460

accacuggca gaugucccgg cgaagagaag agacacauug uuggaagaag cagcccauga 2520

cagcuccccu uccugggacu cgcccucauc cucuuccugc uccccuuccu ggggugcagc 2580

cuaaaaggac cuauguccuc acaccauuga aaccacuagu ucuguccccc caggagaccu 2640

gguugugugu gugugagugg uugaccuucc uccauccccu gguccuuccc uucccuuccc 2700

gaggcacaga gagacagggc aggauccacg ugcccauugu ggaggcagag aaaagagaaa 2760

guguuuuaua uacgguacuu auuuaauauc ccuuuuuaau uagaaauuaa aacaguuaau 2820

uuaauuaaag aguaggguuu uuuuucagua uucuugguua auauuuaauu ucaacuauuu 2880

augagaugua ucuuuugcuc ucucuugcuc ucuuauuugu accgguuuuu guauauaaaa 2940

uucauguuuc caaucucucu cucccugauc ggugacaguc acuagcuuau cuugaacaga 3000

uauuuaauuu ugcuaacacu cagcucugcc cuccccgauc cccuggcucc ccagcacaca 3060

uuccuuugaa auaagguuuc aauauacauc uacauacuau auauauauuu ggcaacuugu 3120

auuugugugu auauauauau auauauguuu auguauauau gugauucuga uaaaauagac 3180

auugcuauuc uguuuuuuau auguaaaaac aaaacaagaa aaaauagaga auucuacaua 3240

cuaaaucucu cuccuuuuuu aauuuuaaua uuuguuauca uuuauuuauu ggugcuacug 3300

uuuauccgua auaauugugg ggaaaagaua uuaacaucac gucuuugucu cuagugcagu 3360

uuuucgagau auuccguagu acauauuuau uuuuaaacaa cgacaaagaa auacagauau 3420

aucuuaaaaa aaaaaaagca uuuuguauua aagaauuuaa uucugaucuc aaaaaaaaaa 3480

aaaaaaaa 3488

<210> 32
<211> 171
<212> PRT
<213> Homo sapiens

<400> 32

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Lys Ser Val
    130                 135                 140

Arg Gly Lys Gly Lys Gly Gln Lys Arg Lys Arg Lys Lys Ser Arg Tyr
145                 150                 155                 160

Lys Ser Trp Ser Val Cys Asp Lys Pro Arg Arg
                165                 170

<210>  33
<211>  3494
<212>  RNA
<213>  Homo sapiens

<400>  33
ucgcggaggc uuggggcagc cggguagcuc ggaggucgug gcgcuggggg cuagcaccag      60

cgcucugucg ggaggcgcag cgguuaggug gaccggucag cggacucacc ggccagggcg     120

cucggugcug gaauuugaua uucauugauc cggguuuuau cccucuucuu uuuucuuaaa     180

cauuuuuuuu uaaaacugua uuguuucucg uuuuaauuua uuuuugcuug ccauucccca     240

cuugaaucgg gccgacggcu uggggagauu gcucuacuuc cccaaaucac uguggauuuu     300

ggaaaccagc agaaagagga aagagguagc aagagcucca gagagaaguc gaggaagaga     360

gagacggggu cagagagagc gcgcgggcgu gcgagcagcg aaagcgacag gggcaaagug     420

agugaccugc uuuugggggu gaccgccgga gcgcggcgug agcccucccc cuugggaucc     480

cgcagcugac cagucgcgcu gacggacaga cagacagaca ccgcccccag ccccagcuac     540

caccuccucc ccggccggcg gcggacagug gacgcggcgg cgagccgcgg gcagggccgg     600

gagcccgcgc ccggaggcgg gguggagggg gucggggcuc gcggcgucgc acugaaacuu     660

uucguccaac uucugggcug uucucgcuuc ggaggagccg ugguccgcgc gggggaagcc     720

gagccgagcg gagccgcgag aagugcuagc ucgggccggg aggagccgca gccggaggag     780

ggggaggagg aagaagagaa ggaagaggag agggggccgc aguggcgacu cggcgcucgg     840

aagccgggcu cauggacggg ugaggcggcg gugugcgcag acagugcucc agccgcgcgc 900

gcuccccagg cccuggcccg ggccucgggc cggggaggaa gaguagcucg ccgaggcgcc 960

gaggagagcg ggccgcccca cagcccgagc cggagaggga gcgcgagccg cgccggcccc 1020

ggucgggccu ccgaaaccau gaacuuucug cugucuuggg ugcauuggag ccuugccuug 1080

cugcucuacc uccaccaugc caaguggucc caggcugcac ccauggcaga aggaggaggg 1140

cagaaucauc acgaaguggu gaaguucaug gaugucuauc agcgcagcua cugccaucca 1200

aucgagaccc ugguggacau cuuccaggag uacccugaug agaucgagua caucuucaag 1260

ccauccugug ugccccugau gcgaugcggg ggcugcugca augacgaggg ccuggagugu 1320

gugcccacug aggaguccaa caucaccaug cagauuaugc ggaucaaacc ucaccaaggc 1380

cagcacauag gagagaugag cuuccuacag cacaacaaau gugaaugcag accaaagaaa 1440

gauagagcaa gacaagaaaa aaaaucaguu cgaggaaagg gaaaggggca aaaacgaaag 1500

cgcaagaaau cccgguauaa guccuggagc guaugugaca agccgaggcg gugagccggg 1560

caggaggaag gagccucccu caggguuucg ggaaccagau cucucaccag gaaagacuga 1620

uacagaacga ucgauacaga aaccacgcug ccgccaccac accaucacca ucgacagaac 1680

aguccuuaau ccagaaaccu gaaaugaagg aagaggagac ucugcgcaga gcacuuuggg 1740

uccggagggc gagacuccgg cggaagcauu cccgggcggg ugacccagca cggucccucu 1800

uggaauugga uucgccauuu uauuuuucuu gcugcuaaau caccgagccc ggaagauuag 1860

agaguuuuau uucugggauu ccuguagaca cacccaccca cauacauaca uuuauauaua 1920

uauauauuau auauauauaa aaauaaauau cucuauuuua uauauauaaa auauauauau 1980

ucuuuuuuua aauuaacagu gcuaauguua uuggugucuu cacuggaugu auuugacugc 2040

uguggacuug aguugggagg ggaauguucc cacucagauc cugacaggga agaggaggag 2100

augagagacu cuggcaugau cuuuuuuuug ucccacuugg uggggccagg guccucuccc 2160

cugcccagga augugcaagg ccagggcaug ggggcaaaua ugacccaguu uugggaacac 2220

cgacaaaccc agcccuggcg cugagccucu cuaccccagg ucagacggac agaaagacag 2280

aucacaggua cagggaugag gacaccggcu cugaccagga guuuggggag cuucaggaca 2340

uugcugugcu uuggggauuc ccuccacaug cugcacgcgc aucucgcccc caggggcacu 2400

gccuggaaga uucaggagcc ugggcggccu ucgcuuacuc ucaccugcuu cugaguugcc 2460

caggagacca cuggcagaug ucccggcgaa gagaagagac acauuguugg aagaagcagc 2520

ccaugacagc uccccuuccu gggacucgcc cucauccucu uccugcuccc cuuccugggg 2580

ugcagccuaa aaggaccuau guccucacac cauugaaacc acuaguucug uccccccagg 2640

agaccugguu gugugugugu gagugguuga ccuuccucca uccccugguc cuucccuucc 2700

cuucccgagg cacagagaga cagggcagga uccacgugcc cauuguggag gcagagaaaa 2760

gagaaagugu uuuauauacg guacuuauuu aauaucccuu uuuaauuaga aauuaaaaca 2820

guuaauuuaa uuaaagagua ggguuuuuuu ucaguauucu ugguuaauau uuaauuucaa 2880

cuauuuauga gauguaucuu uugcucucuc uugcucucuu auuuguaccg guuuuuguau 2940

auaaaauuca uguuuccaau cucucucucc cugaucggug acagucacua gcuuaucuug 3000

aacagauauu uaauuuugcu aacacucagc ucugcccucc ccgauccccu ggcuccccag 3060

cacacauucc uuugaaauaa gguuucaaua uacaucuaca uacuauauau auauuuggca 3120

acuuguauuu guguguauau auauauauau auguuuaugu auauauguga uucugauaaa 3180

auagacauug cuauucuguu uuuuauaugu aaaaacaaaa caagaaaaaa uagagaauuc 3240

uacauacuaa aucucucucc uuuuuuaauu uuaauauuug uuaucauuua uuuauuggug 3300

cuacuguuua uccguaauaa uuguggggaa aagauauuaa caucacgucu uugucucuag 3360

ugcaguuuuu cgagauauuc cguaguacau auuuauuuuu aaacaacgac aaagaaauac 3420

agauauaucu uaaaaaaaaa aaagcauuuu guauuaaaga auuuaauucu gaucucaaaa 3480

aaaaaaaaaa aaaa 3494

<210> 34
<211> 351
<212> PRT
<213> Homo sapiens

<400> 34

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Lys Lys Ser Val Arg Gly Lys Gly Lys Gly Gln Lys Arg Lys Arg Lys
                325                 330                 335

Lys Ser Arg Tyr Lys Ser Trp Ser Val Cys Asp Lys Pro Arg Arg
            340                 345                 350

<210>  35
<211>  3477

<212>  RNA
<213>  Homo sapiens

<400>  35
gcggaggcuu ggggcagccg gguagcucgg aggucguggc gcugggggcu agcaccagcg      60

cucugucggg aggcgcagcg guuaggugga ccggucagcg gacucaccgg ccagggcgcu     120

cggugcugga auuugauauu cauugauccg gguuuuaucc cucuucuuuu uucuuaaaca     180

uuuuuuuuua aaacuguauu guuucucguu uuaauuuauu uuugcuugcc auuccccacu     240

ugaaucgggc cgacggcuug gggagauugc ucuacuuccc caaaucacug uggauuuugg      300

aaaccagcag aaagaggaaa gagguagcaa gagcuccaga gagaagucga ggaagagaga     360

gacgggguca gagagagcgc gcgggcgugc gagcagcgaa agcgacaggg gcaaagugag     420

ugaccugcuu uuggggguga ccgccggagc gcggcgugag cccucccccu ugggaucccg     480

cagcugacca gucgcgcuga cggacagaca gacagacacc gcccccagcc ccagcuacca     540

ccuccucccc ggccggcggc ggacagugga cgcggcggcg agccgcgggc aggggccgga     600

gcccgcgccc ggaggcgggg uggagggggu cggggcucgc ggcgucgcac ugaaacuuuu     660

cguccaacuu cugggcuguu cucgcuucgg aggagccgug guccgcgcgg gggaagccga     720

gccgagcgga gccgcgagaa gugcuagcuc gggccgggag gagccgcagc cggaggaggg     780

ggaggaggaa gaagagaagg aagaggagag ggggccgcag uggcgacucg gcgcucggaa      840

gccgggcuca uggacgggug aggcggcggu gugcgcagac agugcuccag ccgcgcgcgc     900

uccccaggcc cuggcccggg ccucgggccg gggaggaaga guagcucgcc gaggcgccga     960

ggagagcggg ccgccccaca gcccgagccg gagagggagc gcgagccgcg ccggccccgg    1020

ucgggccucc gaaaccauga acuuucugcu gucuugggug cauuggagcc uugccuugcu    1080

gcucuaccuc caccaugcca agugguccca ggcugcaccc auggcagaag gaggagggca    1140

gaaucaucac gaagugguga aguucaugga ugucuaucag cgcagcuacu gccauccaau    1200

cgagacccug guggacaucu uccaggagua cccugaugag aucgaguaca ucuucaagcc    1260

auccugugug ccccugaugc gaugcggggg cugcugcaau gacgagggcc uggagugugu    1320

gcccacugag gaguccaaca ucaccaugca gauuaugcgg aucaaaccuc accaaggcca    1380

gcacauagga gagaugagcu uccuacagca caacaaaugu gaaugcagac caaagaaaga    1440

uagagcaaga caagaaaaaa aaucaguucg aggaaaggga aaggggcaaa aacgaaagcg    1500

caagaaaucc cgguauaagu ccuggagcgu augugacaag ccgaggcggu gagccgggca    1560

ggaggaagga gccucccuca ggguuucggg aaccagaucu cucaccagga aagacugaua    1620

cagaacgauc gauacagaaa ccacgcugcc gccaccacac caucaccauc gacagaacag    1680

uccuuaaucc agaaaccuga aaugaaggaa gaggagacuc ugcgcagagc acuuuggguc     1740

cggagggcga gacuccggcg gaagcauucc cgggcgggug acccagcacg gucccucuug    1800

gaauuggauu cgccauuuua uuuuucuugc ugcuaaauca ccgagcccgg aagauuagag 1860

aguuuuauuu cugggauucc uguagacaca cccacccaca uacauacauu uauauauaua 1920

uauauuauau auauauaaaa auaaauaucu cuauuuuaua uauauaaaau auauauauuc 1980

uuuuuuuaaa uuaacagugc uaauguuauu ggugucuuca cuggauguau uugacugcug 2040

uggacuugag uugggagggg aauguuccca cucagauccu gacagggaag aggaggagau 2100

gagagacucu ggcaugaucu uuuuuuuguc ccacuuggug gggccagggu ccucuccccu 2160

gcccaggaau gugcaaggcc agggcauggg ggcaaauaug acccaguuuu gggaacaccg 2220

acaaacccag cccuggcgcu gagccucucu accccagguc agacggacag aaagacagau 2280

cacagguaca gggaugagga caccggcucu gaccaggagu uuggggagcu ucaggacauu 2340

gcugugcuuu ggggauuccc uccacaugcu gcacgcgcau cucgccccca ggggcacugc 2400

cuggaagauu caggagccug ggcggccuuc gcuuacucuc accugcuucu gaguugccca 2460

ggagaccacu ggcagauguc ccggcgaaga gaagagacac auuguuggaa gaagcagccc 2520

augacagcuc cccuuccugg gacucgcccu cauccucuuc cugcuccccu uccuggggug 2580

cagccuaaaa ggaccuaugu ccucacacca uugaaaccac uaguucuguc cccccaggag 2640

accugguugu guguguguga gugguugacc uuccuccauc cccugguccu ucccuucccu 2700

ucccgaggca cagagagaca gggcaggauc cacgugccca uuguggaggc agagaaaaga 2760

gaaaguguuu uauauacggu acuuauuuaa uaucccuuuu uaauuagaaa uuaaaacagu 2820

uaauuuaauu aaagaguagg guuuuuuuuc aguauucuug guuaauauuu aauuucaacu 2880

auuuaugaga uguaucuuuu gcucucucuu gcucucuuau uuguaccggu uuuuguauau 2940

aaaauucaug uuuccaaucu cucucucccu gaucggugac agucacuagc uuaucuugaa 3000

cagauauuua auuuugcuaa cacucagcuc ugcccucccc gauccccugg cuccccagca 3060

cacauuccuu ugaaauaagg uuucaauaua caucuacaua cuauauauau auuuggcaac 3120

uuguauuugu guguauauau auauauauau guuuauguau auaugugauu cugauaaaau 3180

agacauugcu auucuguuuu uuauauguaa aaacaaaaca agaaaaaaua gagaauucua 3240

cauacuaaau cucucuccuu uuuuaauuuu aauauuuguu aucauuuauu uauuggugcu 3300

acuguuuauc cguaauaauu guggggaaaa gauauuaaca ucacgucuuu gucucuagug 3360

caguuuuucg agauauuccg uaguacauau uuauuuuuaa acaacgacaa agaaauacag 3420

auauaucuua aaaaaaaaaa agcauuuugu auuaaagaau uuaauucuga ucucaaa 3477

<210> 36
<211> 163
<212> PRT
<213> Homo sapiens

<400>  36

Met Ala Glu Gly Gly Gly Gln Asn His His Glu Val Val Lys Phe Met
1               5                   10                  15

Asp Val Tyr Gln Arg Ser Tyr Cys His Pro Ile Glu Thr Leu Val Asp
            20                  25                  30

Ile Phe Gln Glu Tyr Pro Asp Glu Ile Glu Tyr Ile Phe Lys Pro Ser
        35                  40                  45

Cys Val Pro Leu Met Arg Cys Gly Gly Cys Cys Asn Asp Glu Gly Leu
    50                  55                  60

Glu Cys Val Pro Thr Glu Glu Ser Asn Ile Thr Met Gln Ile Met Arg
65                  70                  75                  80

Ile Lys Pro His Gln Gly Gln His Ile Gly Glu Met Ser Phe Leu Gln
                85                  90                  95

His Asn Lys Cys Glu Cys Arg Pro Lys Lys Asp Arg Ala Arg Gln Glu
            100                 105                 110

Asn Pro Cys Gly Pro Cys Ser Glu Arg Arg Lys His Leu Phe Val Gln
        115                 120                 125

Asp Pro Gln Thr Cys Lys Cys Ser Cys Lys Asn Thr Asp Ser Arg Cys
    130                 135                 140

Lys Ala Arg Gln Leu Glu Leu Asn Glu Arg Thr Cys Arg Cys Asp Lys
145                 150                 155                 160

Pro Arg Arg

<210>  37
<211>  2569
<212>  RNA
<213>  Homo sapiens

<400>  37
   gcggagccga uuacaucagc ccgggccugg ccggccgcgu guucccggag ccucggcugc      60
   ccgaaugggg agcccagagu ggcgagcggc accccucccc ccgccagccc uccgcgggaa     120
   ggugaccucu cgaguggucc caggcugcac ccauggcaga aggaggaggg cagaaucauc     180
   acgaaguggu gaaguucaug gaugucuauc agcgcagcua cugccaucca aucgagaccc     240
   ugguggacau cuuccaggag uacccugaug agaucgagua caucuucaag ccauccugug     300
   ugccccugau gcgaugcggg ggcugcugca augacgaggg ccuggagugu gugcccacug     360
   aggaguccaa caucaccaug cagauuaugc ggaucaaacc ucaccaaggc cagcacauag 420
   gagagaugag cuuccuacag cacaacaaau gugaaugcag accaaagaaa gauagagcaa 480
   gacaagaaaa ucccuguggg ccuugcucag agcggagaaa gcauuuguuu guacaagauc 540
   cgcagacgug uaaauguucc ugcaaaaaca cagacucgcg uugcaaggcg aggcagcuug 600
   aguuaaacga acguacuugc agaugugaca agccgaggcg gugagccggg caggaggaag 660
   gagccucccu caggguuucg ggaaccagau cucucaccag gaaagacuga uacagaacga 720
   ucgauacaga aaccacgcug ccgccaccac accaucacca ucgacagaac aguccuuaau 780
   ccagaaaccu gaaaugaagg aagaggagac ucugcgcaga gcacuuuggg uccggagggc 840
   gagacuccgg cggaagcauu cccgggcggg ugacccagca cggucccucu uggaauugga 900
   uucgccauuu uauuuuucuu gcugcuaaau caccgagccc ggaagauuag agaguuuuau 960
   uucugggauu ccuguagaca cacccaccca cauacauaca uuuauauaua uauauauuau 1020
   auauauauaa aaauaaauau cucuauuuua uauauauaaa auauauauau ucuuuuuuua 1080
   aauuaacagu gcuaauguua uuggugucuu cacuggaugu auuugacugc uguggacuug 1140
   aguugggagg ggaauguucc cacucagauc cugacaggga agaggaggag augagagacu 1200
   cuggcaugau cuuuuuuuug ucccacuugg uggggccagg guccucuccc cugcccagga 1260
   augugcaagg ccagggcaug gggcaaauaa ugacccaguu uugggaacac cgacaaaccc 1320
   agcccuggcg cugagccucu cuaccccagg ucagacggac agaaagacag aucacaggua 1380
   cagggaugag gacaccggcu cugaccagga guuuggggag cuucaggaca uugcugugcu 1440
   uuggggauuc ccuccacaug cugcacgcgc aucucgcccc caggggcacu gccuggaaga 1500
   uucaggagcc ugggcggccu ucgcuuacuc ucaccugcuu cugaguugcc caggagacca 1560
   cuggcagaug ucccggcgaa gagaagagac acauuguugg aagaagcagc ccaugacagc 1620
   uccccuuccu gggacucgcc cucauccucu uccugcuccc cuuccugggg ugcagccuaa 1680
   aaggaccuau guccucacac cauugaaacc acuaguucug uccccccagg agaccugguu 1740
   gugugugugu gagugguuga ccuuccucca uccccugguc cuucccuucc cuucccgagg 1800
   cacagagaga cagggcagga uccacgugcc cauuguggag gcagagaaaa gagaaagugu 1860
   uuuauauacg guacuuauuu aauaucccuu uuuaauuaga aauuaaaaca guuaauuuaa 1920
   uuaaagagua ggguuuuuuu ucaguauucu ugguuaauau uuaauuucaa cuauuuauga 1980
   gauguaucuu uugcucucuc uugcucucuu auuuguaccg guuuuuguau auaaaauuca 2040
   uguuuccaau cucucucucc cugaucggug acagucacua gcuuaucuug aacagauauu 2100
   uaauuuugcu aacacucagc ucugcccucc ccgauccccu ggcuccccag cacacauucc 2160
   uuugaaauaa gguuucaaua uacaucuaca uacuauauau auauuuggca acuuguauuu 2220
   guguguauau auauauauau auguuuaugu auauauguga uucugauaaa auagacauug 2280
   cuauucuguu uuuuauaugu aaaaacaaaa caagaaaaaa uagagaauuc uacauacuaa 2340
   aucucucucc uuuuuuaauu uuaauauuug uuaucauuua uuuauuggug cuacuguuua 2400
   uccguaauaa uuguggggaa aagauauuaa caucacgucu uugucucuag ugcaguuuuu 2460
   cgagauauuc cguaguacau auuuauuuuu aaacaacgac aaagaaauac agauauaucu 2520
   uaaaaaaaaa aaagcauuuu guauuaaaga auuuaauucu gaucucaaa 2569

<210> 38
<211> 213
<212> PRT
<213> Homo sapiens

<400> 38

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Asn Pro Cys Gly
    130                 135                 140

Pro Cys Ser Glu Arg Arg Lys His Leu Phe Val Gln Asp Pro Gln Thr
145                 150                 155                 160

Cys Lys Cys Ser Cys Lys Asn Thr Asp Ser Arg Cys Lys Ala Arg Gln
                165                 170                 175

Leu Glu Leu Asn Glu Arg Thr Cys Arg Cys Asp Lys Pro Arg Arg Ser
            180                 185                 190

Ala Gly Gln Glu Glu Gly Ala Ser Leu Arg Val Ser Gly Thr Arg Ser
        195                 200                 205

Leu Thr Arg Lys Asp
    210

<210>  39
<211>  3554
<212>  RNA
<213>  Homo sapiens

<400>  39
ucgcggaggc uuggggcagc cggguagcuc ggaggucgug gcgcuggggg cuagcaccag      60

cgcucugucg ggaggcgcag cgguuaggug gaccggucag cggacucacc ggccagggcg     120

cucggugcug gaauuugaua uucauugauc cggguuuuau cccucuucuu uuuucuuaaa     180

cauuuuuuuu uaaaacugua uuguuucucg uuuuaauuua uuuuugcuug ccauucccca     240

cuugaaucgg gccgacggcu uggggagauu gcucuacuuc cccaaaucac uguggauuuu     300

ggaaaccagc agaaagagga aagagguagc aagagcucca gagagaaguc gaggaagaga     360

gagacggggu cagagagagc gcgcgggcgu gcgagcagcg aaagcgacag gggcaaagug     420

agugaccugc uuuugggggu gaccgccgga gcgcggcgug agcccucccc cuugggaucc     480

cgcagcugac cagucgcgcu gacggacaga cagacagaca ccgcccccag ccccagcuac     540

caccuccucc ccggccggcg gcggacagug gacgcggcgg cgagccgcgg gcaggggccg     600

gagcccgcgc ccggaggcgg gguggagggg gucggggcuc gcggcgucgc acugaaacuu     660

uucguccaac uucugggcug uucucgcuuc ggaggagccg ugguccgcgc gggggaagcc     720

gagccgagcg gagccgcgag aagugcuagc ucgggccggg aggagccgca gccggaggag     780

ggggaggagg aagaagagaa ggaagaggag agggggccgc aguggcgacu cggcgcucgg     840

aagccgggcu cauggacggg ugaggcggcg gugugcgcag acagugcucc agccgcgcgc     900

gcuccccagg cccuggcccg ggccucgggc cggggaggaa gaguagcucg ccgaggcgcc     960

gaggagagcg ggccgcccca cagcccgagc cggagaggga gcgcgagccg cgccggcccc    1020

ggucgggccu ccgaaaccau gaacuuucug cugucuuggg ugcauuggag ccuugccuug    1080

cugcucuacc uccaccaugc caaguggucc caggcugcac ccauggcaga aggaggaggg    1140

cagaaucauc acgaaguggu gaaguucaug gaugucuauc agcgcagcua cugccaucca    1200

aucgagaccc ugguggacau cuuccaggag uacccugaug agaucgagua caucuucaag    1260

ccauccugug ugccccugau gcgaugcggg ggcugcugca augacgaggg ccuggagugu    1320

gugcccacug aggaguccaa caucaccaug cagauuaugc ggaucaaacc ucaccaaggc 1380

cagcacauag gagagaugag cuuccuacag cacaacaaau gugaaugcag accaaagaaa 1440

gauagagcaa gacaagaaaa ucccuguggg ccuugcucag agcggagaaa gcauuuguuu 1500

guacaagauc cgcagacgug uaaauguucc ugcaaaaaca cagacucgcg uugcaaggcg 1560

aggcagcuug aguuaaacga acguacuugc agaugugaca agccgaggcg gugagccggg 1620

caggaggaag gagccucccu cagggguucg ggaaccagau cucucaccag gaaagacuga 1680

uacagaacga ucgauacaga aaccacgcug ccgccaccac accaucacca ucgacagaac 1740

aguccuuaau ccagaaaccu gaaaugaagg aagaggagac ucugcgcaga gcacuuuggg 1800

uccggagggc gagacuccgg cggaagcauu cccgggcggg ugacccagca cggucccucu 1860

uggaauugga uucgccauuu uauuuuucuu gcugcuaaau caccgagccc ggaagauuag 1920

agaguuuuau uucugggauu ccuguagaca cacccaccca cauacauaca uuuauauaua 1980

uauauauuau auauauauaa aaauaaauau cucuauuuua uauauauaaa auauauauau 2040

ucuuuuuuua aauuaacagu gcuaauguua uuggugucuu cacuggaugu auuugacugc 2100

uguggacuug aguuggaggg ggaauguucc cacucagauc cugacaggga agaggaggag 2160

augagagacu cuggcaugau cuuuuuuuug ucccacuugg uggggccagg guccucuccc 2220

cugcccagga augugcaagg ccagggcaug gggcaaauau gacccaguuu uggaacaccc 2280

cgacaaaccc agcccuggcg cugagccucu cuaccccagg ucagacggac agaaagacag 2340

aucacaggua cagggaugag gacaccggcu cugaccagga guuuggggag cuucaggaca 2400

uugcugugcu uggggauucc ccuccacaug cugcacgcgc aucucgcccc caggggcacu 2460

gccuggaaga uucaggagcc ugggcggccu ucgcuuacuc ucaccugcuu cugaguugcc 2520

caggagacca cuggcagaug ucccggcgaa gagaagagac acauuguugg aagaagcagc 2580

ccaugacagc uccccuuccu gggacucgcc cucauccucu uccugcuccc cuuccugggg 2640

ugcagccuaa aaggaccuau guccucacac cauugaaacc acuaguucug uccccccagg 2700

agaccugguu gugugugugu gagugguuga ccuuccucca uccccugguc cuucccuucc 2760

cuucccgagg cacagagaga cagggcagga uccacgugcc cauuguggag gcagagaaaa 2820

gagaaagugu uuuauauacg guacuuauuu aauaucccuu uuuaauuaga aauuaaaaca 2880

guuaauuuaa uuaaagagua ggguuuuuuu ucaguauucu ugguuaauau uuaauuucaa 2940

cuauuuauga gauguaucuu uugcucucuc uugcucucuu auuuguaccg guuuuuguau 3000

auaaaauuca uguuuccaau cucucucucc cugaucggug acagucacua gcuuaucuug 3060

aacagauauu uaauuuugcu aacacucagc ucugcccucc ccgauccccu ggcuccccag 3120

cacacauucc uuugaaauaa gguuucaaua uacaucuaca uacuauauau auauuuggca 3180

acuuguauuu guguguauau auauauauau auguuuaugu auauauguga uucugauaaa 3240

auagacauug cuauucuguu uuuuauaugu aaaaacaaaa caagaaaaaa uagagaauuc 3300

uacauacuaa aucucucucc uuuuuuaauu uuaauauuug uuaucauuua uuuauuggug 3360

cuacuguuua uccguaauaa uuguggggaa aagauauuaa caucacgucu uugucucuag 3420

ugcaguuuuu cgagauauuc cguaguacau auuuauuuuu aaacaacgac aaagaaauac 3480

agauauaucu uaaaaaaaaa aaagcauuuu guauuaaaga auuuaauucu gaucucaaaa 3540

aaaaaaaaaa aaaa 3554

<210> 40
<211> 188
<212> PRT
<213> Homo sapiens

<400> 40

Met Ser Pro Leu Leu Arg Arg Leu Leu Leu Ala Ala Leu Leu Gln Leu
1               5                   10                  15

Ala Pro Ala Gln Ala Pro Val Ser Gln Pro Asp Ala Pro Gly His Gln
            20                  25                  30

Arg Lys Val Val Ser Trp Ile Asp Val Tyr Thr Arg Ala Thr Cys Gln
        35                  40                  45

Pro Arg Glu Val Val Val Pro Leu Thr Val Glu Leu Met Gly Thr Val
    50                  55                  60

Ala Lys Gln Leu Val Pro Ser Cys Val Thr Val Gln Arg Cys Gly Gly
65                  70                  75                  80

Cys Cys Pro Asp Asp Gly Leu Glu Cys Val Pro Thr Gly Gln His Gln
                85                  90                  95

Val Arg Met Gln Ile Leu Met Ile Arg Tyr Pro Ser Ser Gln Leu Gly
            100                 105                 110

Glu Met Ser Leu Glu Glu His Ser Gln Cys Glu Cys Arg Pro Lys Lys
        115                 120                 125

Lys Asp Ser Ala Val Lys Pro Asp Ser Pro Arg Pro Leu Cys Pro Arg
    130                 135                 140

Cys Thr Gln His His Gln Arg Pro Asp Pro Arg Thr Cys Arg Cys Arg
145                 150                 155                 160

Cys Arg Arg Arg Ser Phe Leu Arg Cys Gln Gly Arg Gly Leu Glu Leu
                165                 170                 175

Asn Pro Asp Thr Cys Arg Cys Arg Lys Leu Arg Arg
            180                 185

<210>  41
<211>  1704
<212>  RNA
<213>  Homo sapiens

<400>  41
gccguccccg ccgccgcugc ccgccgccac cggccgcccg cccgcccggc uccuccggcc      60

gccuccgcug cgcugcgcug cgcugccugc acccagggcu cgggaggggg ccgcggagga     120

gucgcccccc gcgcccggcc cccgcccgcc gcgcccgggc ccgcgccaug gggcucuggc      180

ugucgccgcc ccccgcgccg ccgggcuagg gcgaugcggg cgcccccggc gggcggcccc     240

ggcgggcacc augagcccuc ugcuccgccg ccugcugcuc gccgcacucc ugcagcuggc     300

ccccgcccag gccccugucu cccagccuga ugccccuggc caccagagga aguggugguc     360

auggauagau guguauacuc gcgcuaccug ccagccccgg gagguggugg ugcccuugac     420

uguggagcuc augggcaccg uggccaaaca gcuggugccc agcugcguga cugugcagcg     480

cugugguggc ugcugcccug acgauggccu ggagugugug cccacugggc agcaccaagu     540

ccggaugcag auccucauga uccgguaccc gagcagucag cugggggaga ugucccugga     600

agaacacagc cagugugaau gcagaccuaa aaaaaaggac agugcuguga agccagacag     660

ccccaggccc cucugcccac gcugcaccca gcaccaccag cgcccugacc cccggaccug     720

ccgcugccgc ugccgacgcc gcagcuuccu ccguugccaa gggcggggcu uagagcucaa     780

cccagacacc ugcaggugcc ggaagcugcg aaggugacac auggcuuuuc agacucagca     840

gggugacuug ccucagaggc uauaucccag ugggggaaca aagaggagcc ugguaaaaaa     900

cagccaagcc cccaagaccu cagcccaggc agaagcugcu cuaggaccug ggccucucag     960

agggcucuuc ugccaucccu ugucucccug aggccaucau caaacaggac agaguuggaa    1020

gaggagacug ggaggcagca agagggguca cauaccagcu caggggagaa uggaguacug    1080

ucucaguuuc uaaccacucu gugcaaguaa gcaucuuaca acuggcucuu ccuccccuca    1140

cuaagaagac ccaaaccucu gcauaauggg auuugggcuu ugguacaaga acugugaccc    1200

ccaacccuga uaaaagagau ggaaggagcu gucccugccu gugucacugu uugucacugu    1260

ccaggcuggc ugguuugggc augaaugucu gcaucacuaa auccagagcu ugucuugcuc    1320

ccucauugug cagauggagg aaaugaggac uaaggcccca cagcagaucc caggcagggc    1380

cagaauuaug uauucaucac uuucaaguua uugccacgca ugggagucag ggauagccca    1440

gucaauacag acugccugcc cuccugcucu ucaccagggu ucuuuucuag aaggagacag    1500

ccuucugugg ccagagagcu ugggguagga cccagaucua cugagugacc uugcuuguca    1560

cuaccccugc cucucugagc agcaguuucc acaugugcac auagagggaa cagaagauug 1620

cugugguugg cguccucggg ccccagagaa guuugagacu aucuuuacgu aauagaaaag 1680

aacacuuguu cuuccugcca ggca 1704

<210> 42
<211> 207
<212> PRT
<213> Homo sapiens

<400> 42

Met Ser Pro Leu Leu Arg Arg Leu Leu Leu Ala Ala Leu Leu Gln Leu
1               5                   10                  15

Ala Pro Ala Gln Ala Pro Val Ser Gln Pro Asp Ala Pro Gly His Gln
            20                  25                  30

Arg Lys Val Val Ser Trp Ile Asp Val Tyr Thr Arg Ala Thr Cys Gln
        35                  40                  45

Pro Arg Glu Val Val Val Pro Leu Thr Val Glu Leu Met Gly Thr Val
    50                  55                  60

Ala Lys Gln Leu Val Pro Ser Cys Val Thr Val Gln Arg Cys Gly Gly
65                  70                  75                  80

Cys Cys Pro Asp Asp Gly Leu Glu Cys Val Pro Thr Gly Gln His Gln
                85                  90                  95

Val Arg Met Gln Ile Leu Met Ile Arg Tyr Pro Ser Ser Gln Leu Gly
            100                 105                 110

Glu Met Ser Leu Glu Glu His Ser Gln Cys Glu Cys Arg Pro Lys Lys
        115                 120                 125

Lys Asp Ser Ala Val Lys Pro Asp Arg Ala Ala Thr Pro His His Arg
    130                 135                 140

Pro Gln Pro Arg Ser Val Pro Gly Trp Asp Ser Ala Pro Gly Ala Pro
145                 150                 155                 160

Ser Pro Ala Asp Ile Thr His Pro Thr Pro Ala Pro Gly Pro Ser Ala
                165                 170                 175

His Ala Ala Pro Ser Thr Thr Ser Ala Leu Thr Pro Gly Pro Ala Ala
            180                 185                 190

Ala Ala Ala Asp Ala Ala Ala Ser Ser Val Ala Lys Gly Gly Ala
        195                 200                 205

<210>  43
<211>  1805
<212>  RNA
<213>  Homo sapiens

<400>  43
gccguccccg ccgccgcugc ccgccgccac cggccgcccg cccgcccggc uccuccggcc      60

gccuccgcug cgcugcgcug cgcugccugc acccagggcu cgggaggggg ccgcggagga     120

gucgcccccc gcgcccggcc cccgcccgcc gcgcccgggc ccgcgccaug ggcucuggcc     180

ugucgccgcc ccccgcgccg ccgggcuagg gcgaugcggg cgcccccggc gggcggcccc     240

ggcgggcacc augagcccuc ugcuccgccg ccugcugcuc gccgcacucc ugcagcuggc     300

ccccgcccag gccccugucu cccagccuga ugccccuggc caccagagga aagugguguc     360

auggauagau guguauacuc gcgcuaccug ccagccccgg gagguggugg ugcccuugac     420

uguggagcuc augggcaccg uggccaaaca gcuggugccc agcugcguga cugugcagcg     480

cugugguggc ugcugcccug acgauggccu ggagugugug cccacugggc agcaccaagu     540

ccggaugcag auccucauga uccgguaccc gagcagucag cugggggaga ugucccugga     600

agaacacagc cagugugaau gcagaccuaa aaaaaaggac agugcuguga agccagacag     660

ggcugccacu ccccaccacc guccccagcc ccguucuguu ccgggcuggg acucugcccc     720

cggagcaccc uccccagcug acaucaccca ucccacucca gccccaggcc ccucugccca     780

cgcugcaccc agcaccacca gcgcccugac ccccggaccu gccgcugccg cugccgacgc     840

cgcagcuucc uccguugcca agggcggggc uuagagcuca acccagacac cugcaggugc     900

cggaagcugc gaaggugaca cauggcuuuu cagacucagc agggugacuu gccucagagg     960

cuauauccca gugggggaac aaagaggagc cugguaaaaa acagccaagc ccccaagacc    1020

ucagcccagg cagaagcugc ucuaggaccu gggccucuca gagggcucuu cugccauccc    1080

uugucucccu gaggccauca ucaaacagga cagaguugga agaggagacu gggaggcagc    1140

aagagggguc acauaccagc ucaggggaga auggaguacu gucucaguuu cuaaccacuc    1200

ugugcaagua agcaucuuac aacuggcucu uccuccccuc acuaagaaga cccaaaccuc    1260

ugcauaaugg gauuugggcu uugguacaag aacugugacc cccaacccug auaaaagaga    1320

uggaaggagc ugucccugcc ugugucacug uuugucacug uccaggcugg cugguuuggg    1380

caugaauguc ugcaucacua aauccagagc uugucuugcu cccucauugu gcagauggag    1440

gaaaugagga cuaaggcccc acagcagauc ccaggcaggg ccagaauuau guauucauca    1500

cuuucaaguu auugccacgc augggaguca gggauagccc agucaauaca gacugccugc    1560

ccuccugcuc uucaccaggg uucuuuucua gaaggagaca gccuucugug gccagagagc    1620

uugggguagg acccagaucu acugagugac cuugcuuguc acuaccccug ccucucugag 1680

cagcaguuuc cacaugugca cauagaggga acagaagauu gcugugguug gcguccucgg 1740

gccccagaga aguuugagac uaucuuuacg uaauagaaaa gaacacuugu ucuuccugcc 1800

aggca 1805

<210> 44
<211> 419
<212> PRT
<213> Homo sapiens

<400> 44

Met His Leu Leu Gly Phe Phe Ser Val Ala Cys Ser Leu Leu Ala Ala
1               5                   10                  15

Ala Leu Leu Pro Gly Pro Arg Glu Ala Pro Ala Ala Ala Ala Ala Phe
            20                  25                  30

Glu Ser Gly Leu Asp Leu Ser Asp Ala Glu Pro Asp Ala Gly Glu Ala
        35                  40                  45

Thr Ala Tyr Ala Ser Lys Asp Leu Glu Glu Gln Leu Arg Ser Val Ser
    50                  55                  60

Ser Val Asp Glu Leu Met Thr Val Leu Tyr Pro Glu Tyr Trp Lys Met
65                  70                  75                  80

Tyr Lys Cys Gln Leu Arg Lys Gly Gly Trp Gln His Asn Arg Glu Gln
                85                  90                  95

Ala Asn Leu Asn Ser Arg Thr Glu Glu Thr Ile Lys Phe Ala Ala Ala
            100                 105                 110

His Tyr Asn Thr Glu Ile Leu Lys Ser Ile Asp Asn Glu Trp Arg Lys
        115                 120                 125

Thr Gln Cys Met Pro Arg Glu Val Cys Ile Asp Val Gly Lys Glu Phe
    130                 135                 140

Gly Val Ala Thr Asn Thr Phe Phe Lys Pro Pro Cys Val Ser Val Tyr
145                 150                 155                 160

Arg Cys Gly Gly Cys Cys Asn Ser Glu Gly Leu Gln Cys Met Asn Thr
                165                 170                 175

Ser Thr Ser Tyr Leu Ser Lys Thr Leu Phe Glu Ile Thr Val Pro Leu
            180                 185                 190

Ser Gln Gly Pro Lys Pro Val Thr Ile Ser Phe Ala Asn His Thr Ser
        195                 200                 205

Cys Arg Cys Met Ser Lys Leu Asp Val Tyr Arg Gln Val His Ser Ile
    210                 215                 220

Ile Arg Arg Ser Leu Pro Ala Thr Leu Pro Gln Cys Gln Ala Ala Asn
225                 230                 235                 240

Lys Thr Cys Pro Thr Asn Tyr Met Trp Asn Asn His Ile Cys Arg Cys
                245                 250                 255

Leu Ala Gln Glu Asp Phe Met Phe Ser Ser Asp Ala Gly Asp Asp Ser
            260                 265                 270

Thr Asp Gly Phe His Asp Ile Cys Gly Pro Asn Lys Glu Leu Asp Glu
        275                 280                 285

Glu Thr Cys Gln Cys Val Cys Arg Ala Gly Leu Arg Pro Ala Ser Cys
    290                 295                 300

Gly Pro His Lys Glu Leu Asp Arg Asn Ser Cys Gln Cys Val Cys Lys
305                 310                 315                 320

Asn Lys Leu Phe Pro Ser Gln Cys Gly Ala Asn Arg Glu Phe Asp Glu
                325                 330                 335

Asn Thr Cys Gln Cys Val Cys Lys Arg Thr Cys Pro Arg Asn Gln Pro
            340                 345                 350

Leu Asn Pro Gly Lys Cys Ala Cys Glu Cys Thr Glu Ser Pro Gln Lys
        355                 360                 365

Cys Leu Leu Lys Gly Lys Lys Phe His His Gln Thr Cys Ser Cys Tyr
    370                 375                 380

Arg Arg Pro Cys Thr Asn Arg Gln Lys Ala Cys Glu Pro Gly Phe Ser
385                 390                 395                 400

Tyr Ser Glu Glu Val Cys Arg Cys Val Pro Ser Tyr Trp Lys Arg Pro
                405                 410                 415

Gln Met Ser

<210>  45

<211>  2259
<212>  RNA
<213>  Homo sapiens

<400>  45
augcccugcc ccugcgcccg ccgccgccgc cgccgccgcu cagcccggcg cgcucuggag      60

gauccugcgc cgcggcgcuc ccgggccccg ccgccgccag ccgccccgcc gcccuccucc     120

cgcccccggc accgccgcca gcgcccccgc cgcagcgccc gcggcccggc uccucucacu     180

ucggggaagg ggagggagga gggggacgag ggcucuggcg gguuuggagg ggcugaacau     240

cgcggggugu ucuggugucc cccgccccgc cucuccaaaa agcuacaccg acgcggaccg     300

cggcggcguc cucccucgcc cucgcuucac cucgcgggcu ccgaaugcgg ggagcucgga     360

uguccgguuu ccugugaggc uuuuaccuga cacccgccgc cuuuccccgg cacuggcugg     420

gagggcgccc ugcaaaguug ggaacgcgga gccccggacc cgcucccgcc gccuccggcu     480

cgcccagggg gggucgccgg gaggagcccg ggggagaggg accaggaggg gcccgcggcc     540

ucgcaggggc gcccgcgccc ccaccccugc ccccgccagc ggaccggucc cccacccccg     600

guccuuccac caugcacuug cugggcuucu ucucuguggc guguucucug cucgccgcug     660

cgcugcuccc ggguccucgc gaggcgcccg ccgccgccgc cgccuucgag uccggacucg     720

accucucgga cgcggagccc gacgcgggcg aggccacggc uuaugcaagc aaagaucugg     780

aggagcaguu acggucugug uccaguguag augaacucau gacuguacuc uacccagaau     840

auuggaaaau guacaagugu cagcuaagga aaggaggcug gcaacauaac agagaacagg     900

ccaaccucaa cucaaggaca gaagagacua uaaaauuugc ugcagcacau uauaauacag     960

agaucuugaa aaguauugau aaugagugga gaaagacuca augcaugcca cgggaggugu    1020

guauagaugu ggggaaggag uuuggagucg cgacaaacac cuucuuuaaa ccuccaugug    1080

uguccgucua cagauguggg gguugcugca auagugaggg gcugcagugc augaacacca    1140

gcacgagcua ccucagcaag acguuauuug aaauuacagu gccucucucu caaggcccca    1200

aaccaguaac aaucaguuuu gccaaucaca cuuccugccg augcaugucu aaacuggaug    1260

uuuacagaca aguucauucc auuauuagac guucccugcc agcaacacua ccacaguguc    1320

aggcagcgaa caagaccugc cccaccaauu acauguggaa uaaucacauc ugcagaugcc    1380

uggcucagga agauuuuaug uuuuccucgg augcuggaga ugacucaaca gauggauucc    1440

augacaucug uggaccaaac aaggagcugg augaagagac cugucagugu gucugcagag    1500

cggggcuucg gccugccagc uguggacccc acaaagaacu agacagaaac ucaugccagu    1560

gugucuguaa aaacaaacuc uuccccagcc aauguggggc caaccgagaa uuugaugaaa    1620

acacaugcca guguguaugu aaaagaaccu gccccagaaa ucaaccccua aauccuggaa    1680

aaugugccug ugaauguaca gaaaguccac agaaaugcuu guuaaaagga aagaaguucc    1740

accaccaaac augcagcugu uacagacggc cauguacgaa ccgccagaag gcuugugagc 1800

caggauuuuc auauagugaa gaaguguguc guuguguccc uucauauugg aaaagaccac 1860

aaaugagcua agauuguacu guuuuccagu ucaucgauuu ucuauuaugg aaaacugugu 1920

ugccacagua gaacugucug ugaacagaga gacccuugug gguccaugcu aacaaagaca 1980

aaagucuguc uuuccugaac cauguggaua acuuuacaga aauggacugg agcucaucug 2040

caaaaggccu cuuguaaaga cugguuuucu gccaaugacc aaacagccaa gauuuuccuc 2100

uugugauuuc uuuaaaagaa ugacuauaua auuuauuucc acuaaaaaua uuguuucugc 2160

auucauuuuu auagcaacaa caauugguaa aacucacugu gaucaauauu uuuauaucau 2220

gcaaaauaug uuuaaaauaa aaugaaaauu guauuauaa 2259

<210> 46
<211> 354
<212> PRT
<213> Homo sapiens

<400> 46

Met Tyr Arg Glu Trp Val Val Val Asn Val Phe Met Met Leu Tyr Val
1               5                   10                  15

Gln Leu Val Gln Gly Ser Ser Asn Glu His Gly Pro Val Lys Arg Ser
            20                  25                  30

Ser Gln Ser Thr Leu Glu Arg Ser Glu Gln Gln Ile Arg Ala Ala Ser
        35                  40                  45

Ser Leu Glu Glu Leu Leu Arg Ile Thr His Ser Glu Asp Trp Lys Leu
    50                  55                  60

Trp Arg Cys Arg Leu Arg Leu Lys Ser Phe Thr Ser Met Asp Ser Arg
65                  70                  75                  80

Ser Ala Ser His Arg Ser Thr Arg Phe Ala Ala Thr Phe Tyr Asp Ile
                85                  90                  95

Glu Thr Leu Lys Val Ile Asp Glu Glu Trp Gln Arg Thr Gln Cys Ser
            100                 105                 110

Pro Arg Glu Thr Cys Val Glu Val Ala Ser Glu Leu Gly Lys Ser Thr
        115                 120                 125

Asn Thr Phe Phe Lys Pro Pro Cys Val Asn Val Phe Arg Cys Gly Gly
    130                 135                 140

Cys Cys Asn Glu Glu Ser Leu Ile Cys Met Asn Thr Ser Thr Ser Tyr

145                 150                 155                 160

Ile Ser Lys Gln Leu Phe Glu Ile Ser Val Pro Leu Thr Ser Val Pro
                165                 170                 175

Glu Leu Val Pro Val Lys Val Ala Asn His Thr Gly Cys Lys Cys Leu
            180                 185                 190

Pro Thr Ala Pro Arg His Pro Tyr Ser Ile Ile Arg Arg Ser Ile Gln
        195                 200                 205

Ile Pro Glu Glu Asp Arg Cys Ser His Ser Lys Lys Leu Cys Pro Ile
    210                 215                 220

Asp Met Leu Trp Asp Ser Asn Lys Cys Lys Cys Val Leu Gln Glu Glu
225                 230                 235                 240

Asn Pro Leu Ala Gly Thr Glu Asp His Ser His Leu Gln Glu Pro Ala
                245                 250                 255

Leu Cys Gly Pro His Met Met Phe Asp Glu Asp Arg Cys Glu Cys Val
            260                 265                 270

Cys Lys Thr Pro Cys Pro Lys Asp Leu Ile Gln His Pro Lys Asn Cys
        275                 280                 285

Ser Cys Phe Glu Cys Lys Glu Ser Leu Glu Thr Cys Cys Gln Lys His
    290                 295                 300

Lys Leu Phe His Pro Asp Thr Cys Ser Cys Glu Asp Arg Cys Pro Phe
305                 310                 315                 320

His Thr Arg Pro Cys Ala Ser Gly Lys Thr Ala Cys Ala Lys His Cys
                325                 330                 335

Arg Phe Pro Lys Glu Lys Arg Ala Ala Gln Gly Pro His Ser Arg Lys
            340                 345                 350

Asn Pro

<210>  47
<211>  2069
<212>  RNA
<213>  Homo sapiens

<400>  47
aagacacaug cuucugcaag cuuccaugaa gguugugcaa aaaaguuuca auccagaguu        60

ggguuccagc uuucuguagc uguaagcauu gguggccaca ccaccuccuu acaaagcaac 120

uagaaccugc ggcauacauu ggagagauuu uuuuaauuuu cuggacauga aguaaauuua 180

gagugcuuuc uaauuucagg uagaagacau guccaccuuc ugauuauuuu uggagaacau 240

uuugauuuuu uucaucucuc ucuccccacc ccuaagauug ugcaaaaaaa gcguaccuug 300

ccuaauugaa auaauuucau uggauuuuga ucagaacuga uuauuugguu uucuguguga 360

aguuuugagg uuucaaacuu uccuucugga gaaugccuuu ugaaacaauu uucucuagcu 420

gccugauguc aacugcuuag uaaucagugg auauugaaau auucaaaaug uacagagagu 480

ggguaguggu gaauguuuuc augauguugu acguccagcu ggugcagggc uccaguaaug 540

aacauggacc agugaagcga ucaucucagu ccacauugga acgaucugaa cagcagauca 600

gggcugcuuc uaguuuggag gaacuacuuc gaauuacuca cucugaggac uggaagcugu 660

ggagaugcag gcugaggcuc aaaaguuuua ccaguaugga cucucgcuca gcaucccauc 720

gguccacuag guuugcggca acuuucuaug acauugaaac acuaaaaguu auagaugaag 780

aauggcaaag aacucagugc agcccuagag aaacgugcgu ggagguggcc agugagcugg 840

ggaagaguac caacacauuc uucaagcccc cuugugugaa cguguuccga ugugguggcu 900

guugcaauga agagagccuu aucuguauga acaccagcac cucguacauu uccaaacagc 960

ucuuugagau aucagugccu uugacaucag uaccugaauu agugccuguu aaaguugcca 1020

aucauacagg uuguaagugc uugccaacag ccccccgcca uccauacuca auuaucagaa 1080

gauccaucca gaucccugaa gaagaucgcu guucccauuc caagaaacuc uguccuauug 1140

acaugcuaug ggauagcaac aaauguaaau guguuuugca ggaggaaaau ccacuugcug 1200

gaacagaaga ccacucucau cuccaggaac cagcucucug ugggccacac augauguuug 1260

acgaagaucg uugcgagugu gucuguaaaa caccaugucc caaagaucua auccagcacc 1320

ccaaaaacug caguugcuuu gagugcaaag aaagucugga gaccugcugc cagaagcaca 1380

agcuauuuca cccagacacc ugcagcugug aggacagaug ccccuuucau accagaccau 1440

gugcaagugg caaaacagca ugugcaaagc auugccgcuu uccaaaggag aaaagggcug 1500

cccaggggcc ccacagccga aagaauccuu gauucagcgu uccaaguucc ccaucccugu 1560

cauuuuuaac agcaugcugc uuugccaagu ugcugucacu guuuuuuucc cagguguuaa 1620

aaaaaaaauc cauuuuacac agcaccacag ugaauccaga ccaaccuucc auucacacca 1680

gcuaaggagu cccugguuca uugauggaug ucuucuagcu gcagaugccu cugcgcacca 1740

aggaauggag aggaggggac ccauguaauc cuuuuguuua guuuuguuuu uguuuuuugg 1800

ugaaugagaa aggugugcug gucauggaau ggcagguguc auaugacuga uuacucagag 1860

cagaugagga aaacuguagu cucugagucc uuugcuaauc gcaacucuug ugaauuauuc 1920

ugauucuuuu uuaugcagaa uuugauucgu augaucagua cugacuuucu gauuacuguc 1980

cagcuuauag ucuuccaguu uaaugaacua ccaucugaug uuucauauuu aaguguauuu 2040

aaagaaaaua aacaccauua uucaagcca 2069